# EUROPEAN PATENT APPLICATION

(11) **EP 2 626 357 A1**
(43) Date of publication of application: **14.08.2013**
(21) Application number: 12305149.2
(22) Date of filing: 10.02.2012
(51) Int. Cl.: C07D 405/06, C07D 409/06, C07D 207/333, A61K 31/40, A61K 31/4025, A61P 31/04

(54) **Pyrrolic derivatives, processes for preparing the same and their uses as antibiotics**

(71) Applicant: Université Joseph Fourier, 38041 Grenoble Cedex 09 (FR); Centre Hospitalier Universitaire de Grenoble, 38043 Grenoble Cedex 9 (FR); Centre National de la Recherche Scientifique, 75794 Paris Cedex 16 (FR)
(72) Inventor: Pelloux-Leon, Nadia, 38190 Bernin (FR); Minassian, Frédéric, 38340 Voreppe (FR); Maurin, Max, Maurin, Louis, 38240 Meylan (FR); Denis, Jean-Noël, 38560 Jarrie (FR)
(74) Representative: Grosset-Fournier, Chantal Catherine

(57) **Abstract**

The present invention relates to pyrrolic derivatives processes for their preparation, and their use as new antibacterial drugs.

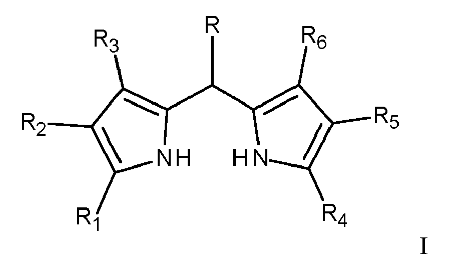

## Description

The present invention relates to pyrrolic derivatives, processes for their preparation, and their use as new antibacterial drugs.

Bacteria are an abundant and diverse life form on earth. Some of them have colonized the human body and ensure for this latter functions that are essential to life. Others, however, are pathogenic for the host.

Louis Pasteur's experiments in 1870 highlighted the fact that such organisms could be infectious agents. Between 1878 and 1880, three pathogens were isolated: *staphylococcus, streptococcus* and *pneumococcus.*

The first antibiotic, penicillin G discovered by Alexander Fleming in 1929, was used on a human being for the first time in 1941 to treat a staphylococcal sepsis. Since then, the number of antibiotics has steadily increased and the use of these drugs has become widespread. Their excessive use and a trivialization of their use within both human and veterinary medicine have favored the emergence of antibiotic resistance phenomena.

*Staphylococcus aureus,* first described in 1881 by Alexander Ogston, are Gram-positive bacteria which frequently colonize animals and human beings. They are found in healthy carriers on mucous membranes (nasal cavity, gastrointestinal tract) and on the skin (axilla, perineum). This carriage state is usually transient.

Sometimes, however, following the breakdown of the mucocutaneous barrier, or due to reduced immune defenses, *Staphylococcus aureus* are responsible for infections of the skin (boils, paronychia, impetigo, etc.) or the mucosa (otitis, conjunctivitis, etc.), or even deep infections and bacteremia. These infections are either community- or hospital-acquired. In addition, *Staphylococcus aureus* secretes many toxins including enterotoxins and TSST-1 that may cause food poisoning and severe toxic shock syndromes.

*Staphylococcus aureus* infections were first treated with penicillin G. Unfortunately, from 1953, *Staphylococcus aureus* strains resistant to penicillin G because of synthesis of a penicillinase have emerged. This problem was temporarily resolved by the discovery in 1959 of penicillin M derivatives such as methicillin (a β-lactam derivative of penicillin G, but resistant to the action of penicillinase). These penicillin M derivatives allowed treatment of infections caused by methicillin-susceptible *Staphylococcus aureus* (MSSA) strains.

Two years later, in 1961, *Staphylococcus aureus* strains resistant to methicillin (MRSA) were described. These MRSA strains are resistant to all currently available betalactam compounds, and they are often multi-drug resistant bacteria because they rapidly accumulate resistance mechanisms to other antibiotic families. They are frequently responsible for severe and even fatal nosocomial infections. More recently, however, community-acquired infections caused by MRSA strains have been described, including in previously healthy persons.

Treatment options for MRSA-related infections are few, often associated with potentially serious side effects, and are associated with a high proportion of relapses and failures (I. M. Goud, R. Cauda, S. Esposito, F. Gudiol, T. Mazzei, J. Garau Int. J. Antimicrob. Agents 2011, 37, 202-209). MRSA strains are usually susceptible to the glycopeptides vancomycin (discovered in 1956) and teicoplanin. However, these drugs may induce severe side effects (especially renal and cochleovestibular toxicity), are only administrable intravenously, and have a high manufacturing cost due to their structural complexity. In 1997, a MRSA strain with high-level resistance to both methicillin and glycopeptides was reported in Japan, but such strains have remained very exceptional. In contrast, *S. aureus* strains with intermediate-level resistance to glycopeptides (referred as VISA or GISA strains) have become common and widespread.

To overcome said resistance to both beta-lactams and glycopeptides, linezolid (Y. Van Laethem, J. Sternon Rev. Med. Brux. 2004, 25, 45-50), a synthetic antibiotic belonging to the family oxazolidinones, was launched in 2006 by Pfizer. Its spectrum includes several Gram-positive bacterial species and it is indicated in the treatment of lung infections, and skin and soft tissue infections caused by multi-drug resistant *Staphylococcus aureus* strains. Side effects are numerous, including frequent superinfections with *Candida* species and more rarely myelotoxicity (anemia, neutropenia, thrombocytopenia).

Another molecule, daptomycin (F. P. Tally, M. F. DeBruin J. Antimicrob. Chemother. 2000, 46, 523-526; L. Röbbel, M. A. Marahiel J. Biol. Chem. 2010, 285, 27501-27508), a natural lipopeptide, was marketed in Europe under the name Cubicin^{®}. Its spectrum of activity includes many gram-positive aerobic or anaerobic bacteria. Daptomycin is effective for the treatment of complicated skin and soft tissue infections. In contrast, it is not effective in the treatment of nosocomial pneumonia. The use of this molecule is limited by its potential muscle toxicity.

Finally, tigecycline (L. R. Peterson Int. J. Antimicrob. Agents 2008, 32, S215-S222), a glycylcycline derivative of minocycline, has been used for the treatment of complicated skin and soft tissue infections, for intra-abdominal infections and nosocomial pneumonia. However, its spectrum of activity is both too broad in the context of staphylococcal infections, and too narrow in the context of complicated pulmonary or gastrointestinal infections, said infections usually being polymicrobial. This molecule also has many potential side effects, including coagulation disorders and hepatotoxicity. Following clinical trials showing an excess mortality in patients treated with tigecycline for complicated skin and soft tissue or intraabdominal infections, Pfizer, which markets tigecycline under the name of Tygacil^{®}, therefore, issued a statement indicating that "this drug should be used in the absence of appropriate treatment alternative".

The rapid emergence of multi-drug resistant MRSA strains may lead more and more often to therapeutic impasses. There is an urgent need for development of new therapeutic alternatives. The majority of the antibiotics synthesized during the last three decades were derived from older molecules to which bacteria had already developed resistances. Because these new compounds share the same active moiety, bacteria have rapidly developed further resistances to them. The oxazolidinones and lipopeptides are the only new antibiotic families developed during that period. However, the use of these new compounds is very limited due to their high toxicity. Also, most of these new molecules have very complex structures. The big pharmaceutical companies now hesitate to invest in this medicinal domain because the earning potential is unpredictable and can even be null in case of rapid emergence of bacterial resistances to the newly developed compounds.

One objective of the present invention is to provide compounds liable to inhibit growth of *Staphylococcus,* in particular *Staphylococcus aureus,* and with original and easy to obtain structures.

Another aim of the invention is to provide compounds liable to inhibit growth of *Staphylococcus aureus* strains resistant to currently available antibiotics.

Another aim of the invention is to provide compounds liable to selectively inhibit growth of *Staphylococcus aureus* species.

Another aim of the invention is to provide pharmaceutical compositions comprising said compounds.

Still another aim of the invention is to provide compounds with a new structure.

The present invention relates to compounds, for use as antibacterial drugs to treat infection(s) caused by bacteria of the genus *Staphylococcus,* in particular *Staphylococcus aureus* species of the following formula I: 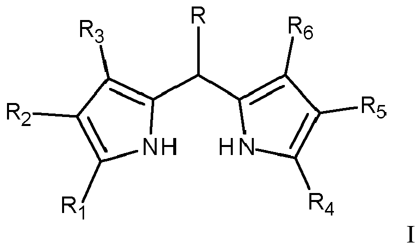 wherein:
R₁, R₂, R₃, R₄, R₅ and R₆ represent independently from each other:
   - a H,
   - a linear or branched (C₁-C₇)-alkyl,
   - a linear or branched (C₂-C₇)-alkenyl,
   - a linear or branched (C₂-C₇)-alkynyl,
   - a (C₃-C₇)-cycloalkyl,
   - a (C₅-C₇)-cycloalkenyl,
   - a (C₃-C₇)-heterocycloalkyl,
   - a (C₅-C₇)-heterocycloalkenyl,
   - an aryl, said aryl being an aromatic or heteroaromatic group,
   - an alkyl aryl, wherein the aryl is an aromatic or heteroaromatic group,
   - a CHO,
   - a CO-(C₁-C₇)-alkyl,
   - a CO-aryl, wherein aryl is an aromatic or heteroaromatic group,
   - a CO₂H,
   - a CO₂-(C₁-C₇)-alkyl,
   - a CONH-(C₁-C₇)-alkyl,
   - a halogen selected from the group comprising F, Cl, Br, and I,
   - CF₃,
   - ORₐ, wherein Rₐ represents:
      H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
   - NR_{b}R_{c}, wherein R_{b} and R_{c} represent independently from each other:
      H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
   - NO₂,
   - CN,
R represents:
   - a H,
   - a linear or branched (C₁-C₇)-alkyl,
   - a linear or branched (C₂-C₇)-alkenyl,
   - a linear or branched (C₂-C₇)-alkynyl,
   - a (C₃-C₇)-cycloalkyl,
   - a (C₅-C₇)-cycloalkenyl,
   - a (C₃-C₇)-heterocycloalkyl,
   - a (C₅-C₇)-heterocycloalkenyl,
   - an aryl, said aryl being an aromatic or heteroaromatic group,
   - an alkyl aryl, wherein the aryl is an aromatic or heteroaromatic group,
   - a CHO,
   - a CO-(C₁-C₇)-alkyl,
   - a CO-aryl, wherein aryl is an aromatic or heteroaromatic group,
   - a CO₂H,
   - a CO₂-(C₁-C₇)-alkyl,
   - a CO₂-aryl, wherein aryl is an aromatic or heteroaromatic group,
   - a CONH-(C₁-C₇)-alkyl,
said R₁, R₂, R₃, R₄, R₅, R₆ and R being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
said (C₁-C₇)-alkyl, (C₂-C₇)-alkenyl, (C₂-C₇)-alkynyl, (C₃-C₇)-cycloalkyl, (C₅-C₇)-cycloalkenyl, (C₃-C₇)-heterocycloalkyl, (C₅-C₇)-heterocycloalkenyl, CO-(C₁-C₇)-alkyl, CO₂-(C₁-C₇)-alkyl, CONH-(C₁-C₇)-alkyl, aryl, alkyl aryl and CO-aryl being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
and their pharmaceutically acceptable salts.

By linear (C₁-C₇) alkyl group is meant a group such as methyl, ethyl, propyl, butyl, pentyl, hexyl or heptyl.

By branched alkyl group is meant an alkyl group as defined above bearing substituents selected from the list of linear alkyl groups defined above, said linear alkyl group being also liable to be branched.

By linear (C₂-C₇) alkenyl group is meant a linear hydrocarbon group constituted by 2 to 7 carbon atoms, with one or more carbon-carbon double bond(s).

By branched alkenyl group is meant an alkenyl group as defined above bearing substituents selected from the list of linear alkyl groups defined above, said linear alkyl group being also liable to be branched.

By linear (C₂-C₇) alkynyl group is meant a linear hydrocarbon group constituted by 2 to 7 carbon atoms, with one or more carbon-carbon triple bond(s).

By branched alkynyl group is meant an alkynyl group as defined above bearing substituents selected from the list of linear alkyl groups defined above, said linear alkyl group being also liable to be branched.

By (C₃-C₇)-cycloalkyl group is meant a group such as cyclopropyl, cyclobutyl, cyclopentyl, cyclohexyl or cycloheptyl.

By (C₅-C₇)-cycloalkenyl group is meant a cyclic hydrocarbon group constituted by 5 to 7 carbon atoms, with one or more carbon-carbon double bond(s).

By (C₃-C₇)-heterocycloalkyl group is meant a (C₃-C₇)-cyclic group having at least one non-carbon atom in the ring.

By (C₅-C₇)-heterocycloalkenyl group is meant a heterocyclic group constituted by 5 to 7 carbon atoms, with one or more double bond(s).

The term "aryl" refers to any functional group or substituent derived from a simple aromatic ring.

The term "heteroaromatic" refers to a compound having the characteristics of an aromatic compound whilst having at least one non-carbon atom in the ring.

By alkyl aryl group is meant a linear or branched alkyl group that is substituted by an aryl group.

The above-mentioned definitions apply to the entire specification.

A basic group such as the nitrogen of the pyrrole moiety or an amino group present on the molecule can be under a salt form, the salt being any pharmaceutically acceptable salt obtained by reaction of an inorganic acid, an organic acid or a halogenoalkyl, on an amino group to give a quaternary ammonium.

Examples of inorganic acid allowing to obtain pharmaceutically acceptable salts include, without being limited to them, hydrochloric acid, hydrobromic acid, hydroiodic acid, nitric acid, carbonic acid, formic acid, monohydrogenocarbonic acid, phosphoric acid, monohydrogenophosphoric acid, dihydrogenophosphoric acid, perchloric acid, sulfuric acid, monohydrogenosulfuric acid.

Examples of organic acids allowing to obtain pharmaceutically acceptable salts include, without being limited to them, acetic acid, lactic acid, propionic acid, butyric acid, isobutyric acid, palmitic acid, malic acid, glutamic acid, hydroxymalic acid, malonic acid, benzoic acid, succinic acid, glycolic acid, suberic acid, fumaric acid, mandelic acid, phthalic acid, salicylic acid, benzenesulfonic acid, p-tolylsulfonic acid, citric acid, tartaric acid, methanesulfonic acid, hydroxynaphthoic acid.

As the molecule can also bear an acid group, and as at least one substituent of the pyrrole moiety or of the aryl or heteroaromatic groups can be a phenol, they can also be under a pharmaceutically acceptable salt form.

The salt can be obtained with organic or mineral bases, to give for instance alkali metal salts such as lithium, sodium, potassium salts.

As an example, see S. M. Berge, L. D. Bighley, D. C. Monkhouse, "Pharmaceutical Salts", Journal of Pharmaceutical Science, 1977, 66, 1-19.

When R is different from H, the molecule presents a stereogenic element and thus compounds of the invention can be the *R* or the *S* enantiomer, a racemic mixture of both enantiomers or a mixture comprising 0.01%-99.99% of the *R* enantiomer and 99.99%-0.01% of the *S* enantiomer.

Interestingly, the inventors have found that some compounds bearing two pyrrole moieties present an antibiotic activity against *Staphylococcus aureus* strains either susceptible or resistant to currently available antibiotics (including MRSA and GISA strains). Their spectrum of activity is almost restricted to this species. In particular, they are not active against *Staphylococcus* species belonging to the commensal cutaneous flora such as *Staphylococcus epidermidis.* Because these bis-pyrrolic compounds have very original structures compared to all existing antibiotics, and their activity is restricted to *Staphylococcus aureus* strains, we can assume that their clinical use will not lead to rapid development of resistances in *Staphylococcus aureus* strains neither in other bacterial species.

By susceptible strains is meant strains whose growth is inhibited by antibiotic concentrations usually achievable in human serum and at the site of infection when the recommended dosage is used.

By resistant strains is meant strains whose growth is not inhibited by antibiotic concentrations achievable in human serum and at the site of infection when the recommended dosage is used.

In practice, susceptibility and resistance of a bacterial strain to a given antibiotic are defined by determining the MIC of this antibiotic for that strain and analyzing results according to lower and higher breakpoints and interpretative standards of the Clinical and Laboratory Standards Institute in the USA (Clinical and Laboratory Standards Institute. Performance standards for antimicrobial susceptibility testing. M100-S19, CLSI, PA, USA) or equivalent standards in other countries. An MIC inferior to the lower breakpoint indicates susceptibility. An MIC superior to the higher breakpoint indicates resistance. An MIC between the two breakpoints indicates intermediate susceptibility.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-1: 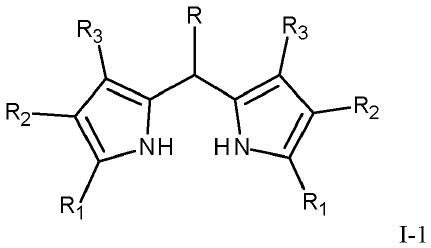 wherein R₁, R₂, R₃ and R are as defined above, R₂ representing preferably CO-CH₃.

In an advantageous embodiment, the present invention relates to compounds of formula I, for use as an antibacterial drug against infection(s) caused by bacteria of the genus *Staphylococcus,* in particular *Staphylococcus aureus* species, wherein at least one of R₁, R₂ and R₃ is different respectively from R₄, R₅ and R₆.

In an advantageous embodiment, the present invention relates to compounds of formula I, for use as an antibacterial drug against infection(s) caused by bacteria of the genus *Staphylococcus,* in particular *Staphylococcus aureus* species, wherein at least one of R₁, R₂, R₃, R₄, R₅ and R₆ represents:
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- CF₃,
- NO₂,
- CN.

In an advantageous embodiment, the present invention relates to compounds of formula I, for use as an antibacterial drug against infection(s) caused by bacteria of the genus *Staphylococcus,* in particular *Staphylococcus aureus* species, wherein R₂ and/or R₅ are/is selected from the group comprising:
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- CF₃,
- NO₂,
- CN.

In an advantageous embodiment, the present invention relates to compounds of formula I, for use as an antibacterial drug against infection(s) caused by bacteria of the genus *Staphylococcus,* in particular *Staphylococcus aureus* species, wherein R₂ and/or R₅ represent(s) CO-CH₃.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-1bis: 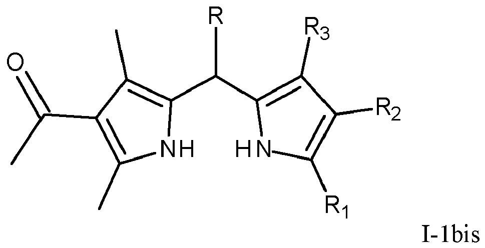 wherein R₁, R₂, R₃ and R are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-2: 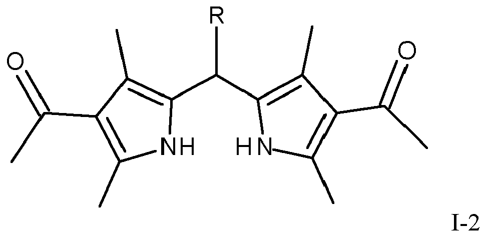 wherein R is as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-2bis: 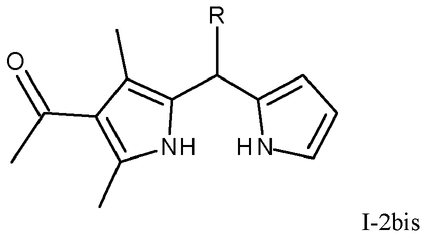 wherein R is as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-2ter : 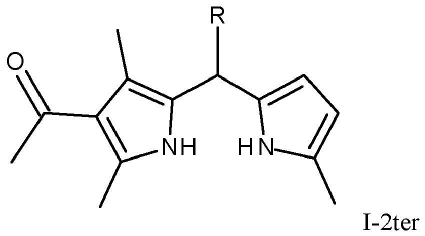 wherein R is as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-2quater : 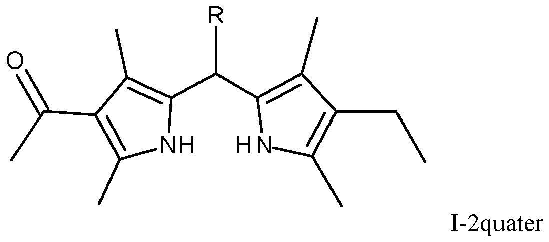 wherein R is as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, wherein R represents:
- a (C₁-C₇)-alkyl,
- an aryl, said aryl being an aromatic or heteroaromatic group,
   R representing preferably an aryl,
   said (C₁-C₇)-alkyl and aryl being substituted or not by one or more substituent(s), each independently selected from:
   - a linear or branched (C₁-C₇)-alkyl,
   - a linear or branched (C₂-C₇)-alkenyl,
   - a linear or branched (C₂-C₇)-alkynyl,
   - a (C₃-C₇)-cycloalkyl,
   - a (C₅-C₇)-cycloalkenyl,
   - a (C₃-C₇)-heterocycloalkyl,
   - a (C₅-C₇)-heterocycloalkenyl,
   - an aryl, wherein the aryl is an aromatic or heteroaromatic group,
   - an alkyl aryl, wherein the aryl is an aromatic or heteroaromatic group,
   - a CHO,
   - a CO-(C₁-C₇)-alkyl,
   - a CO-aryl, wherein aryl is an aromatic or heteroaromatic group,
   - a CO₂H,
   - a CO₂-(C₁-C₇)-alkyl,
   - a CONH-(C₁-C₇)-alkyl,
   - a halogen selected from the group comprising F, Cl, Br, and I,
   - CF₃,
   - ORₐ, wherein Rₐ represents:
      H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
   - NR_{b}R_{c}, wherein R_{b} and R_{c} represent independently from each other:
      H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
   - NO₂,
   - CN.

In an advantageous embodiment, the present invention relates to compounds of formula I defined above, for use as an antibacterial drug against infection(s) caused by bacteria of the genus *Staphylococcus,* in particular *Staphylococcus aureus* species, wherein R is selected from the group comprising: 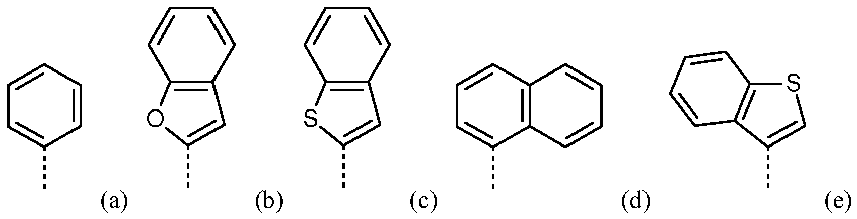 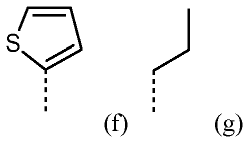 said R being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-3: 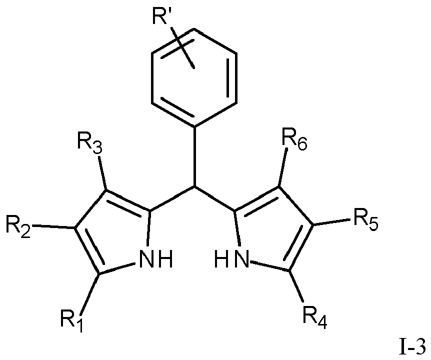 wherein R₁, R₂, R₃, R₄, R₅ and R₆ are as defined above, R₂ and R₅ representing preferably CO-CH₃, and wherein R' represents H, or one or more substituent(s), preferably one substituent, each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
   - an alkyl aryl, wherein the aryl is an aromatic or heteroaromatic group,
   - a CHO,
   - a CO-(C₁-C₇)-alkyl,
   - a CO-aryl, wherein aryl is an aromatic or heteroaromatic group,
   - a CO₂H,
   - a CO₂-(C₁-C₇)-alkyl,
   - a CONH-(C₁-C₇)-alkyl,
   - a halogen selected from the group comprising F, Cl, Br, and I,
   - CF₃,
      - ORₐ, wherein Rₐ represents:
         H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
   - NR_{b}R_{c}, wherein R_{b} and R_{c} represent independently from each other:
      H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
   - NO₂,
   - CN.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-4: 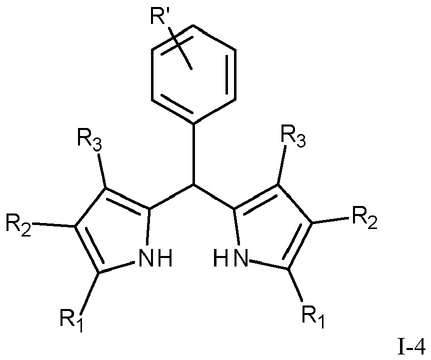 wherein R₁, R₂, R₃, and R' are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-5: 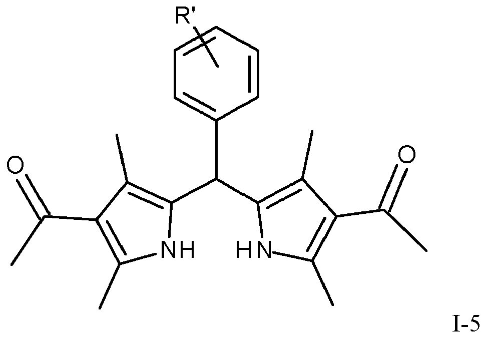 wherein R' is as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-6: 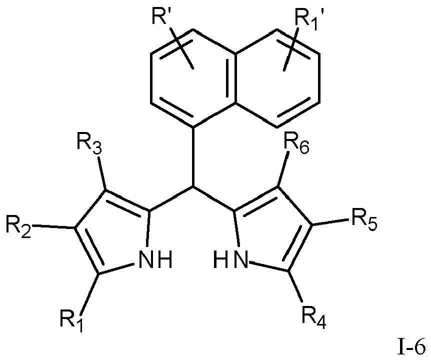 wherein R₁, R₂, R₃, R₄, R₅ and R₆ are as defined above, R₂ and R₅ representing preferably CO-CH₃, and wherein R' and R₁' represent, independently from each other, H or one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
preferably, R' represents H and R₁' represents one substituent, or R' represents one substituent and R₁' represents H.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-7: 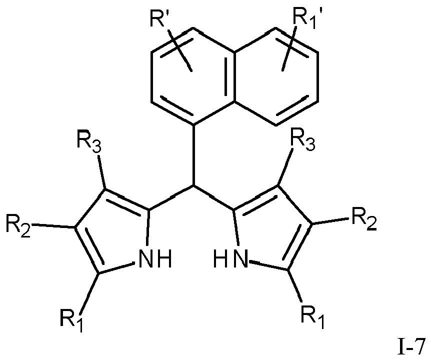 wherein R₁, R₂, R₃, R' and R₁' are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-8: 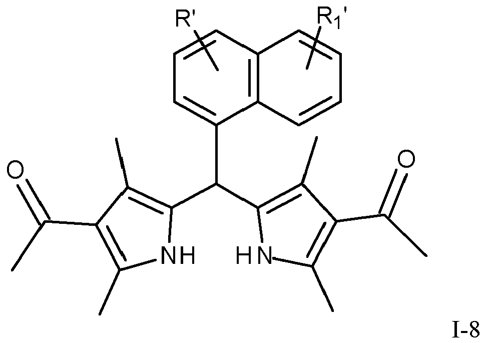 wherein R' and R₁' are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-9: 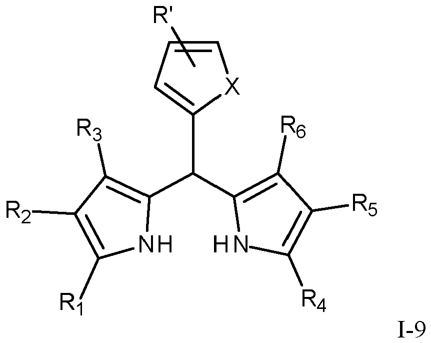 wherein R₁, R₂, R₃, R₄, R₅ and R₆ are as defined above, R₂ and R₅ representing preferably CO-CH₃, and
wherein X is selected from the group comprising O, S and NR₂', X being preferably S,
wherein R₂' represents H, a linear or branched (C₁-C₇)-alkyl, a CHO, a CO-(C₁-C₇)-alkyl, a CO-aryl, wherein aryl is an aromatic or heteroaromatic group, a CO₂(C₁-C₇)-alkyl, a CONH-(C₁-C₇)-alkyl, a CF₃, and
wherein R' represents H, or one or more substituent(s), preferably one substituent, each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
   - CF₃,
   - ORₐ, wherein Rₐ represents:
      H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
   - NR_{b}R_{c}, wherein R_{b} and R_{c} represent independently from each other:
      H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
   - NO₂,
   - CN.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-10: 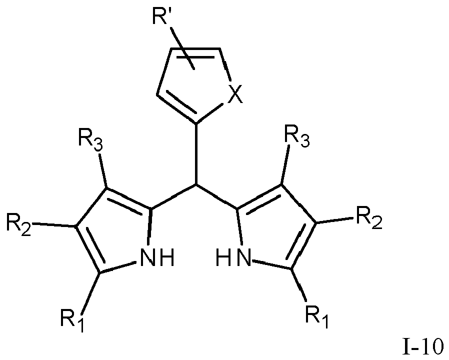 wherein R₁, R₂, R₃, R' and X are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-11: 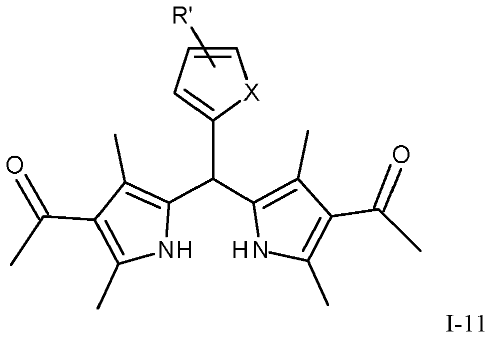 wherein R' and X are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-9bis: 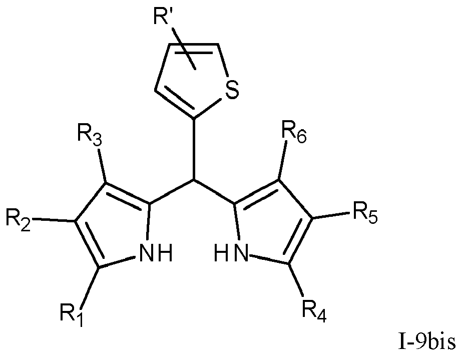 wherein R₁, R₂, R₃, R₄, R₅, R₆ and R' are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-10bis: 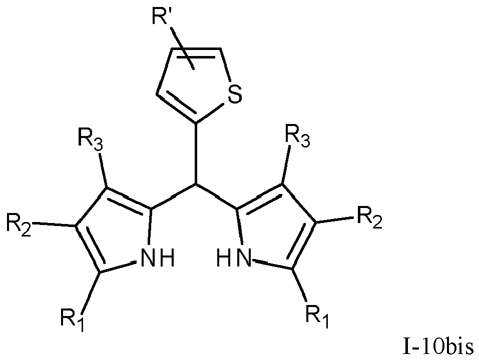 wherein R₁, R₂, R₃, and R' are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-11bis: 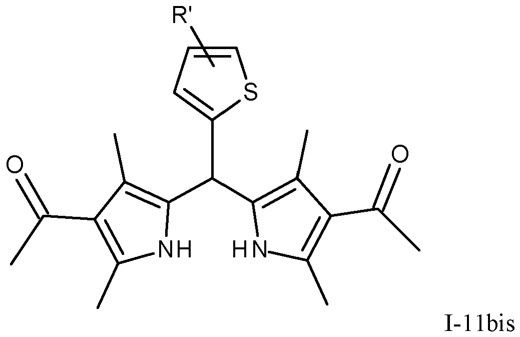 wherein R' is as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-12: 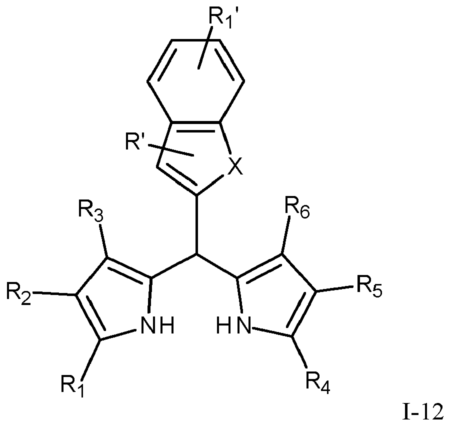 wherein R₁, R₂, R₃, R₄, R₅ and R₆ are as defined above, R₂ and R₅ representing preferably CO-CH₃, and
wherein X is selected from the group comprising O, S and NR₂', X being preferably S or O, wherein R₂' represents H, a linear or branched (C₁-C₇)-alkyl, a CHO, a CO-(C₁-C₇)-alkyl, a CO-aryl, wherein aryl is an aromatic or heteroaromatic group, a CO₂-(C₁-C₇)-alkyl, a CONH-(C₁-C₇)-alkyl, a CF₃ and
wherein R' and R₁' represent, independently from each other, H or one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
preferably, R' represents H and R₁' represents one substituent, or R' represents one substituent and R₁' represents H.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-13: 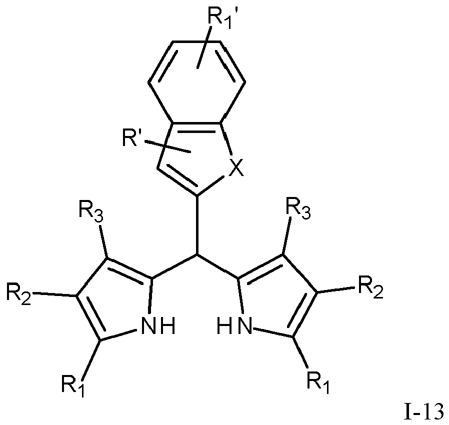 wherein R₁, R₂, R₃, R', R₁' and X are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-14: 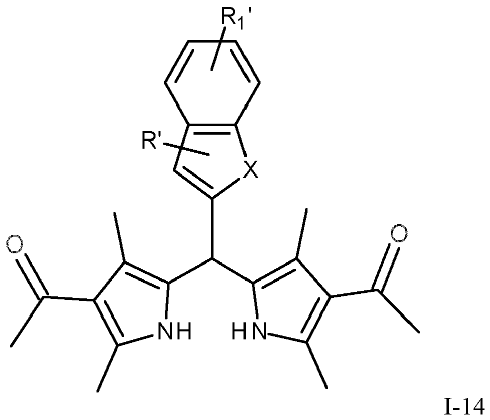 wherein R', R₁' and X are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-12bis: 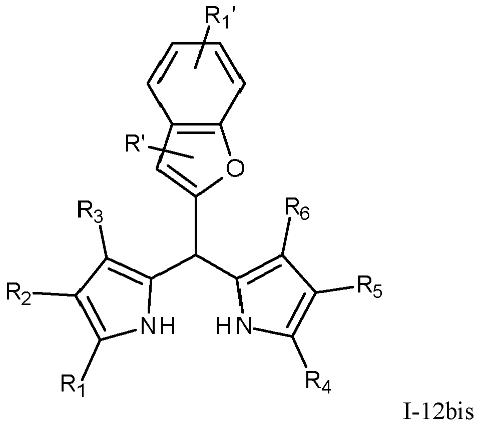 wherein R₁, R₂, R₃, R₄, R₅, R₆, R' and R₁' are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-13bis: 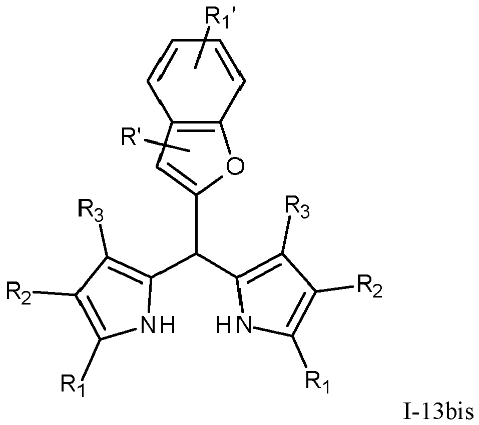 wherein R₁, R₂, R₃, R' and R₁' are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-14bis: 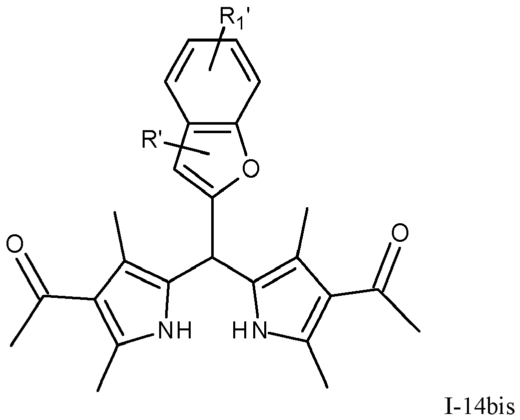 wherein R' and R₁' are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-12ter: 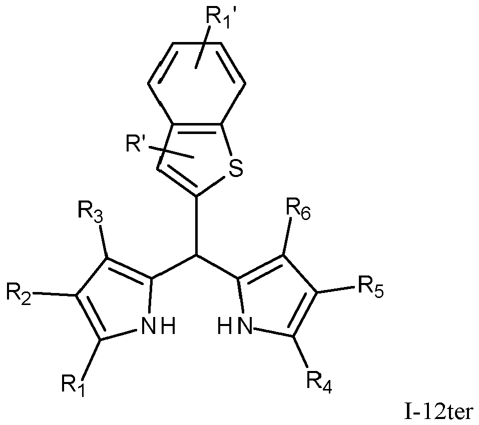 wherein R₁, R₂, R₃, R₄, R₅, R₆, R' and R₁' are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-13ter: 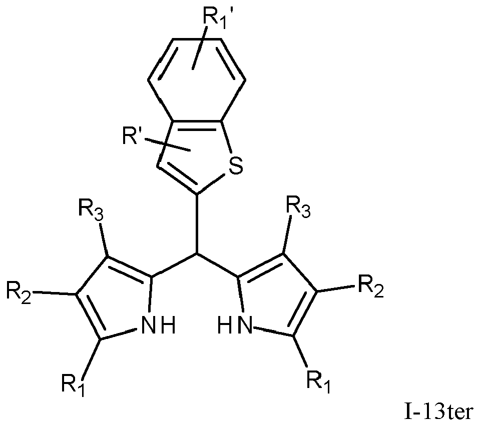 wherein R₁, R₂, R₃, R' and R₁' are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-14ter: 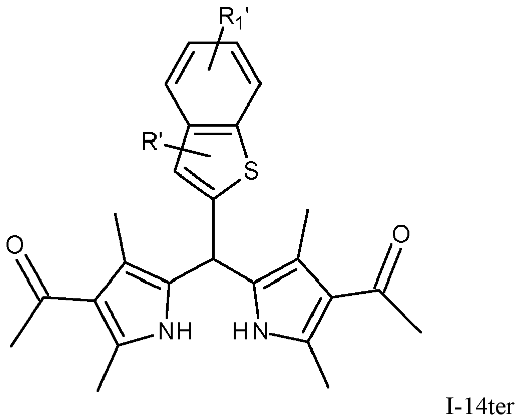 wherein R' and R₁' are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-15: 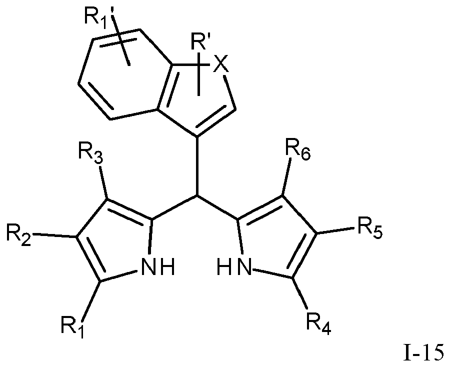 wherein R₁, R₂, R₃, R₄, R₅ and R₆ are as defined above, R₂ and R₅ representing preferably CO-CH₃, and
wherein X is selected from the group comprising O, S and NR₂', X being preferably S,
wherein R₂' represents H, a linear or branched (C₁-C₇)-alkyl, a CHO, a CO-(C₁-C₇)-alkyl, a CO-aryl, wherein aryl is an aromatic or heteroaromatic group, a CO₂-(C₁-C₇)-alkyl, a CONH-(C₁-C₇)-alkyl, a CF₃,
wherein R' and R₁' represent, independently from each other, H or one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
preferably, R' represents H and R₁' represents one substituent, or R' represents one substituent and R₁' represents H.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-16: 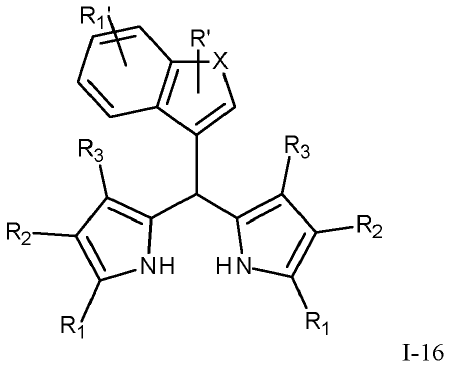 wherein R₁, R₂, R₃, R', R₁' and X are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-17: 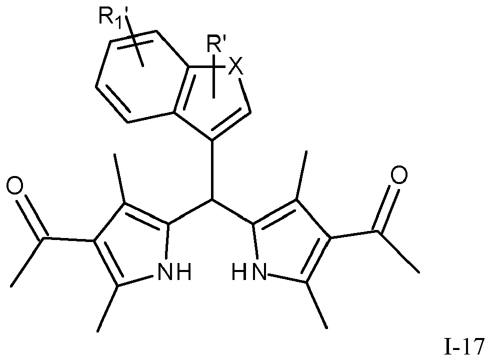 wherein R', R₁' and X are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-15bis: 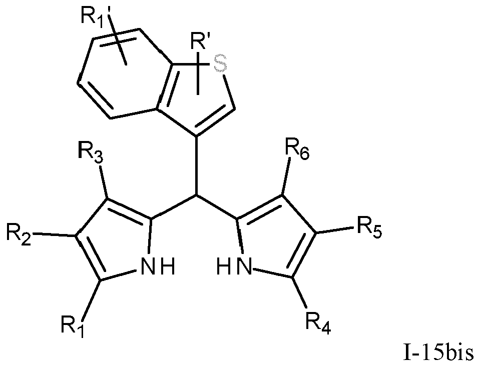 wherein R₁, R₂, R₃, R₄, R₅, R₆, R' and R₁' are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-16bis: 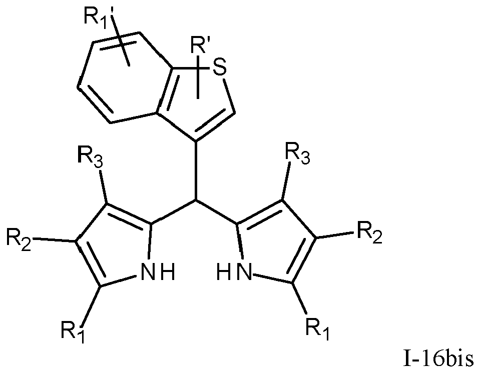 wherein R₁, R₂, R₃, R' and R₁' are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-17bis: 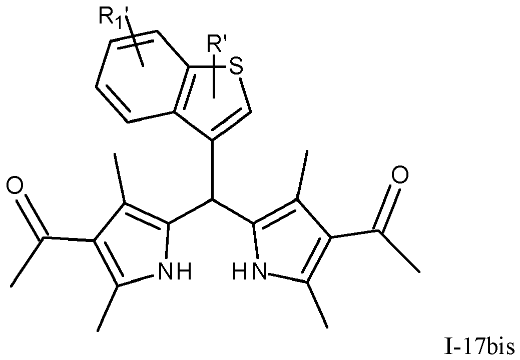 wherein R' and R₁' are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-18: 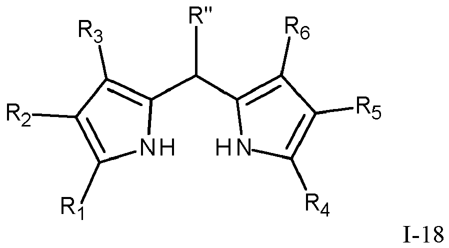 wherein R₁, R₂, R₃, R₄, R₅ and R₆ are as defined above, R₂ and R₅ representing preferably CO-CH₃, and
wherein R" is selected from the aryls of following formulae (a') to (b'): 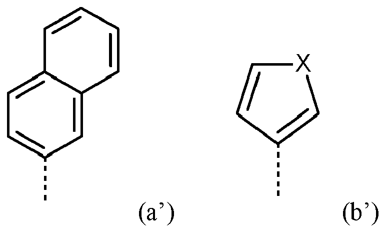 wherein X is selected from the group comprising O, S and NR₂', wherein R₂' represents H, a linear or branched (C₁-C₇)-alkyl, a CHO, a CO-(C₁-C₇)-alkyl, a CO-aryl, wherein aryl is an aromatic or heteroaromatic group, a CO₂-(C₁-C₇)-alkyl, a CONH-(C₁-C₇)-alkyl, a CF₃, said R" group being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-19: 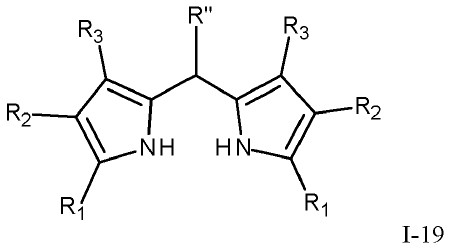 wherein R₁, R₂, R₃, and R" are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula I-20: 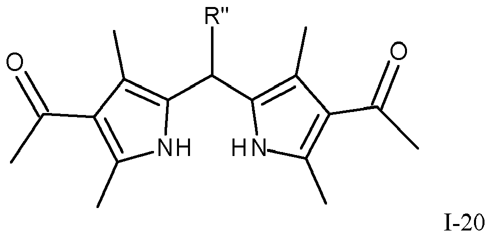 wherein R" is as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula Ibis: 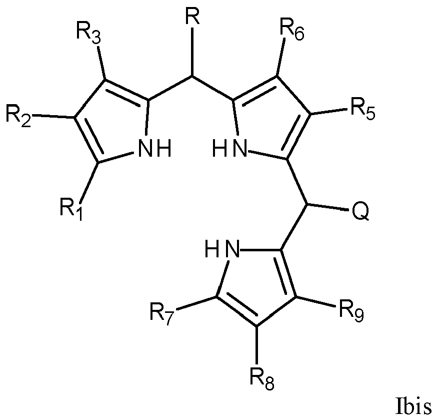 wherein R₁, R₂, R₃, R₅, R₆, and R are as defined above, and wherein R₇, R₈ and R₉ represent independently from each other:
- a H,
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl,
   wherein aryl is an aromatic or heteroaromatic group,
- NO₂,
- CN,
Q represents:
- a H,
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
said (C₁-C₇)-alkyl, (C₂-C₇)-alkenyl, (C₂-C₇)-alkynyl, (C₃-C₇)-cycloalkyl, (C₅-C₇)-cycloalkenyl, (C₃-C₇)-heterocycloalkyl, (C₅-C₇)-heterocycloalkenyl, CO-(C₁-C₇)-alkyl, CO₂-(C₁-C₇)-alkyl, CONH-(C₁-C₇)-alkyl, aryl, alkyl aryl and CO-aryl being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN.

When R is different from H and Q represents H, or when Q is different from H and R represents H, the molecule presents a stereogenic element and thus compounds of the invention can be the *R* or the *S* enantiomer, a racemic mixture of both enantiomers or a mixture comprising 0.01%-99.99% of the *R* enantiomer and 99.99%-0.01% of the *S* enantiomer.

When R and Q are different from H, the molecule presents two stereogenic elements and thus compounds of the invention can be the *RR*, *SS, RS* or *SR* stereoisomer, a racemic mixture of both *RR* and *SS* or *RS* and *SR* stereoisomers, a mixture comprising 0.01%-99.99% of the *RR* stereoisomer and 99.99%-0.01% of the *SS* stereoisomer, a mixture comprising 0.01%-99.99% of the *RS* stereoisomer and 99.99%-0.01% of the *SR* stereoisomer, a mixture comprising 50% of the racemic mixture of the RR and SS stereoisomers and 50% of the racemic mixture of the *RS* and *SR* stereoisomers, a mixture comprising 0.01%-99.99% of the racemic mixture of the *RR* and *SS* stereoisomers and 99.99%-0.01% of the racemic mixture of the *RS* and *SR* stereoisomers, or a mixture of the *RR*, *SS, RS*, and *SR* stereoisomers.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula Ibis-1: 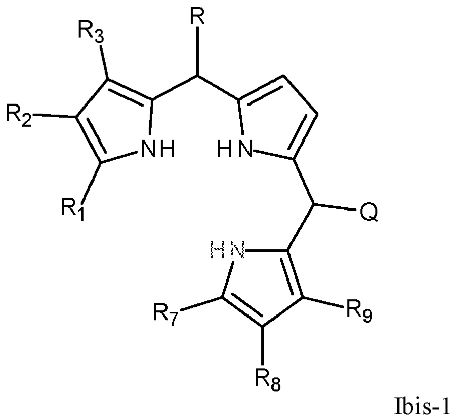 wherein R₁, R₂, R₃, R₇, R₈, R₉, Rand Q are as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula Ibis-2: 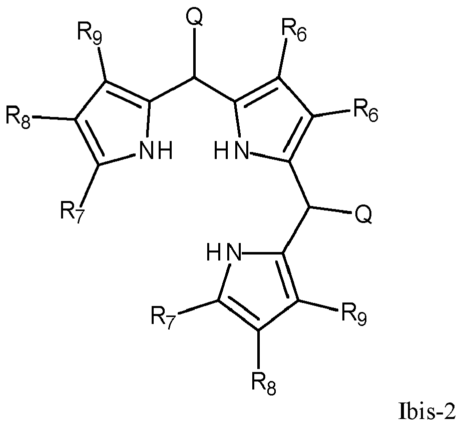 wherein R₆, R₇, R₈, R₉, and Q are as defined above, R₆ representing preferably H.

When Q is different from H, the molecule presents two stereogenic elements and thus compounds of the invention can be the *RR* or *SS* stereoisomer, the meso isomer, a racemic mixture of both RR and SS stereoisomers, a mixture comprising 0.01%-99.99% of the RR stereoisomer and 99.99%-0.01% of the *SS* stereoisomer, a mixture comprising 50% of the racemic mixture of the *RR* and *SS* stereoisomers and 50% of the meso isomer, a mixture comprising 0.01%-99.99% of the racemic mixture of the *RR* and *SS* stereoisomers and 99.99%-0.01% of the meso isomer, or a mixture of the *RR* and *SS* stereoisomer, and the meso isomer.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula Ibis-3: 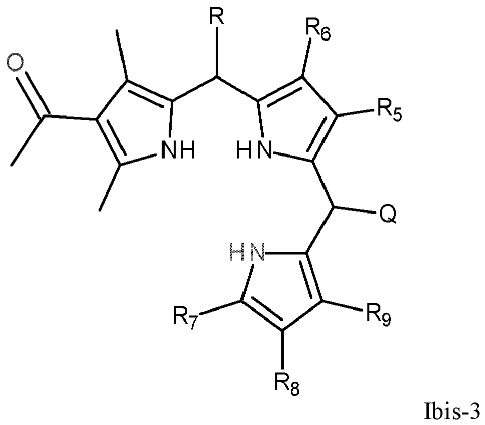 wherein R₅, R₆, R₇, R₈, R₉, R and Q are as defined above, R₅ and/or R₆ representing preferably H.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula Ibis-4: 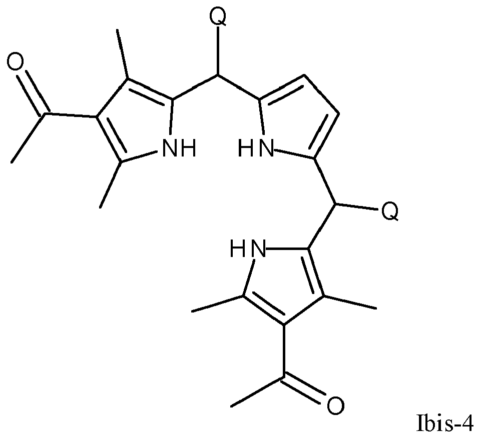 wherein Q is as defined above.

In an advantageous embodiment, the present invention relates to compounds of formula I defined above, for use as an antibacterial drug against infection(s) caused by bacteria of the genus *Staphylococcus,* in particular *Staphylococcus aureus* species, of the following formula Ibis-5: 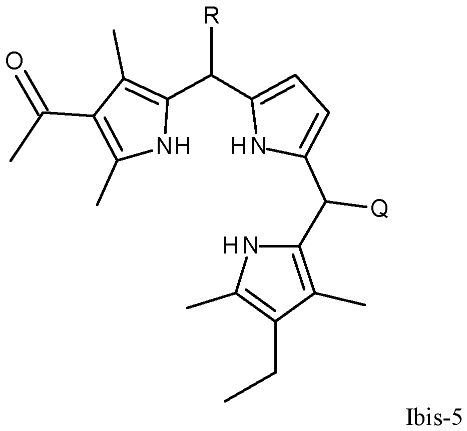 wherein R and Q are as defined above.

In an advantageous embodiment, the present invention relates to compounds of formula Ibis defined above, for use as an antibacterial drug against infection(s) caused by bacteria of the genus *Staphylococcus,* in particular *Staphylococcus aureus* species, wherein R and Q are, independently from each other, selected from the group comprising: 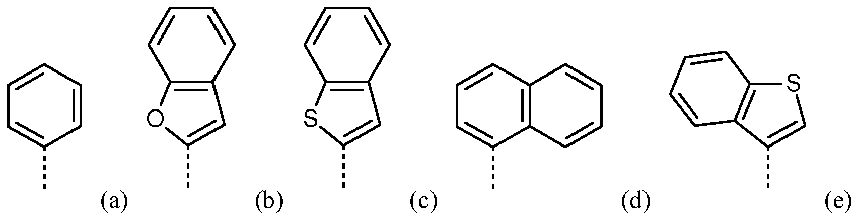 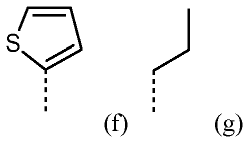 wherein the aryls (a), (b), (c), (d), (e) and (f) are substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN.

In an advantageous embodiment, the present invention relates to compounds of formula I defined above, for use as an antibacterial drug against infection(s) caused by bacteria of the genus *Staphylococcus,* in particular *Staphylococcus aureus* species, selected from the group comprising:

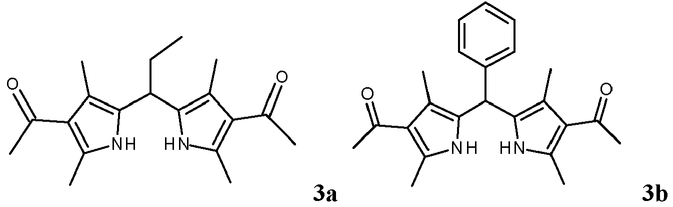

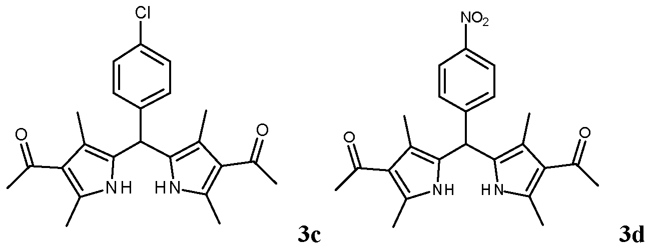

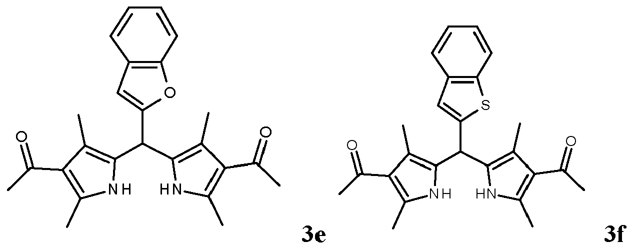

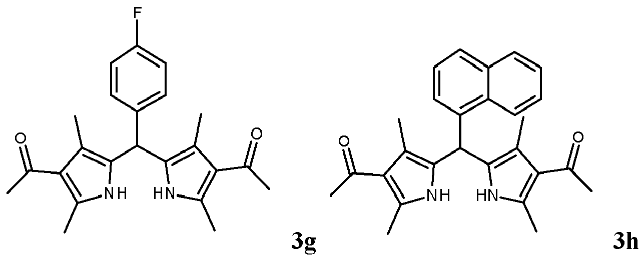

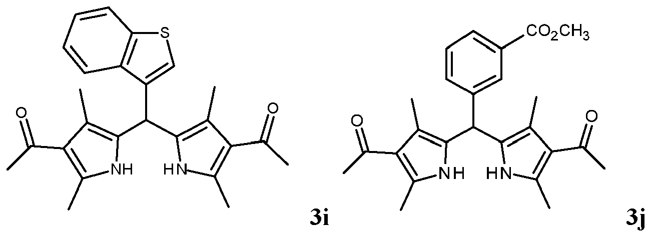

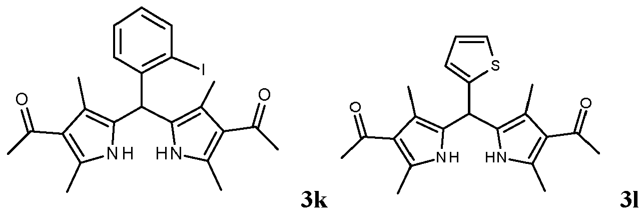

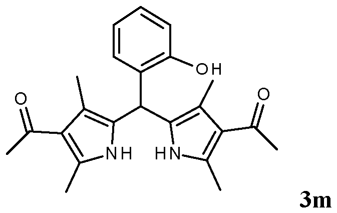

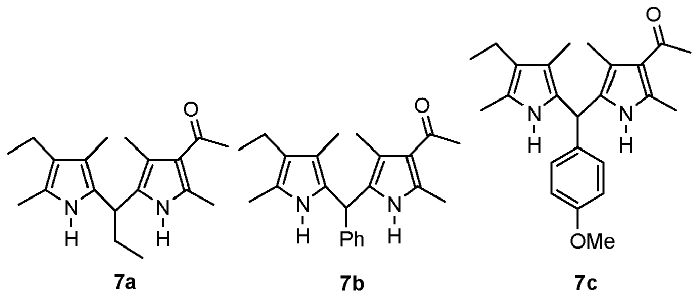

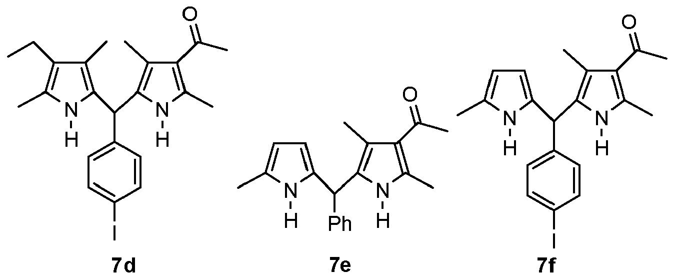

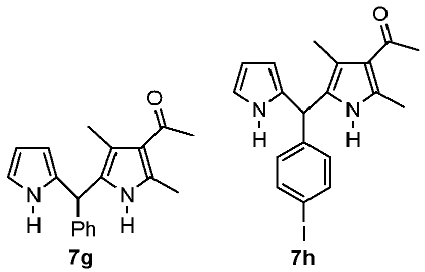

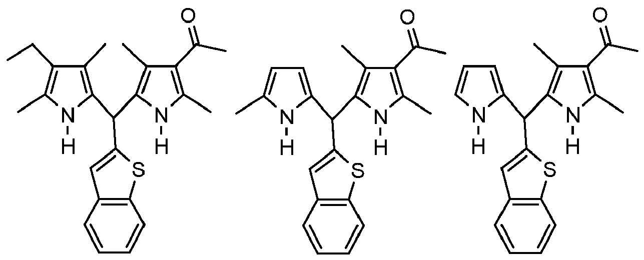

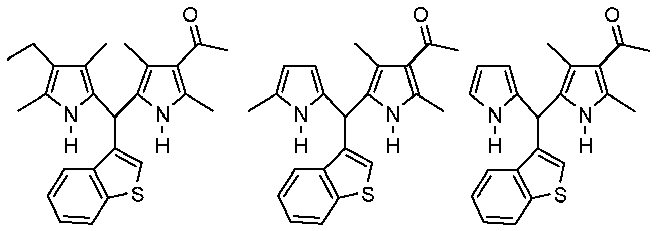

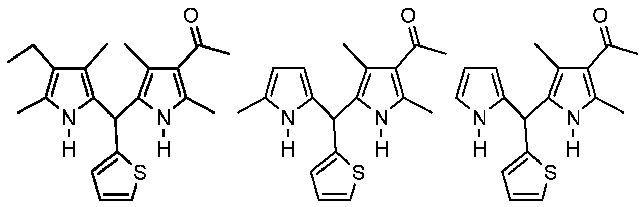

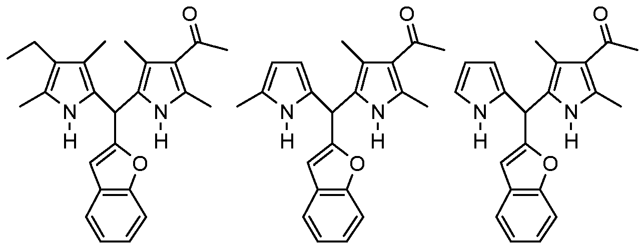

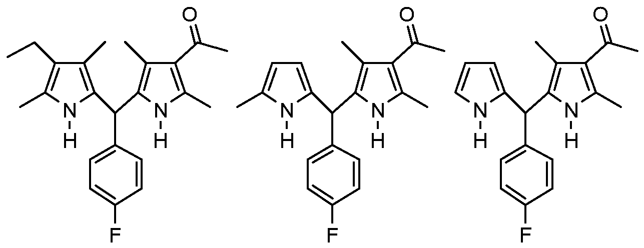

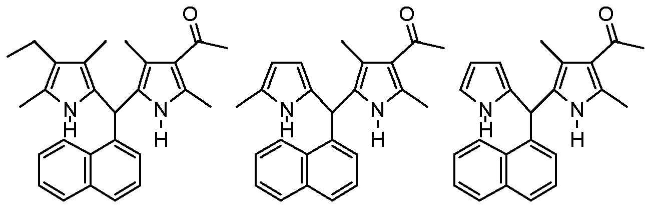

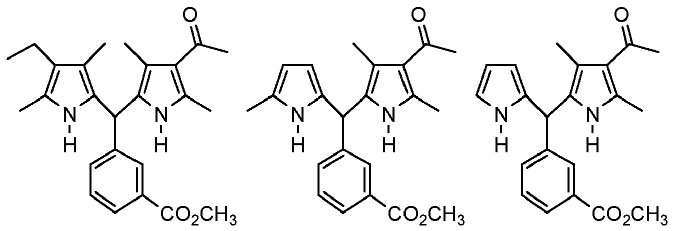

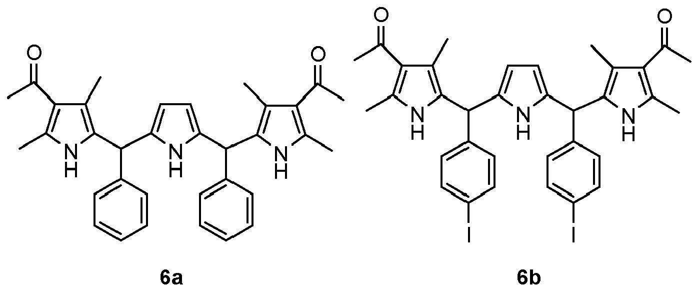

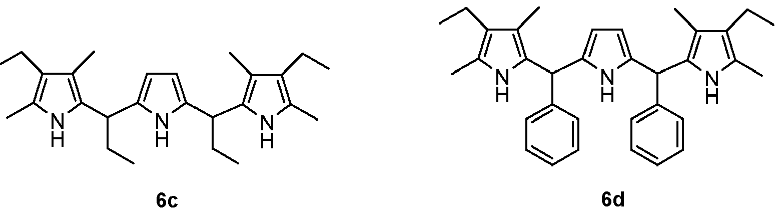

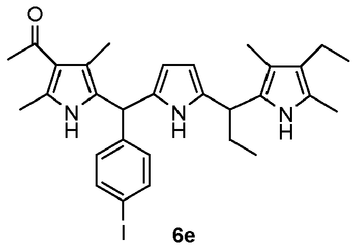

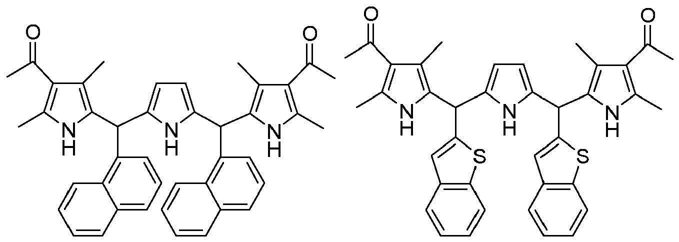

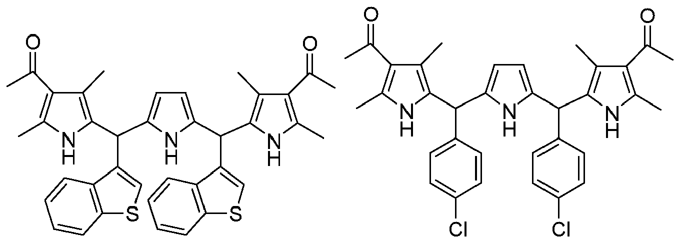

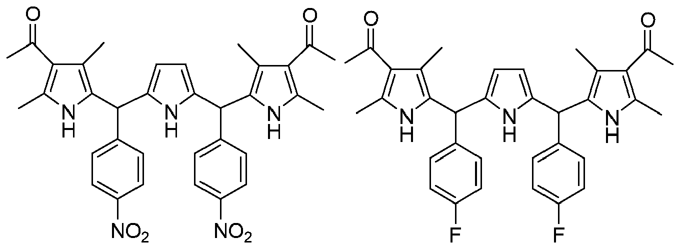

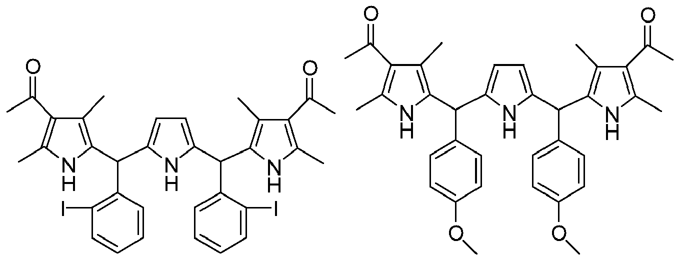

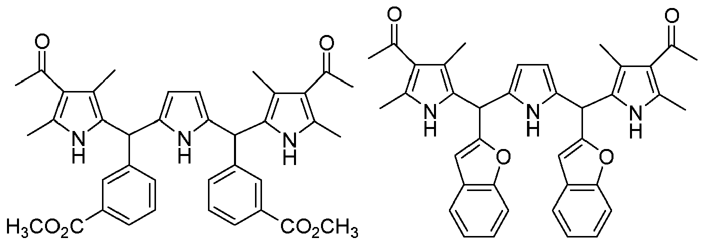

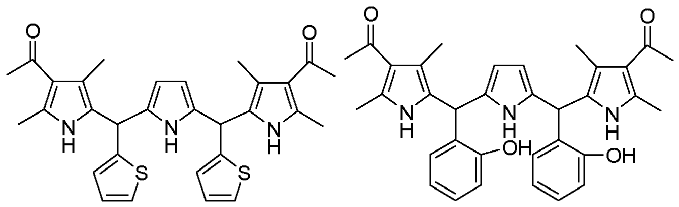

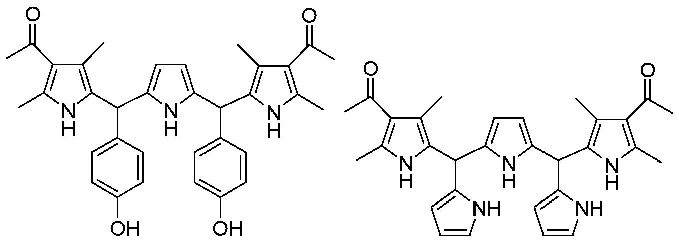

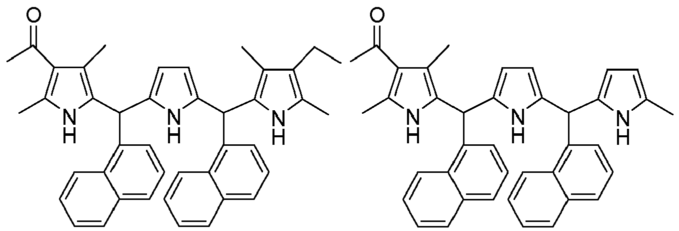

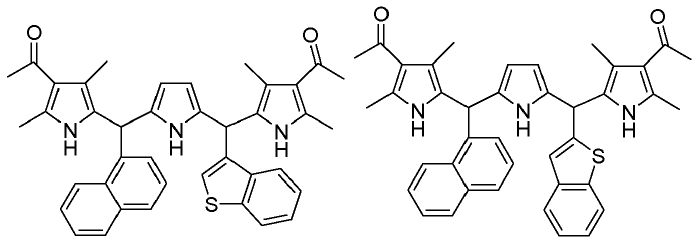

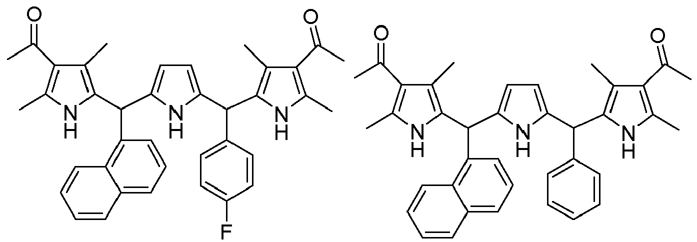

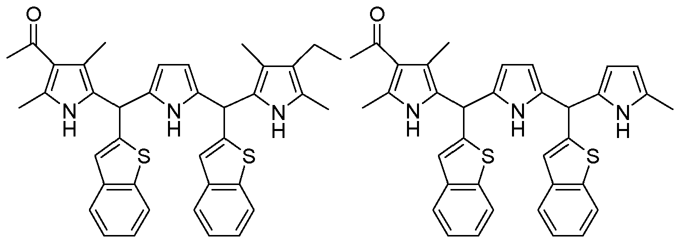

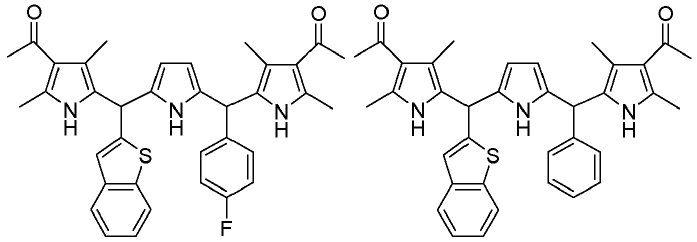

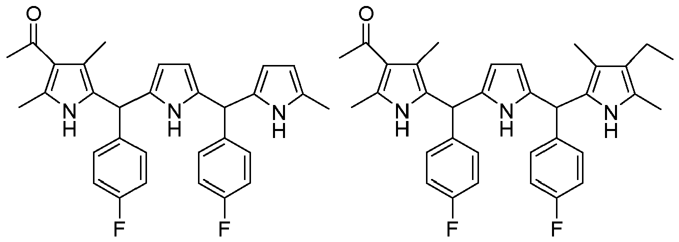

In an advantageous embodiment, the present invention relates to compounds of formula I defined above, for use as an antibacterial drug against infection(s) caused by bacteria of the genus *Staphylococcus,* in particular *Staphylococcus aureus* species, wherein said bacteria of the genus *Staphylococcus,* in particular *Staphylococcus aureus* species, are *Staphylococcus aureus* strains susceptible or resistant to β-lactams (including methicillin-resistant strains also referred as MRSA), and/or susceptible or resistant to glycopeptides (including vancomycin-intermediate or glycopeptides- intermediate *Staphylococcus aureus* strains also referred as VISA or GISA), or multi-drug resistant *Staphylococcus aureus* strains.

Compounds of the invention present the advantage to be active against *Staphylococcus aureus* strains that are multi-resistant, *i.e*., resistant to several classes of antibiotics including those cited above.

In an advantageous embodiment, the present invention relates to compounds of formula I defined above, for use as an antibacterial drug against infection(s) caused by bacteria of the genus *Staphylococcus,* in particular *Staphylococcus aureus* species, wherein said bacteria of the genus *Staphylococcus aureus* belong to the group comprising:
- *Staphylococcus aureus* ATCC 25923,
- *Staphylococcus aureus* ATCC 29213,
- *Staphylococcus aureus* ATCC 9144,
- *Staphylococcus aureus* ATCC 6538,
- *Staphylococcus aureus* CIP 65.6,
- *Staphylococcus aureus* CIP 103428,
- *Staphylococcus aureus* CIP 65.25 MRSA strain,
- *Staphylococcus aureus* ATCC 33592, MRSA strain resistant to aminoglycosides including gentamicin,
- *Staphylococcus aureus* ST 398, MRSA strain,
- *Staphylococcus aureus* USA 300, MRSA strain,
- *Staphylococcus aureus* CIP 106414, VISA/GISA strain,and
- *Staphylococcus aureus* SA-1199B, strain resistant to fluoroquinolones due to NorA efflux pump overproduction.

In an advantageous embodiment, the present invention relates to compounds of formula I defined above, for use as an antibacterial drug against infection(s) caused by bacteria of the genus *Staphylococcus,* in particular *Staphylococcus aureus* species, wherein said infection(s) caused by bacteria of the genus *Staphylococcus,* in particular *Staphylococcus aureus* species, belong to the group comprising:
- pimples,
- impetigo,
- furuncles,
- cellulitis folliculitis,
- carbuncles,
- scalded skin syndrome,
- abscesses,
- suppurations
- pneumonia, pleuro-pneumonia
- urinary tract infections, pyelonephritis
- peritonitis
- prostatitis
- meningitis,
- meningo-encephalitis
- endophthalmitis
- osteomyelitis,
- endocarditis, myocarditis, pericarditis
- toxic shock syndrome (TSS),
- bacteremia, and
- sepsis.

In an advantageous embodiment, the present invention relates to compounds of formula I defined above, for use as an antibacterial drug against infection(s) caused by *Staphylococcus aureus* species, wherein said compounds are not active against bacteria of the species *Staphylococcus epidermidis.*

In an advantageous embodiment, the present invention relates to compounds of formula I defined above, for use as an antibacterial drug against infection(s) caused by *Staphylococcus aureus* species, wherein said bacteria of the species *Staphylococcus epidermidis* belong to the group comprising:
- *Staphylococcus epidermidis ATCC 12228,*
- *Staphylococcus epidermidis CIP 81.55,*
- *Staphylococcus epidermidis CIP 103627.*

In an advantageous embodiment, the present invention relates to a combination of a compound of formula I defined above and an antibiotic selected from the group comprising beta-lactams, aminoglycosides, tetracyclines, glycylcyclines, macrolides, azalides, ketolides, synergistins, lincosanides, fluoroquinolones, phenicols, rifamycins, sulfamides, trimethoprim, glycopeptides, oxazolidinones and lipopeptides, for simultaneous, separated or sequential use in treatment of said infection(s) caused by bacteria of the species *Staphylococcus aureus.*

In another aspect, the present invention relates to a pharmaceutical composition comprising a compound of following formula I: 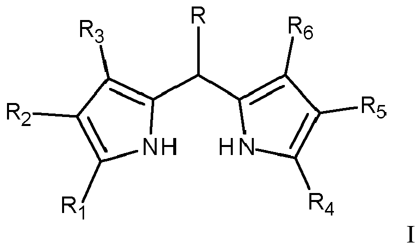 wherein:
R₁, R₂, R₃, R₄, R₅ and R₆ represent independently from each other:
   - a H,
   - a linear or branched (C₁-C₇)-alkyl,
   - a linear or branched (C₂-C₇)-alkenyl,
   - a linear or branched (C₂-C₇)-alkynyl,
   - a (C₃-C₇)-cycloalkyl,
   - a (C₅-C₇)-cycloalkenyl,
   - a (C₃-C₇)-heterocycloalkyl,
   - a (C₅-C₇)-heterocycloalkenyl,
   - an aryl, said aryl being an aromatic or heteroaromatic group,
   - an alkyl aryl, wherein the aryl is an aromatic or heteroaromatic group,
   - a CHO,
   - a CO-(C₁-C₇)-alkyl,
   - a CO-aryl, wherein aryl is an aromatic or heteroaromatic group,
   - a CO₂H,
   - a CO₂-(C₁-C₇)-alkyl,
   - a CONH-(C₁-C₇)-alkyl,
   - a halogen selected from the group comprising F, Cl, Br, and I,
   - CF₃,
   - ORₐ, wherein Rₐ represents:
      H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
   - NR_{b}R_{c}, wherein R_{b} and R_{c} represent independently from each other:
      H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
   - NO₂,
   - CN,
R represents:
   - a H,
   - a linear or branched (C₁-C₇)-alkyl,
   - a linear or branched (C₂-C₇)-alkenyl,
   - a linear or branched (C₂-C₇)-alkynyl,
   - a (C₃-C₇)-cycloalkyl,
   - a (C₅-C₇)-cycloalkenyl,
   - a (C₃-C₇)-heterocycloalkyl,
   - a (C₅-C₇)-heterocycloalkenyl,
   - an aryl, said aryl being an aromatic or heteroaromatic group,
   - an alkyl aryl, wherein the aryl is an aromatic or heteroaromatic group,
   - a CHO,
   - a CO-(C₁-C₇)-alkyl,
   - a CO-aryl, wherein aryl is an aromatic or heteroaromatic group,
   - a CO₂H,
   - a CO₂-(C₁-C₇)-alkyl,
   - a CO₂-aryl, wherein aryl is an aromatic or heteroaromatic group,
   - a CONH-(C₁-C₇)-alkyl,
   said R₁, R₂, R₃, R₄, R₅, R₆ and R being substituted or not by one or more substituent(s), each independently selected from:
   - a linear or branched (C₁-C₇)-alkyl,
   - a linear or branched (C₂-C₇)-alkenyl,
   - a linear or branched (C₂-C₇)-alkynyl,
   - a (C₃-C₇)-cycloalkyl,
   - a (C₅-C₇)-cycloalkenyl,
   - a (C₃-C₇)-heterocycloalkyl,
   - a (C₅-C₇)-heterocycloalkenyl,
   - an aryl, wherein the aryl is an aromatic or heteroaromatic group,
   - an alkyl aryl, wherein the aryl is an aromatic or heteroaromatic group,
   - a CHO,
   - a CO-(C₁-C₇)-alkyl,
   - a CO-aryl, wherein aryl is an aromatic or heteroaromatic group,
   - a CO₂H,
   - a CO₂-(C₁-C₇)-alkyl,
   - a CONH-(C₁-C₇)-alkyl,
   - a halogen selected from the group comprising F, Cl, Br, and I,
   - CF₃,
   - ORₐ, wherein Rₐ represents:
      H, a linear or branched (C₁-C₇)-alkyl, a (C₃-C₇)-cycloalkyl, CO-(C₁-C₇)-alkyl, or
      CO-aryl, wherein aryl is an aromatic or heteroaromatic group,
   - NR_{b}R_{c}, wherein R_{b} and R_{c} represent independently from each other:
      H, a linear or branched (C₁-C₇)-alkyl, a (C₃-C₇)-cycloalkyl, CO-(C₁-C₇)-alkyl, or
      CO-aryl, wherein aryl is an aromatic or heteroaromatic group,
   - NO₂,
   - CN,
   said (C₁-C₇)-alkyl, (C₂-C₇)-alkenyl, (C₂-C₇)-alkynyl, (C₃-C₇)-cycloalkyl, (C₅-C₇)-cycloalkenyl, (C₃-C₇)-heterocycloalkyl, (C₅-C₇)-heterocycloalkenyl, CO-(C₁-C₇)-alkyl, CO₂-(C₁-C₇)-alkyl, CONH-(C₁-C₇)-alkyl, aryl, alkyl aryl and CO-aryl being substituted or not by one or more substituent(s), each independently selected from:
   - a linear or branched (C₁-C₇)-alkyl,
   - a linear or branched (C₂-C₇)-alkenyl,
   - a linear or branched (C₂-C₇)-alkynyl,
   - a (C₃-C₇)-cycloalkyl,
   - a (C₅-C₇)-cycloalkenyl,
   - a (C₃-C₇)-heterocycloalkyl,
   - a (C₅-C₇)-heterocycloalkenyl,
   - an aryl, wherein the aryl is an aromatic or heteroaromatic group,
   - an alkyl aryl, wherein the aryl is an aromatic or heteroaromatic group,
   - a CHO,
   - a CO-(C₁-C₇)-alkyl,
   - a CO-aryl, wherein aryl is an aromatic or heteroaromatic group,
   - a CO₂H,
   - a CO₂-(C₁-C₇)-alkyl,
   - a CONH-(C₁-C₇)-alkyl,
   - a halogen selected from the group comprising F, Cl, Br, and I,
   - CF₃,
   - ORₐ, wherein Rₐ represents:
      H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
   - NR_{b}R_{c}, wherein R_{b} and R_{c} represent independently from each other:
      H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
   - NO₂,
   - CN,
in association with a pharmaceutically acceptable vehicle.

The expression "pharmaceutically acceptable vehicle" denotes in particular cellulose, starch, benzyl alcohol, polyethylene glycol, gelatin, lactose, polysorbate, magnesium or calcium stearate, xanthan gum, guar, alginate, colloidal silica.

The compositions according to the invention can be used by oral, parenteral, topic, or rectal route or in aerosols.

As solid compositions for oral administration, tablets, pills, gelatin capsules, powders or granules can be used. In these compositions, the active ingredient according to the invention is mixed with one or more inert diluents or adjuvants, such as saccharose, lactose or starch. These compositions can comprise substances other than the diluents, for example a lubricant such as magnesium stearate or a coating intended for controlled release.

As liquid compositions for oral administration, pharmaceutically acceptable solutions, suspensions, emulsions, syrups and elixirs containing inert diluents such as water or paraffin oil can be used. These compositions can also comprise substances other than the diluents, for example wetting products, sweeteners or flavourings.

The compositions for parenteral administration can be sterile solutions or emulsions. As solvent or vehicle, water, propylene glycol, a polyethylene glycol, vegetable oils, in particular olive oil, injectable organic esters, for example ethyl oleate can be used. These compositions can also contain adjuvants, in particular wetting agents, isotoning agents, emulsifiers, dispersants and stabilizers.

The sterilization can be carried out in several ways, for example using a bacteriological filter, by irradiation or by heating. They can also be prepared in the form of sterile solid compositions which can be dissolved at the moment of use in sterile water or any other injectable sterile medium.

The compositions for topical administration can be for example creams, ointments, lotions or aerosols.

The compositions for rectal administration are suppositories or rectal capsules, which, in addition to the active ingredient, contain excipients such as cocoa butter, semi-synthetic glycerides or polyethylene glycols.

The compositions can also be aerosols.

For use in the form of liquid aerosols, the compositions can be stable sterile solutions or solid compositions dissolved at the moment of use in pyrogen-free sterile water, in serum or any other pharmaceutically acceptable vehicle. For use in the form of dry aerosols intended to be directly inhaled, the active ingredient is finely divided and combined with a diluent or hydrosoluble solid vehicle, for example dextran, mannitol or lactose.

In an advantageous embodiment, the present invention relates to a pharmaceutical composition defined above, administrable by oral route at a dose comprised from about 10 mg/kg to about 200 mg/kg.

In an advantageous embodiment, the present invention relates to a pharmaceutical composition defined above, under a form liable to be administrable by oral route at an unitary dose comprised from 100 mg to 1,500 mg, in particular from 100 mg to 1,000 mg, in particular from 100 to 500 mg.

Said pharmaceutical composition can be administered 1 to 4 times per day, preferably 2 or 3 times per day.

In an advantageous embodiment, the present invention relates to a pharmaceutical composition defined above, administrable by intraveinous route at a dose comprised from about 5 µg/kg to about 50 mg/kg.

In an advantageous embodiment, the present invention relates to a pharmaceutical composition defined above, under a form liable to be administrable by intraveinous route at an unitary dose comprised from 0.1 mg to 1000 mg, in particular from 10 mg to 1,000 mg, in particular from 10 to 500 mg, in particular from 10 to 100 mg.

Said pharmaceutical composition can be administered 1 to 4 times per day, preferably 2 or 3 times per day.

In an advantageous embodiment, the present invention relates to a pharmaceutical composition defined above, comprising a compound of formula I, of the following formula I-1: 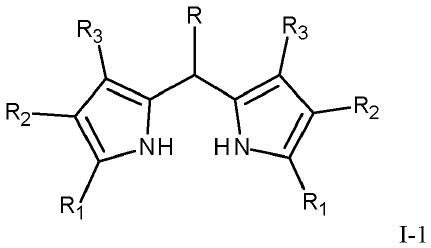 wherein R₁, R₂, R₃ and R are as defined above, R₂ representing preferably CO-CH₃.

In an advantageous embodiment, the present invention relates to a pharmaceutical composition defined above, comprising a compound of formula I, wherein at least one of R₁, R₂ and R₃ is different respectively from R₄, R₅ and R₆.

In an advantageous embodiment, the present invention relates to a pharmaceutical composition defined above, comprising a compound of formula I, wherein at least one of R₁, R₂, R₃, R₄, R₅ and R₆ represents:
- a CO-(C₁-C₇)-alkyl,
- a CO-aryl, wherein aryl is an aromatic or heteroaromatic group,
- CO₂H,
- a CO₂-(C₁-C₇)-alkyl,
- a CONH-(C₁-C₇)-alkyl,
- CF₃,
- NO₂,
- CN.

In an advantageous embodiment, the present invention relates to a pharmaceutical composition defined above, comprising a compound of formula I, wherein R₂ and/or R₅ are/is selected from the group comprising:
- a CO-(C₁-C₇)-alkyl,
- a CO-aryl, wherein aryl is an aromatic or heteroaromatic group,
- CO₂H,
- a CO₂-(C₁-C₇)-alkyl,
- a CONH-(C₁-C₇)-alkyl,
- CF₃,
- NO₂,
- CN.

In an advantageous embodiment, the present invention relates to a pharmaceutical composition defined above, comprising a compound of formula I, wherein R₂ and/or R₅ represent(s) CO-CH₃.

In an advantageous embodiment, the present invention relates to a pharmaceutical composition defined above, comprising a compound of formula I, of the following formula I-1bis: 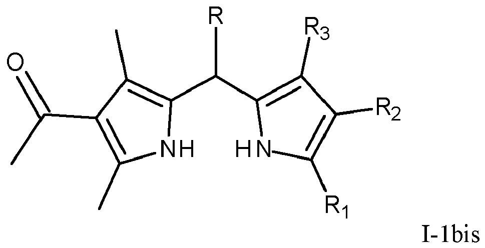 wherein R₁, R₂, R₃ and R are as defined above.

In an advantageous embodiment, the present invention relates to a pharmaceutical composition defined above, comprising a compound of formula I, of the following formula I-2: 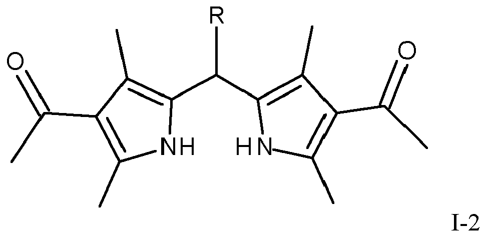 wherein R is as defined above.

In an advantageous embodiment, the present invention relates to a pharmaceutical composition defined above, comprising a compound of formula I, of the following formula I-2bis: 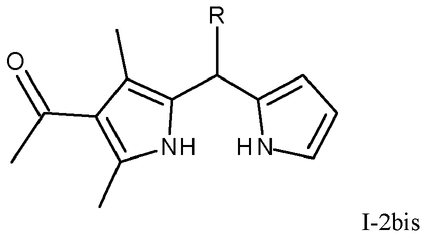 wherein R is as defined above.

In an advantageous embodiment, the present invention relates to a pharmaceutical composition defined above, comprising a compound of formula I, of the following formula I-2ter : 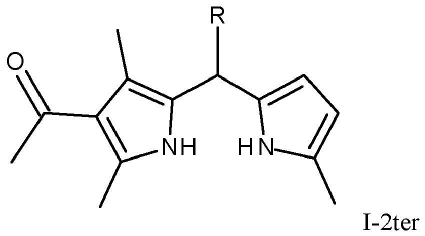 wherein R is as defined above.

In an advantageous embodiment, the present invention relates to a pharmaceutical composition defined above, comprising a compound of formula I, of the following formula I-2quater: 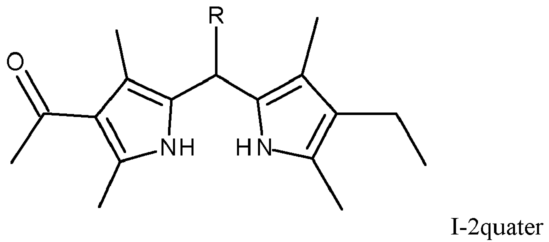 wherein R is as defined above.

In an advantageous embodiment, the present invention relates to a pharmaceutical composition defined above, comprising a compound of formula I, wherein R represents:
- a (C₁-C₇)-alkyl,
- an aryl, said aryl being an aromatic or heteroaromatic group,
   R representing preferably an aryl,
   said (C₁-C₇)-alkyl and aryl being substituted or not by one or more substituent(s), each independently selected from:
   - a linear or branched (C₁-C₇)-alkyl,
   - a linear or branched (C₂-C₇)-alkenyl,
   - a linear or branched (C₂-C₇)-alkynyl,
   - a (C₃-C₇)-cycloalkyl,
   - a (C₅-C₇)-cycloalkenyl,
   - a (C₃-C₇)-heterocycloalkyl,
   - a (C₅-C₇)-heterocycloalkenyl,
   - an aryl, wherein the aryl is an aromatic or heteroaromatic group,
   - an alkyl aryl, wherein the aryl is an aromatic or heteroaromatic group,
   - a CHO,
   - a CO-(C₁-C₇)-alkyl,
   - a CO-aryl, wherein aryl is an aromatic or heteroaromatic group,
   - a CO₂H,
   - a CO₂-(C₁-C₇)-alkyl,
   - a CONH-(C₁-C₇)-alkyl,
   - a halogen selected from the group comprising F, Cl, Br, and I,
   - CF₃,
   - ORₐ, wherein Rₐ represents:
      H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
   - NR_{b}R_{c}, wherein R_{b} and R_{c} represent independently from each other:
      H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
   - NO₂,
   - CN.

In an advantageous embodiment, the present invention relates to a pharmaceutical composition defined above, comprising a compound of formula I, wherein R is selected from the group comprising: 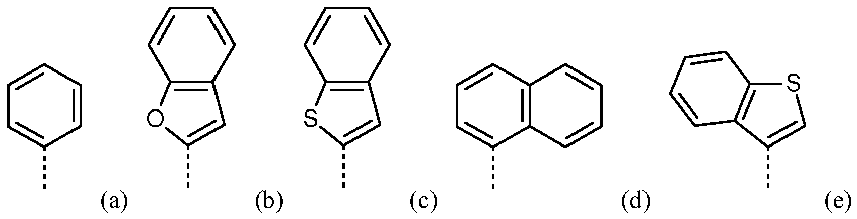 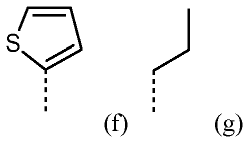 said R being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN.

In an advantageous embodiment, the present invention relates to a pharmaceutical composition defined above, comprising a compound of formula I, of the following formula I-3: 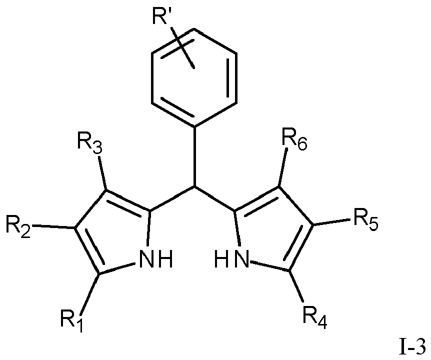 wherein R₁, R₂, R₃, R₄, R₅ and R₆ are as defined above, R₂ and R₅ representing preferably CO-CH₃, and
wherein R' represents H, or one or more substituent(s), preferably one substituent, each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN.

In an advantageous embodiment, the present invention relates to a pharmaceutical composition defined above, comprising a compound of formula I, of the following formula I-4: 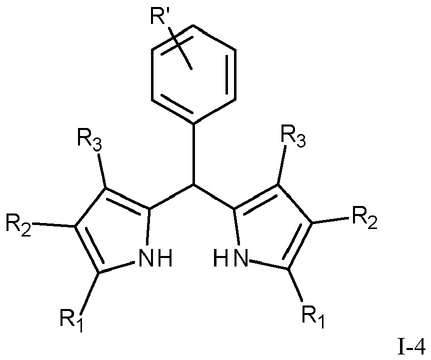 wherein R₁, R₂, R₃, and R' are as defined above.

In an advantageous embodiment, the present invention relates to a pharmaceutical composition defined above, comprising a compound of formula I, of the following formula I-5: 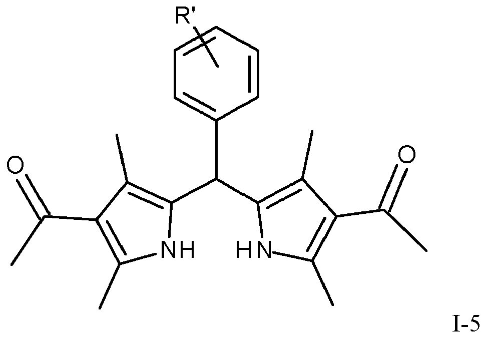 wherein R' is as defined above.

In an advantageous embodiment, the present invention relates to a pharmaceutical composition defined above, comprising a compound of formula I, of the following formula I-6: 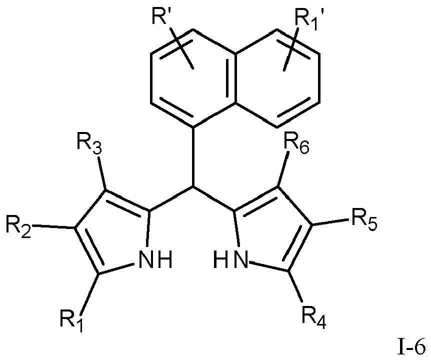 wherein R₁, R₂, R₃, R₄, R₅ and R₆ are as defined above, R₂ and R₅ representing preferably CO-CH₃, and
wherein R' and R₁' represent, independently from each other, H or one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
preferably, R' represents H and R₁' represents one substituent, or R' represents one substituent and R₁' represents H.

In an advantageous embodiment, the present invention relates to a pharmaceutical composition defined above, comprising a compound of formula I, of the following formula I-7: 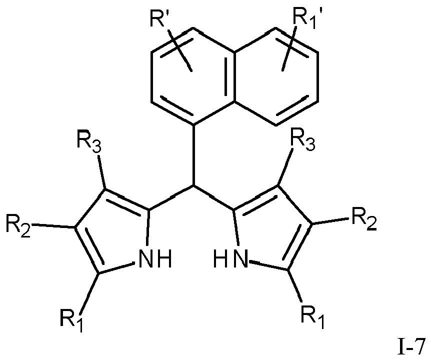 wherein R₁, R₂, R₃, R' and R₁' are as defined above.

In an advantageous embodiment, the present invention relates to a pharmaceutical composition defined above, comprising a compound of formula I, of the following formula I-8: 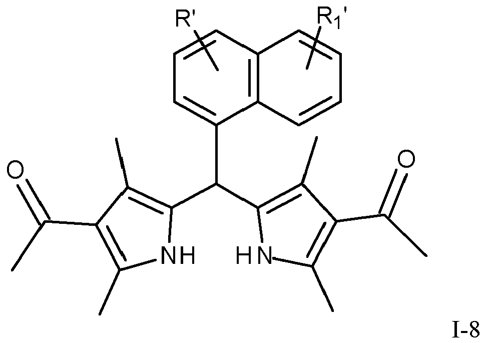 wherein R' and R₁' are as defined above.

In an advantageous embodiment, the present invention relates to a pharmaceutical composition defined above, comprising a compound of formula I, of the following formula I-9: 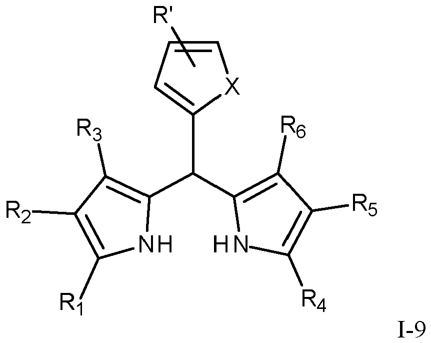 wherein R₁, R₂, R₃, R₄, R₅ and R₆ are as defined above, R₂ and R₅ representing preferably CO-CH₃, and
wherein X is selected from the group comprising O, S and NR₂', X being preferably S, wherein R₂' represents H, a linear or branched (C₁-C₇)-alkyl, a CHO, a CO-(C₁-C₇)-alkyl, a CO-aryl, wherein aryl is an aromatic or heteroaromatic group, a CO₂-(C₁-C₇)-alkyl, a CONH-(C₁-C₇)-alkyl, a CF₃, and
wherein R' represents H, or one or more substituent(s), preferably one substituent, each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN.

In an advantageous embodiment, the present invention relates to a pharmaceutical composition defined above, comprising a compound of formula I, of the following formula I-10: 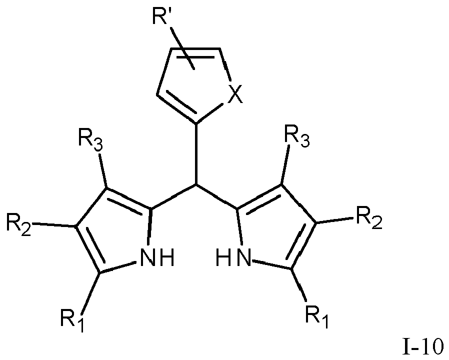 wherein R₁, R₂, R₃, R' and X are as defined above.

In an advantageous embodiment, the present invention relates to a pharmaceutical composition defined above, comprising a compound of formula I, of the following formula I-11: 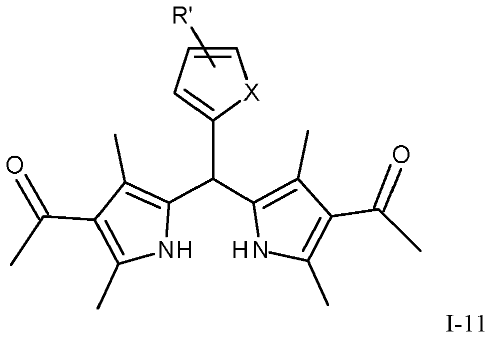 wherein R' and X are as defined above.

In an advantageous embodiment, the present invention relates to a pharmaceutical composition defined above, comprising a compound of formula I, of the following formula I-9bis: 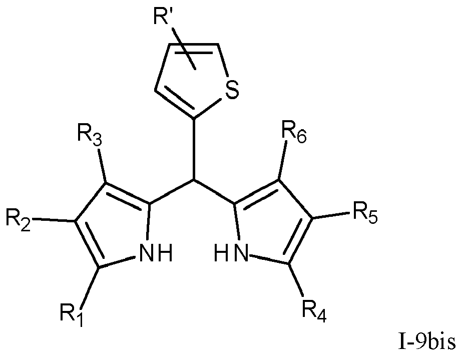 wherein R₁, R₂, R₃, R₄, R₅, R₆ and R' are as defined above.

In an advantageous embodiment, the present invention relates to a pharmaceutical composition defined above, comprising a compound of formula I, of the following formula I-10bis: 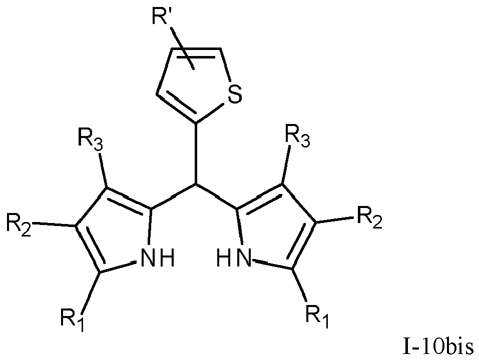 wherein R₁, R₂, R₃, and R' are as defined above.

In an advantageous embodiment, the present invention relates to a pharmaceutical composition defined above, comprising a compound of formula I, of the following formula I-11bis: 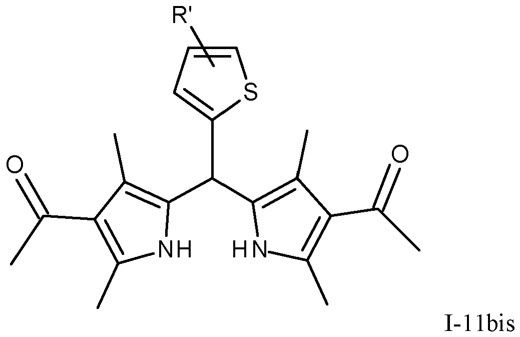 wherein R' is as defined above.

In an advantageous embodiment, the present invention relates to a pharmaceutical composition defined above, comprising a compound of formula I, of the following formula I-12: 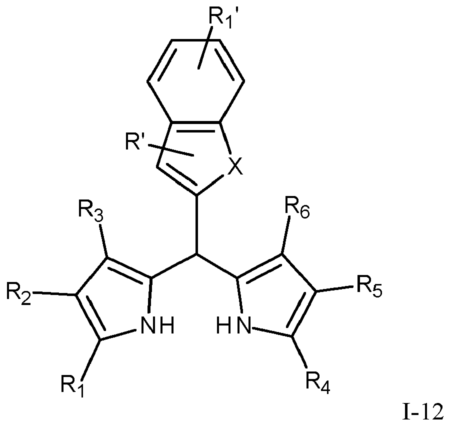 wherein R₁, R₂, R₃, R₄, R₅ and R₆ are as defined above, R₂ and R₅ representing preferably CO-CH₃, and
wherein X is selected from the group comprising O, S and NR₂', X being preferably S or O, wherein R₂' represents H, a linear or branched (C₁-C₇)-alkyl, a CHO, a CO-(C₁-C₇)-alkyl, a CO-aryl, wherein aryl is an aromatic or heteroaromatic group, a CO₂-(C₁-C₇)-alkyl, a CONH-(C₁-C₇)-alkyl, a CF₃, and
wherein R' and R₁' represent, independently from each other, H or one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a CHO,
- a CO-(C₁-C₇)-alkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
preferably, R' represents H and R₁' represents one substituent, or R' represents one substituent and R₁' represents H.

In an advantageous embodiment, the present invention relates to a pharmaceutical composition defined above, comprising a compound of formula I, of the following formula I-13: 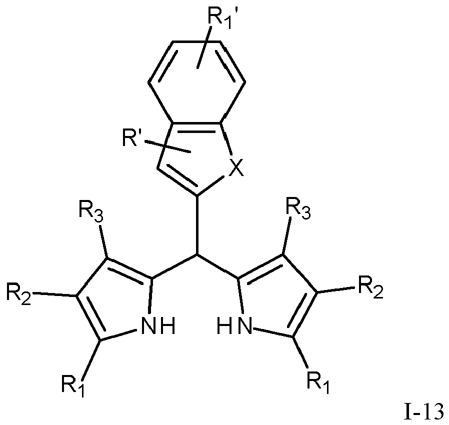 wherein R₁, R₂, R₃, R', R₁' and X are as defined above.

In an advantageous embodiment, the present invention relates to a pharmaceutical composition defined above, comprising a compound of formula I, of the following formula I-14: 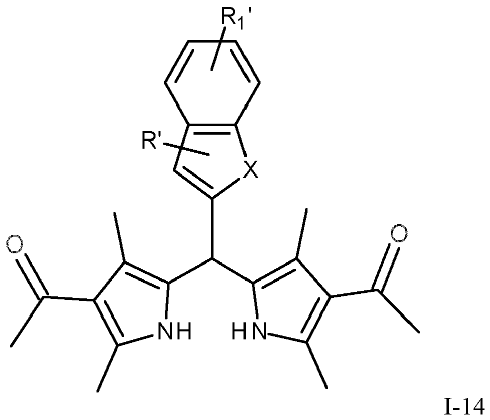 wherein R', R₁' and X are as defined above.

In an advantageous embodiment, the present invention relates to a pharmaceutical composition defined above, comprising a compound of formula I, of the following formula I-12bis: 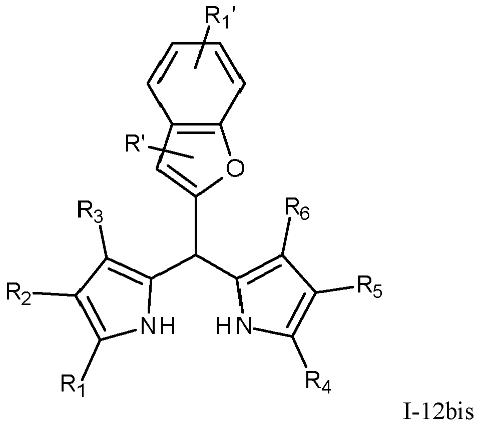 wherein R₁, R₂, R₃, R₄, R₅, R₆, R' and R₁' are as defined above.

In an advantageous embodiment, the present invention relates to a pharmaceutical composition defined above, comprising a compound of formula I, of the following formula I-13bis: 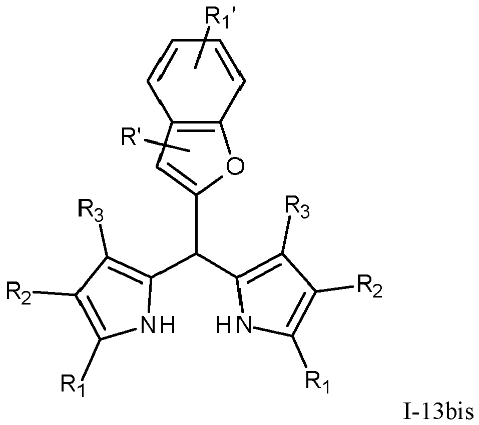 wherein R₁, R₂, R₃, R' and R₁' are as defined above.

In an advantageous embodiment, the present invention relates to a pharmaceutical composition defined above, comprising a compound of formula I, of the following formula I-14bis: 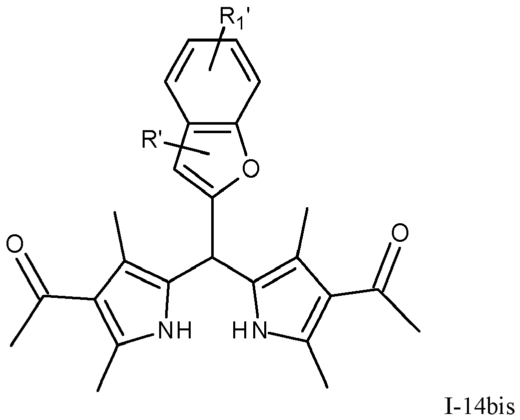 wherein R' and R₁' are as defined above.

In an advantageous embodiment, the present invention relates to a pharmaceutical composition defined above, comprising a compound of formula I, of the following formula I-12ter: 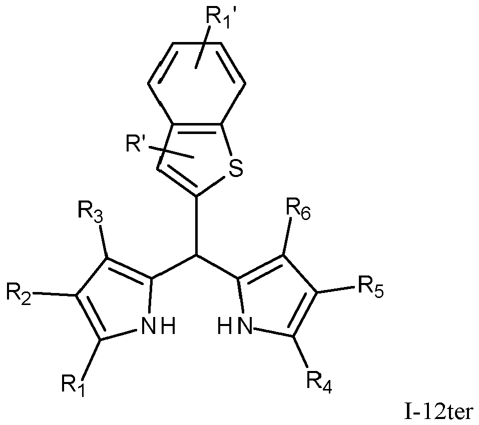 wherein R₁, R₂, R₃, R₄, R₅, R₆, R' and R₁' are as defined above.

In an advantageous embodiment, the present invention relates to a pharmaceutical composition defined above, comprising a compound of formula I, of the following formula I-13ter: 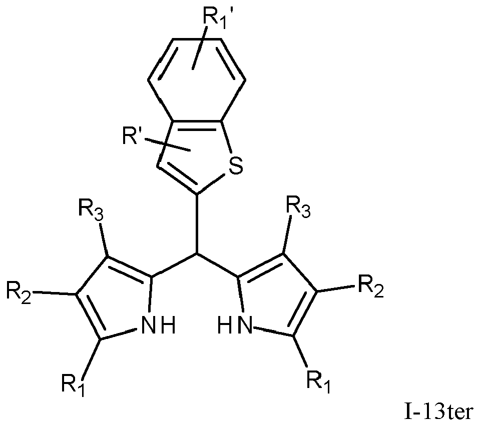 wherein R₁, R₂, R₃, R' and R₁' are as defined above.

In an advantageous embodiment, the present invention relates to a pharmaceutical composition defined above, comprising a compound of formula I, of the following formula I-14ter: 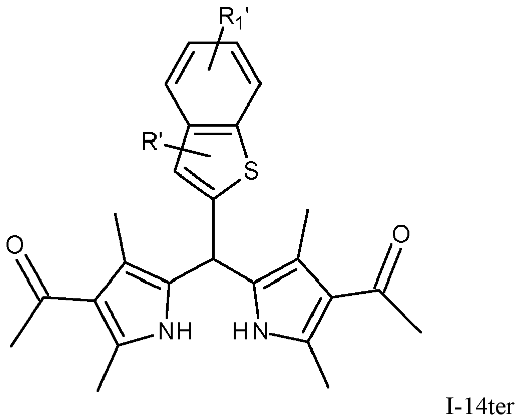 wherein R' and R₁' are as defined above.

In an advantageous embodiment, the present invention relates to a pharmaceutical composition defined above, comprising a compound of formula I, of the following formula I-15: 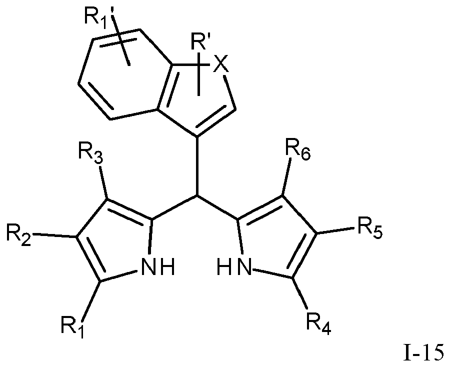 wherein R₁, R₂, R₃, R₄, R₅ and R₆ are as defined above, R₂ and R₅ representing preferably CO-CH₃, and
wherein X is selected from the group comprising O, S and NR₂', X being preferably S,
wherein R₂' represents H, a linear or branched (C₁-C₇)-alkyl, a CHO, a CO-(C₁-C₇)-alkyl, a CO-aryl, wherein aryl is an aromatic or heteroaromatic group, a CO₂-(C₁-C₇)-alkyl, a CONH-(C₁-C₇)-alkyl, a CF₃, and
wherein R' and R₁' represent, independently from each other, H or one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
preferably, R' represents H and R₁' represents one substituent, or R' represents one substituent and R₁' represents H.

In an advantageous embodiment, the present invention relates to a pharmaceutical composition defined above, comprising a compound of formula I, of the following formula I-16: 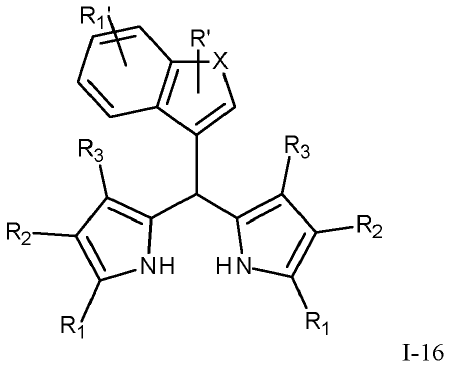 wherein R₁, R₂, R₃, R', R₁' and X are as defined above.

In an advantageous embodiment, the present invention relates to a pharmaceutical composition defined above, comprising a compound of formula I, of the following formula I-17: 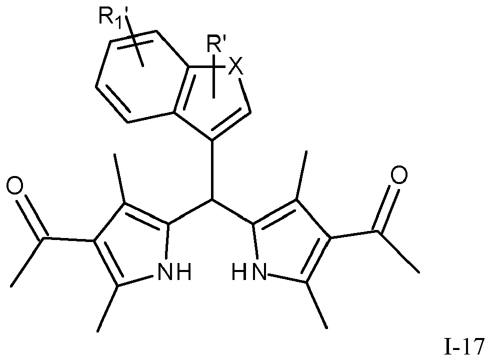 wherein R', R₁' and X are as defined above.

In an advantageous embodiment, the present invention relates to a pharmaceutical composition defined above, comprising a compound of formula I, of the following formula I-15bis: 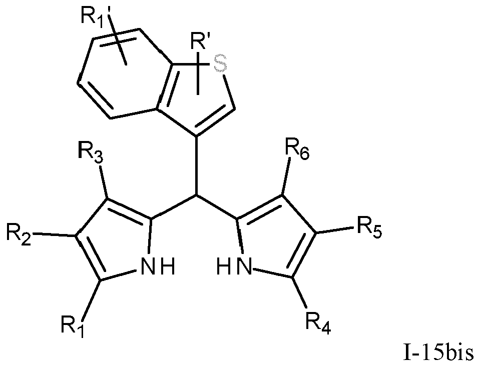 wherein R₁, R₂, R₃, R₄, R₅, R₆, R' and R₁' are as defined above.

In an advantageous embodiment, the present invention relates to a pharmaceutical composition defined above, comprising a compound of formula I, of the following formula I-16bis: 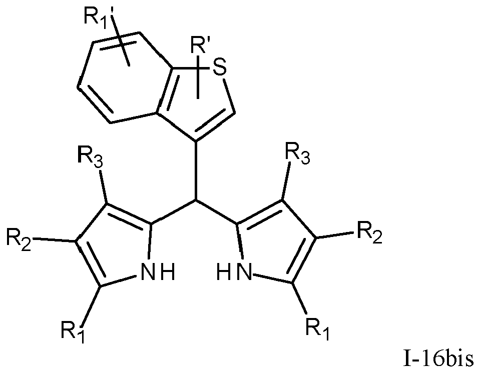 wherein R₁, R₂, R₃, R' and R₁' are as defined above.

In an advantageous embodiment, the present invention relates to a pharmaceutical composition defined above, comprising a compound of formula I, of the following formula I-17bis: 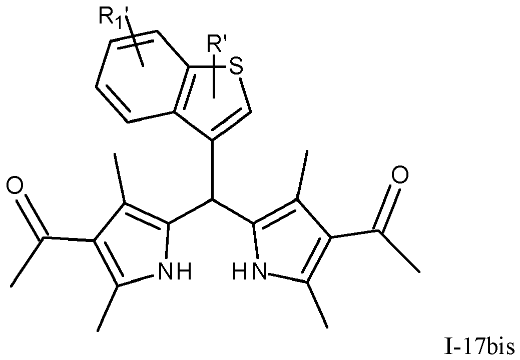 wherein R' and R₁' are as defined above.

In an advantageous embodiment, the present invention relates to a pharmaceutical composition defined above, comprising a compound of formula I, of the following formula I-18: 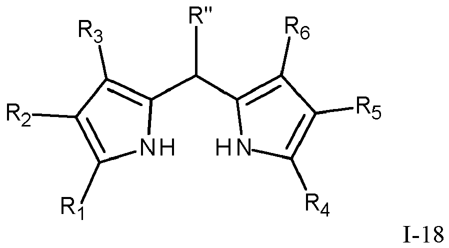 wherein R₁, R₂, R₃, R₄, R₅ and R₆ are as defined above, R₂ and R₅ representing preferably CO-CH₃, and
wherein R" is selected from the aryls of following formulae (a') to (b'): 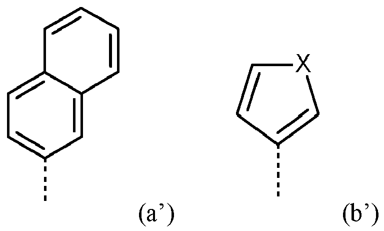 wherein X is selected from the group comprising O, S and NR₂', wherein R₂' represents H, a linear or branched (C₁-C₇)-alkyl, a CHO, a CO-(C₁-C₇)-alkyl, a CO-aryl, wherein aryl is an aromatic or heteroaromatic group, a CO₂-(C₁-C₇)-alkyl, a CONH-(C₁-C₇)-alkyl, a CF₃,
said R" group being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN.

In an advantageous embodiment, the present invention relates to a pharmaceutical composition defined above, comprising a compound of formula I, of the following formula I-19: 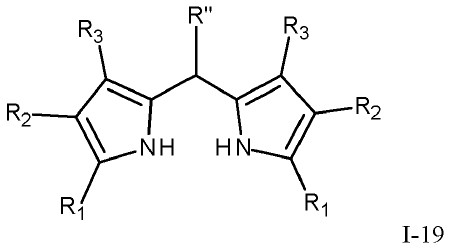 wherein R₁, R₂, R₃, and R" are as defined above.

In an advantageous embodiment, the present invention relates to a pharmaceutical composition defined above, comprising a compound of formula I, of the following formula I-20: 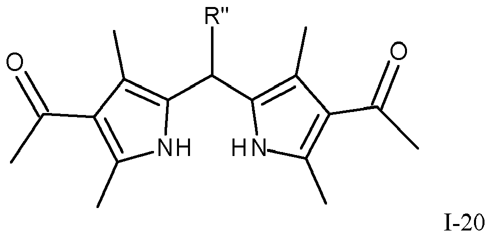 wherein R" is as defined above.

In an advantageous embodiment, the present invention relates to a pharmaceutical composition defined above, comprising a compound of formula I, of the following formula Ibis: 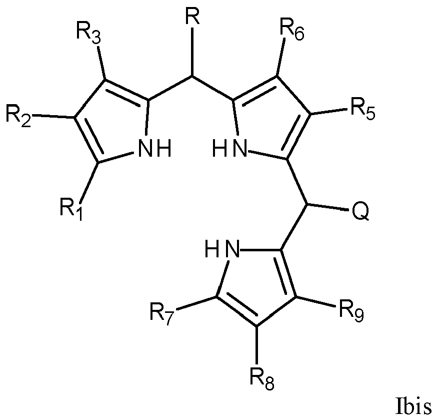 wherein R₁, R₂, R₃, R₅, R₆, and R are as defined above, and wherein R₇, R₈ and R₉ represent independently from each other:
- a H,
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
Q represents:
- a H,
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
said (C₁-C₇)-alkyl, (C₂-C₇)-alkenyl, (C₂-C₇)-alkynyl, (C₃-C₇)-cycloalkyl, (C₅-C₇)-cycloalkenyl, (C₃-C₇)-heterocycloalkyl, (C₅-C₇)-heterocycloalkenyl, CO-(C₁-C₇)-alkyl, CO₂-(C₁-C₇)-alkyl, CONH-(C₁-C₇)-alkyl, aryl, alkyl aryl and CO-aryl being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN.

In an advantageous embodiment, the present invention relates to a pharmaceutical composition defined above, comprising a compound of formula I, of the following formula Ibis-1: 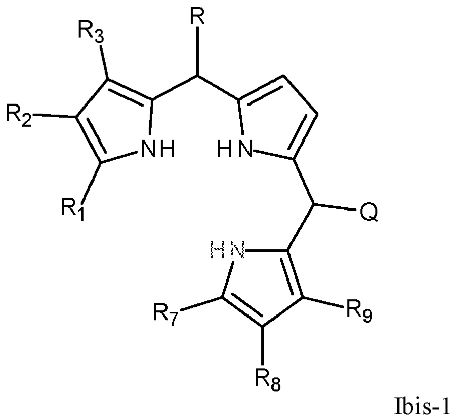 wherein R₁, R₂, R₃, R₇, R₈, R₉, Rand Q are as defined above.

In an advantageous embodiment, the present invention relates to a pharmaceutical composition defined above, comprising a compound of formula I, of the following formula Ibis-2: 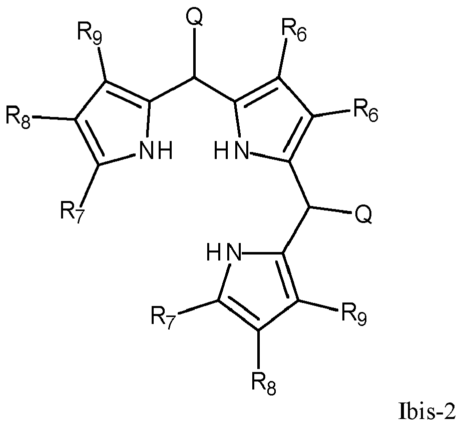 wherein R₆, R₇, R₈, R₉, and Q are as defined above, R₆ representing preferably H.

In an advantageous embodiment, the present invention relates to a pharmaceutical composition defined above, comprising a compound of formula I, of the following formula Ibis-3 : 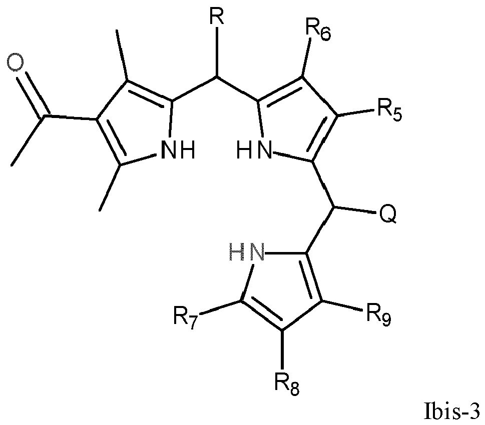 wherein R₅, R₆, R₇, R₈, R₉, R and Q are as defined above, R₅ and/or R₆ representing preferably H.

In an advantageous embodiment, the present invention relates to a pharmaceutical composition defined above, comprising a compound of formula I, of the following formula Ibis-4: 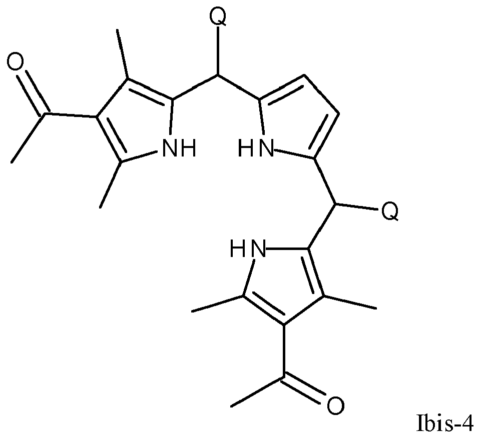 wherein Q is as defined above.

In an advantageous embodiment, the present invention relates to a pharmaceutical composition defined above, comprising a compound of formula I, of the following formula Ibis-5: 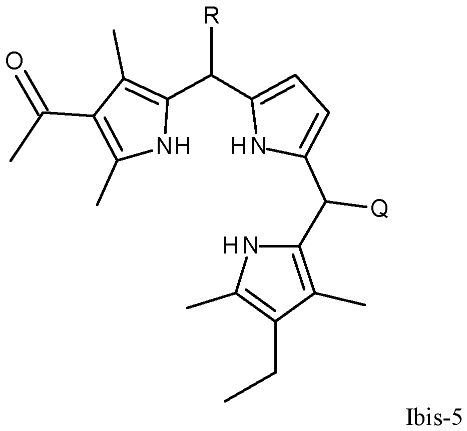 wherein R and Q are as defined above.

In an advantageous embodiment, the present invention relates to a pharmaceutical composition defined above, comprising a compound of formula Ibis, wherein R and Q are, independently from each other, selected from the group comprising: 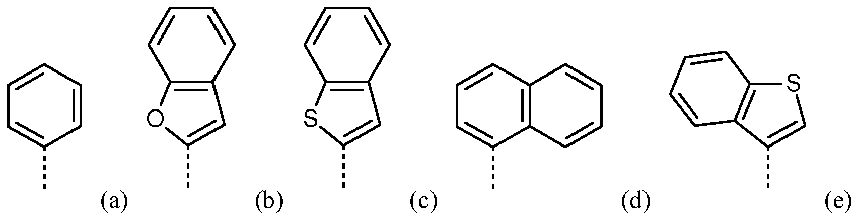 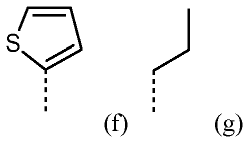 wherein the aryls (a), (b), (c), (d), (e) and (f) are substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN.

In an advantageous embodiment, the present invention relates to a pharmaceutical composition defined above, comprising a compound of formula I, wherein said compound is selected from the group comprising:

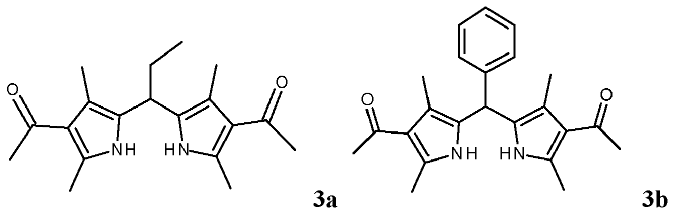

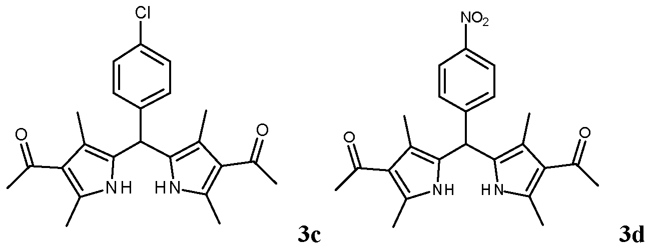

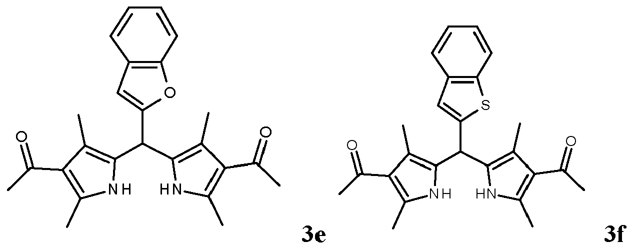

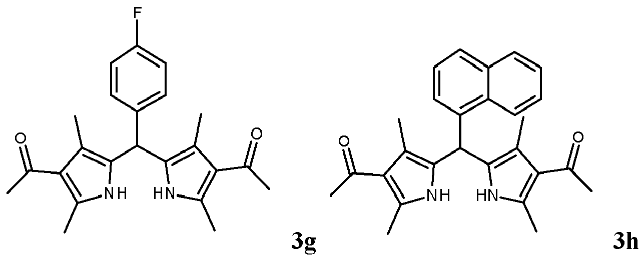

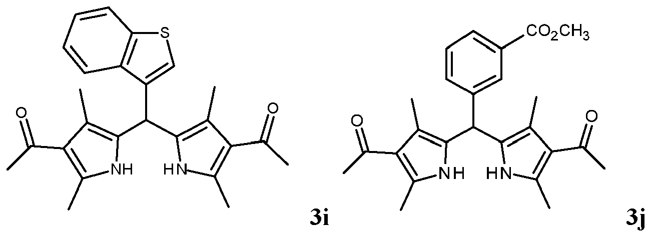

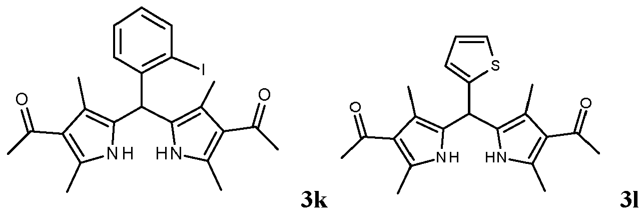

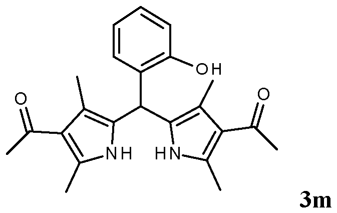

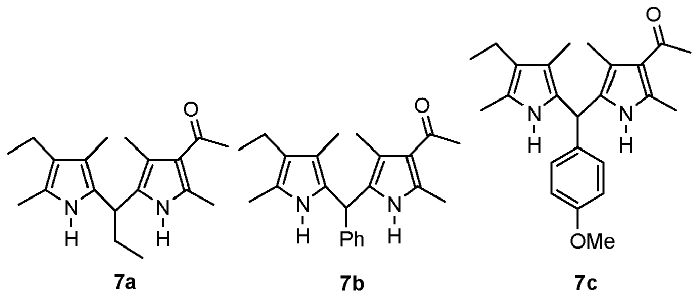

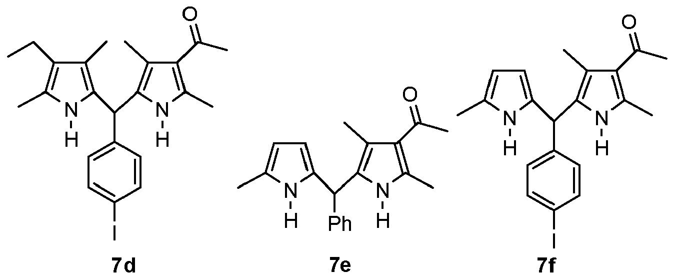

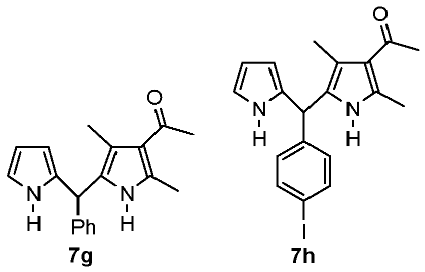

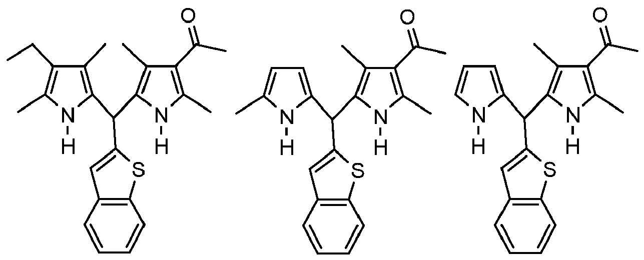

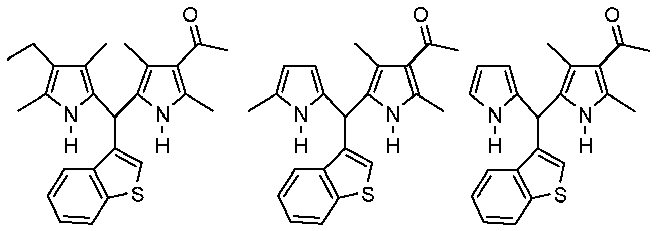

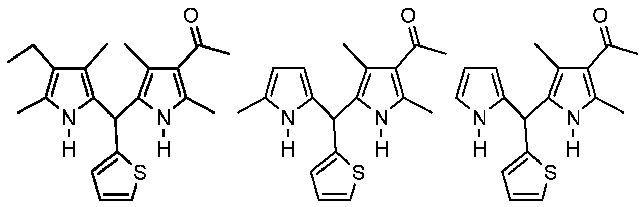

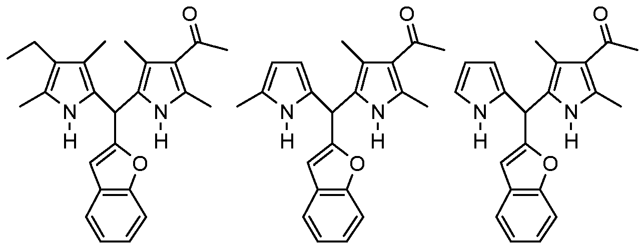

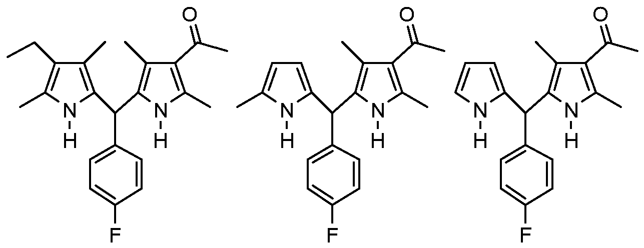

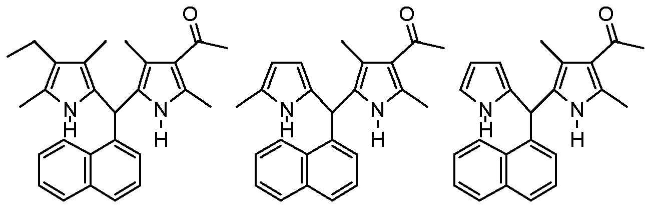

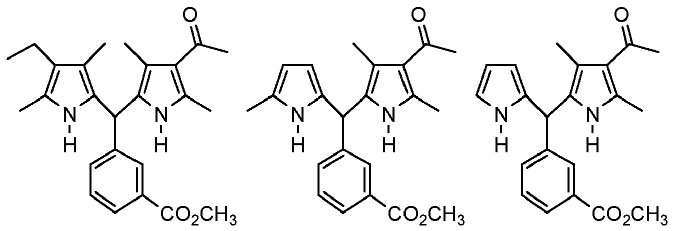

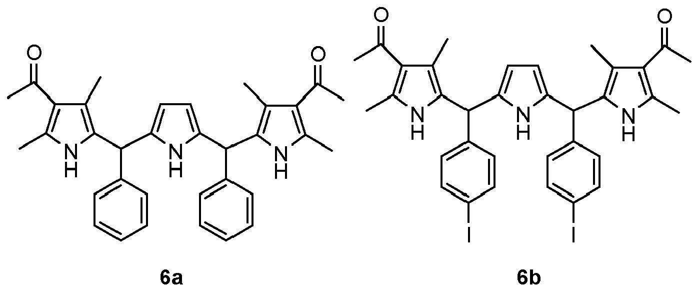

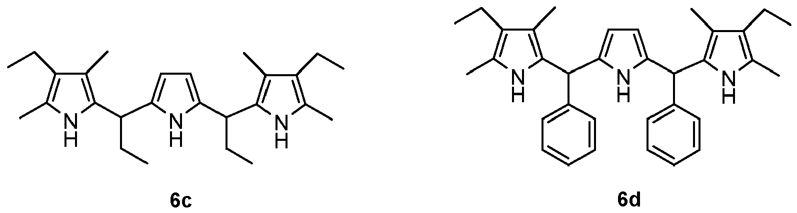

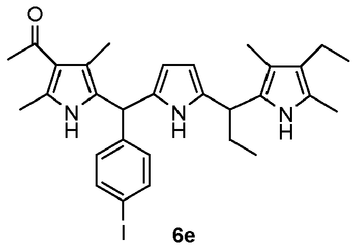

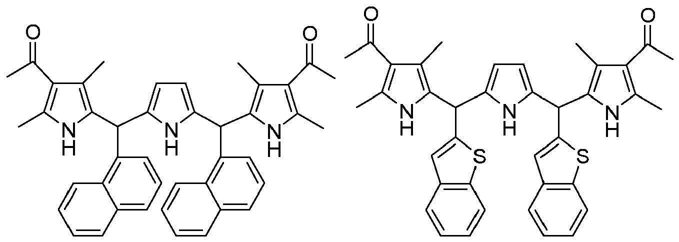

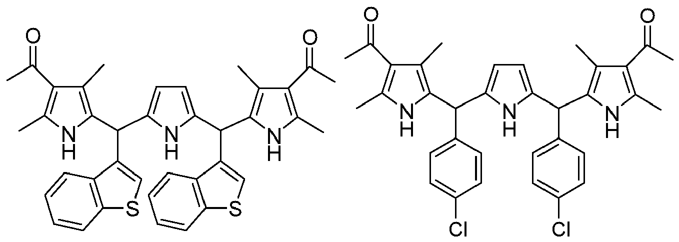

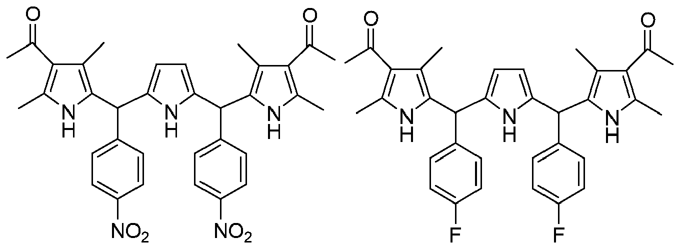

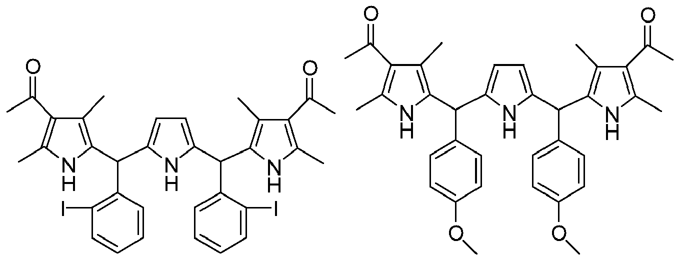

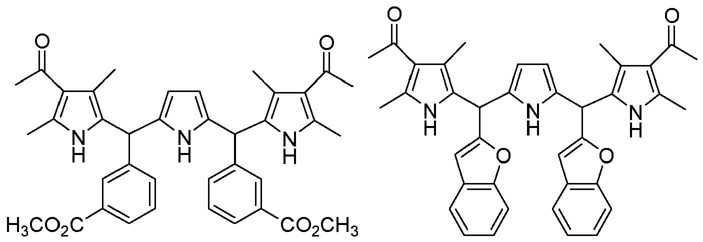

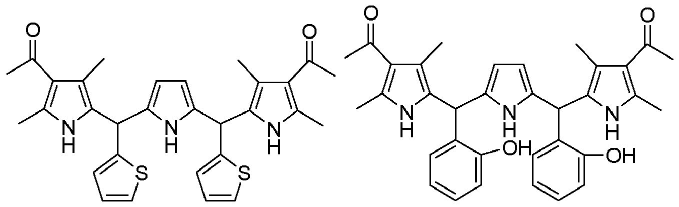

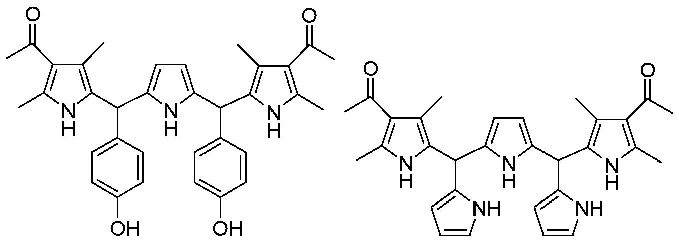

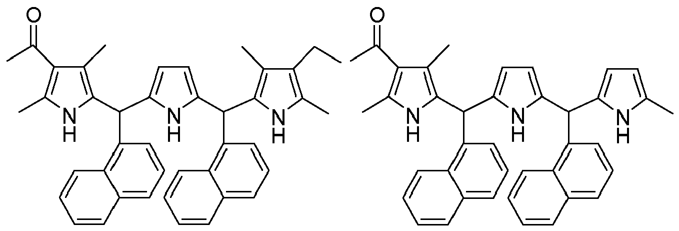

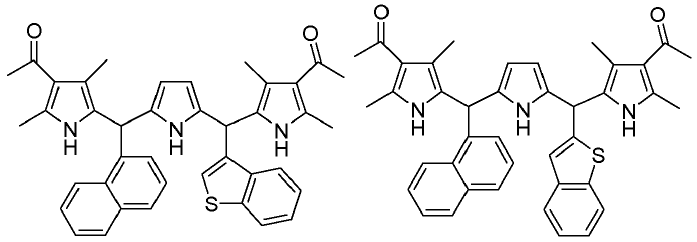

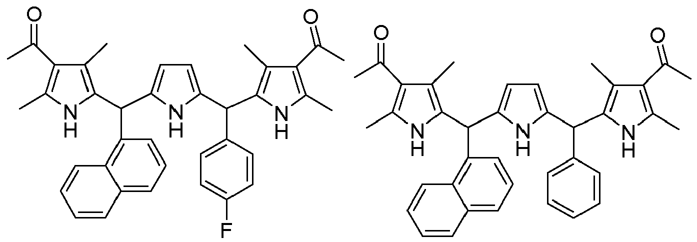

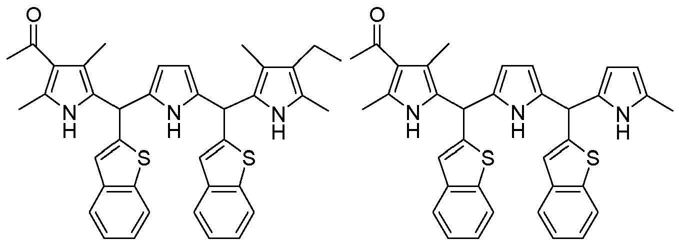

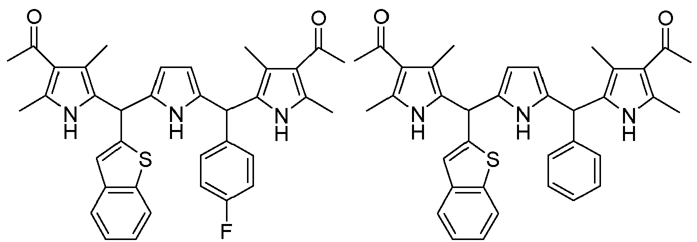

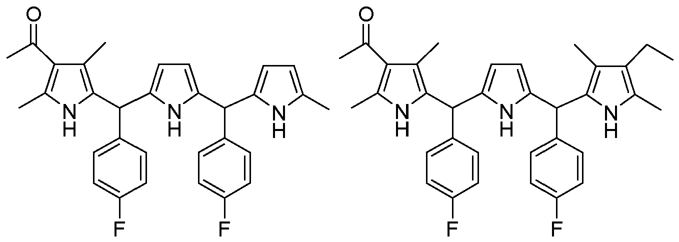

In another aspect, the present invention relates to a pharmaceutical composition comprising, in combination with a pharmaceutically acceptable vehicle:
- at least one compound of formula I as defined above, and
- at least one antibiotic selected from the group comprising beta-lactams, aminoglycosides, tetracyclines, glycylcyclines, macrolides, azalides, ketolides, synergistins, lincosanides, fluoroquinolones, phenicols, rifamycins, sulfamides, trimethoprim, glycopeptides, oxazolidinones and lipopeptides,
said pharmaceutical composition being used for simultaneous or separate use or use spread over time intended for the treatment of pathologies associated with bacterial infections presenting a resistance to an antibacterial compound.

Said antibiotic compound must be from a different family of the one of the compound of the invention.

Administration of a compound of the invention with another antibiotic allows having a broader spectrum or increased activity.

The pharmaceutical composition of the invention as defined above comprises approximately 100 to approximately 6000 mg, preferably approximately 200 to approximately 1500 mg, of compound of formula (I) according to the invention in 1 to 4 administrations per day, preferably in 2 or 3 administrations per day and approximately 100 to approximately 6000 mg, preferably approximately 200 to approximately 1500 mg, of antibiotic compound, in 1 to 4 administrations per day, preferably in 2 or 3 administrations per day.

In another aspect, the present invention relates to products of the following formula I-21: 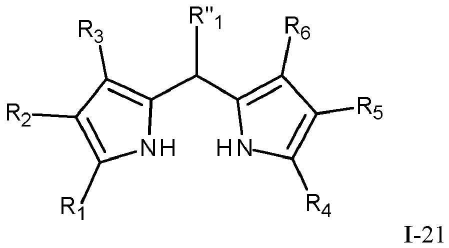 wherein:
R₁, R₂, R₃, R₄, R₅ and R₆ represent independently from each other:
   - a H,
   - a linear or branched (C₁-C₇)-alkyl,
   - a linear or branched (C₂-C₇)-alkenyl,
   - a linear or branched (C₂-C₇)-alkynyl,
   - a (C₃-C₇)-cycloalkyl,
   - a (C₅-C₇)-cycloalkenyl,
   - a (C₃-C₇)-heterocycloalkyl,
   - a (C₅-C₇)-heterocycloalkenyl,
   - an aryl, said aryl being an aromatic or heteroaromatic group,
   - an alkyl aryl, wherein the aryl is an aromatic or heteroaromatic group,
   - a CHO,
   - a CO-(C₁-C₇)-alkyl,
   - a CO-aryl, wherein aryl is an aromatic or heteroaromatic group,
at least one of R₁, R₂ or R₃ representing a CHO, a CO-(C₁-C₇)-alkyl, or a CO-aryl, R"₁ is selected from the aryls of following formulae (a") to (p"): 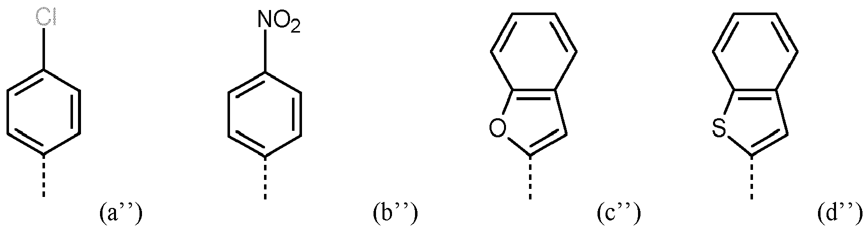 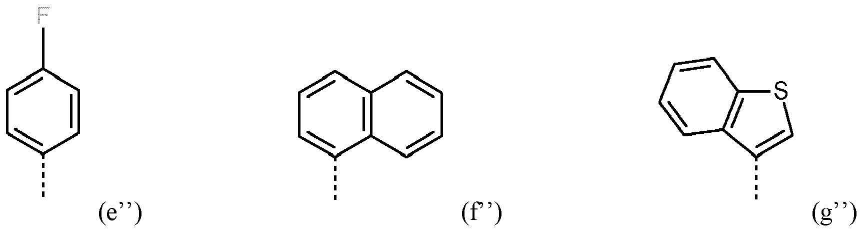 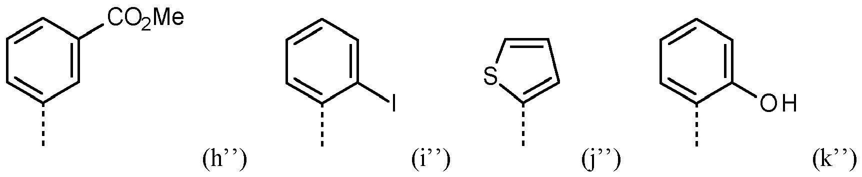 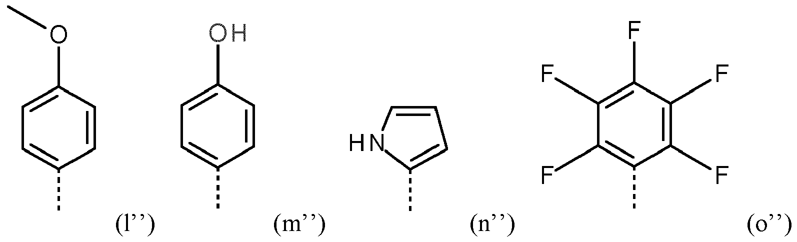 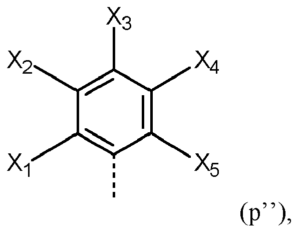 wherein X₁, X₂, X₃, X₄ and X₅ represent independently from each other:
- F, Cl, Br or I, or
- H, F, Cl, Br or I, provided at least one of X₁, X₂, X₃, X₄ and X₅, in particular at least two, in particular at least three, in particular four of X₁, X₂, X₃, X₄ and X₅ do not represent H,
said R₁, R₂, R₃, R₄, R₅, R₆ and R"₁ being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
said (C₁-C₇)-alkyl, (C₁-C₇)-alkenyl, (C₂-C₇)-alkynyl, (C₃-C₇)-cycloalkyl, (C₅-C₇)-cycloalkenyl, (C₃-C₇)-heterocycloalkyl, (C₅-C₇)-heterocycloalkenyl, CO-(C₁-C₇)-alkyl, CO₂-(C₁-C₇)-alkyl, CONH-(C₁-C₇)-alkyl, aryl, alkyl aryl and CO-aryl being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or
   heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
provided that:
- none of R₁, R₂ and R₃ forms a cycle with R₄, R₅ or R₆,
- R₁ and R₄ do not form a cycle with respectively R₂ or R₃, and R₅ or R₆,
- R₂ and R₅ do not form a cycle with respectively R₃ and R₆,
- R₁ and/or R₄ represent(s) an alkyl aryl when R"₁ is selected from groups (1"), (m") and (n"),

Products of formula I-21 are new.

In an advantageous embodiment, the present invention relates to products of formula I-21, wherein at least one of R₁, R₂ and R₃ is different respectively from R₄, R₅ and R₆.

In an advantageous embodiment, the present invention relates to products of formula I-21, of the following formula I-22: 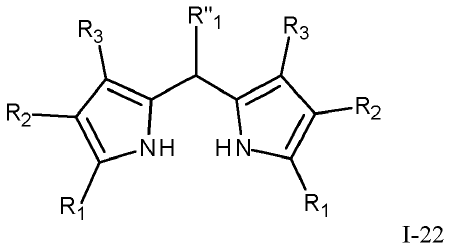 wherein R₁, R₂, R₃ and R"₁ are as defined above,
at least one of R₁, R₂ or R₃ representing a CHO, a CO-(C₁-C₇)-alkyl or a CO-aryl, R₂ representing preferably CO-CH₃.

In an advantageous embodiment, the present invention relates to products of formula I-21, of the following formula I-23: 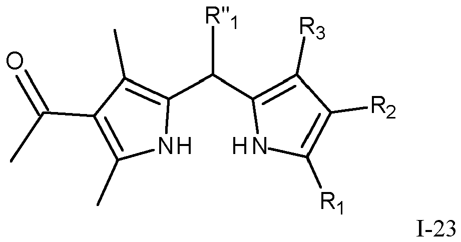 wherein R"₁, R₁, R₂ and R₃ are as defined above.

In an advantageous embodiment, the present invention relates to products of formula I-21, of the following formula I-24: 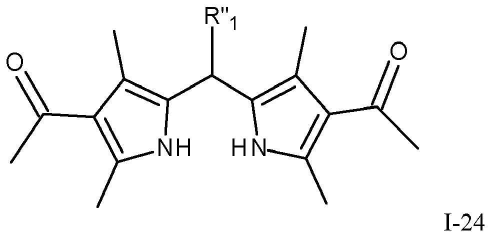 wherein R"₁ is as defined above.

In an advantageous embodiment, the present invention relates to products of formula I-21, of the following formula I-25: 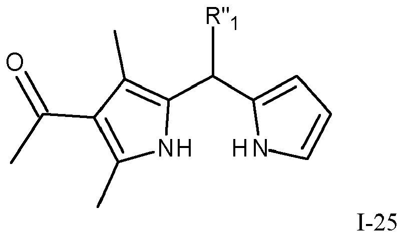 wherein R"₁ is as defined above.

In an advantageous embodiment, the present invention relates to products of formula I-21, of the following formula I-26: 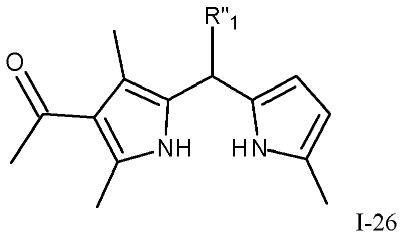 wherein R"₁ is as defined above.

In an advantageous embodiment, the present invention relates to products of formula I-21, of the following formula I-27: 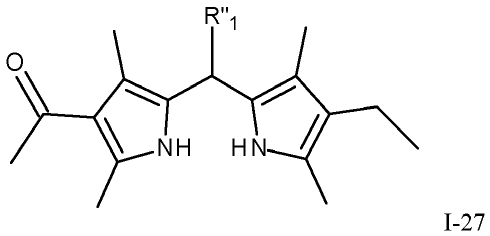 wherein R"₁ is as defined above.

In an advantageous embodiment, the present invention relates to products of formula I-21, of the following formula I-21bis: 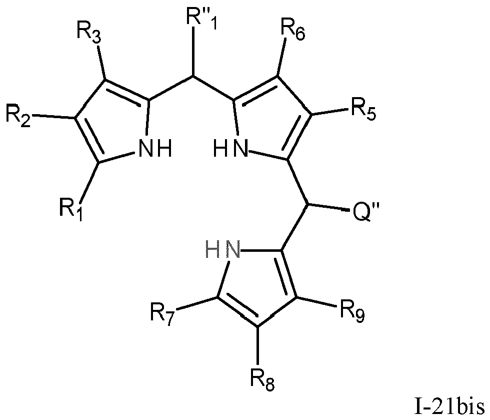 wherein R₁, R₂, R₃, R₅, R₆, and R"₁ are as defined above, and wherein R₇, R₈ and R₉ represent independently from each other:
- a H,
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
Q" represents:
- a H,
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
said (C₁-C₇)-alkyl, (C₂-C₇)-alkenyl, (C₂-C₇)-alkynyl, (C₃-C₇)-cycloalkyl, (C₅-C₇)-cycloalkenyl, (C₃-C₇)-heterocycloalkyl, (C₅-C₇)-heterocycloalkenyl, CO-(C₁-C₇)-alkyl, CO₂-(C₁-C₇)-alkyl, CONH-(C₁-C₇)-alkyl, aryl, alkyl aryl and CO-aryl being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
Q" representing preferably a linear or branched (C₁-C₇)-alkyl or an aryl, more preferably a linear or branched (C₁-C₇)-alkyl or an aryl selected from the aryls of following formulae (a") to (r"): 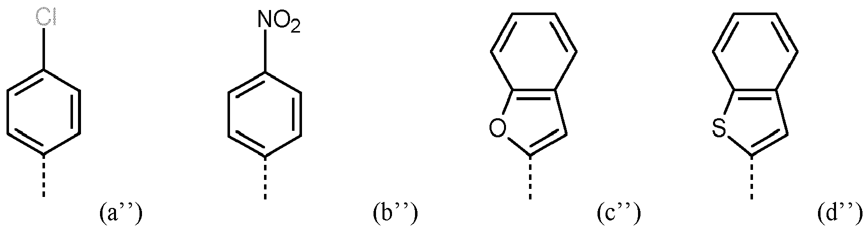 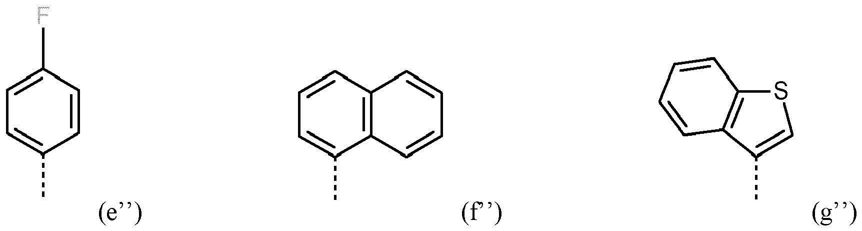 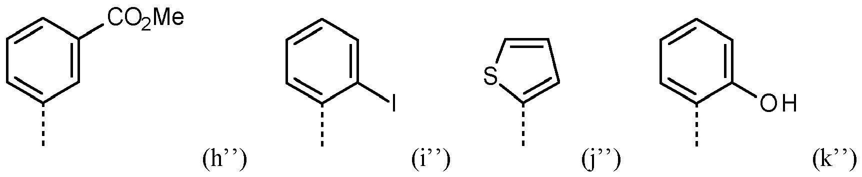 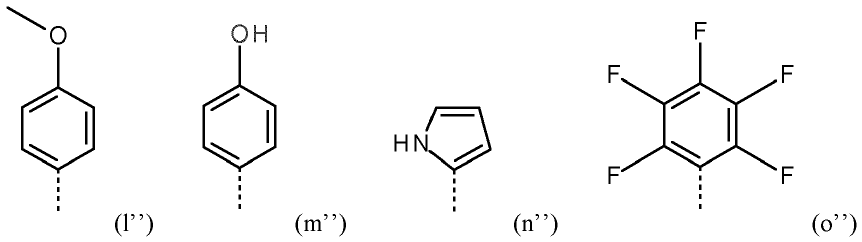 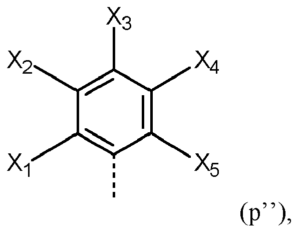 wherein X₁, X₂, X₃, X₄ and X₅ represent independently from each other:
- F, Cl, Br or I, or
- H, F, Cl, Br or I, provided at least one of X₁, X₂, X₃, X₄ and X₅, in particular at least two, in particular at least three, in particular four of X₁, X₂, X₃, X₄ and X₅ do not represent H, 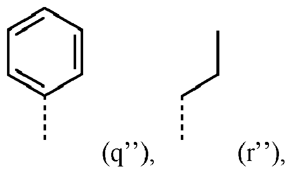
R₅ and R₆ representing preferably H.

In an advantageous embodiment, the present invention relates to products of formula I-21, of the following formula I-28: 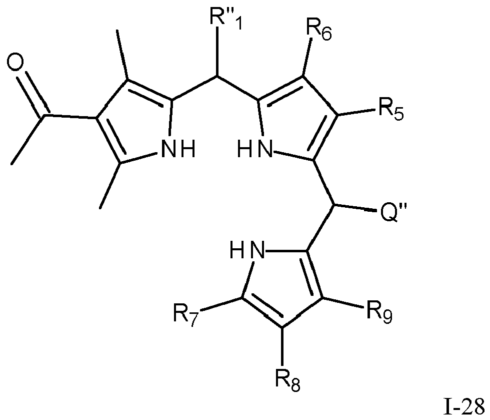 wherein R₅, R₆, R₇, R₈, R₉, R"₁ and Q" are as defined above, R₅ and/or R₆ representing preferably H.

In an advantageous embodiment, the present invention relates to products of formula I-21, of the following formula I-29: 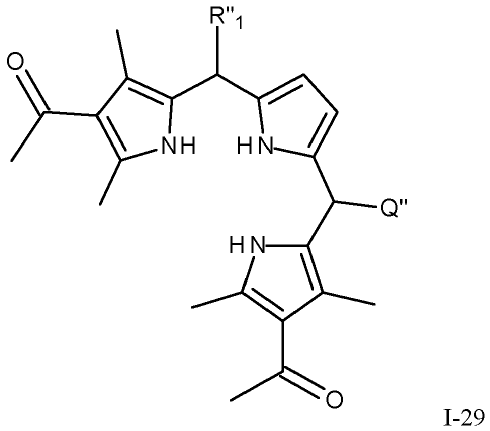 wherein R"₁ and Q" are as defined above.

In an advantageous embodiment, the present invention relates to products of formula I-21, of the following formula I-30: 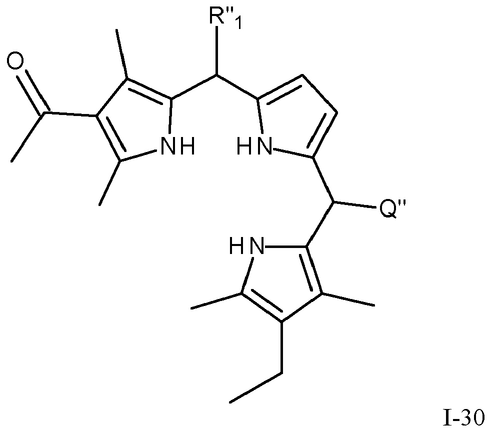 wherein R"₁ and Q" are as defined above.

In an advantageous embodiment, the present invention relates to products of formula I-21, of the following formula I-31: 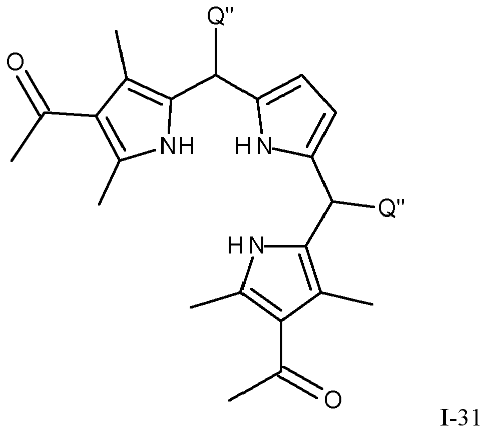 wherein Q" is selected from the aryls of following formulae (a") to (p"): 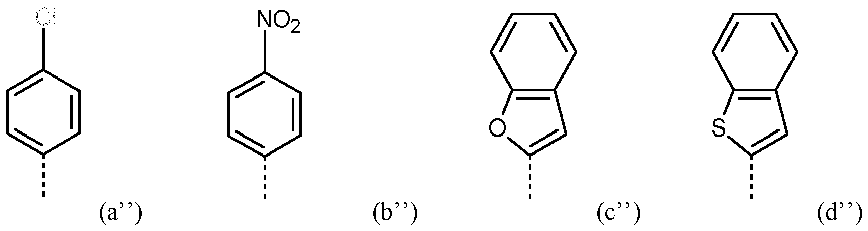 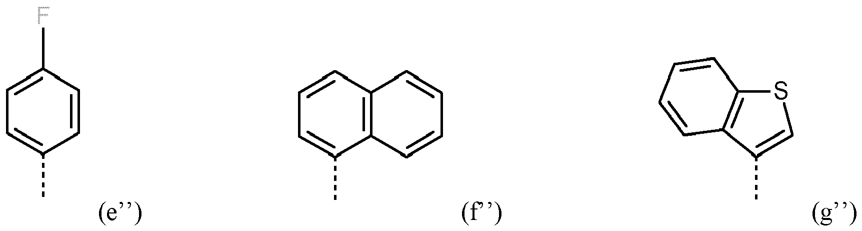 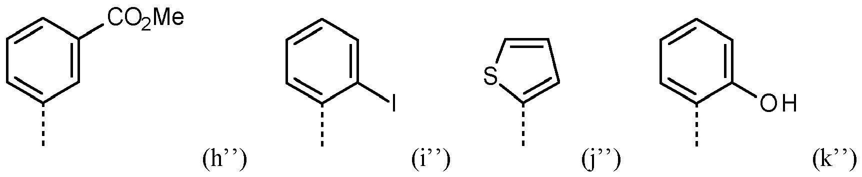 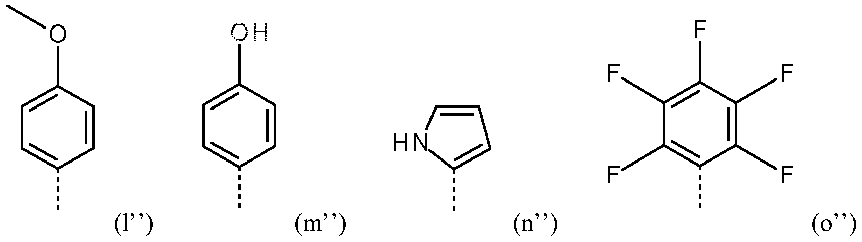 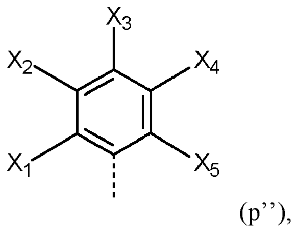 wherein X₁, X₂, X₃, X₄ and X₅ represent independently from each other:
- F, Cl, Br or I, or
- H, F, Cl, Br or I, provided at least one of X₁, X₂, X₃, X₄ and X₅, in particular at least two, in particular at least three, in particular four of X₁, X₂, X₃, X₄ and X₅ do not represent H,
said Q" being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN.

In an advantageous embodiment, the present invention relates to products of formula I-21, selected from the group comprising :

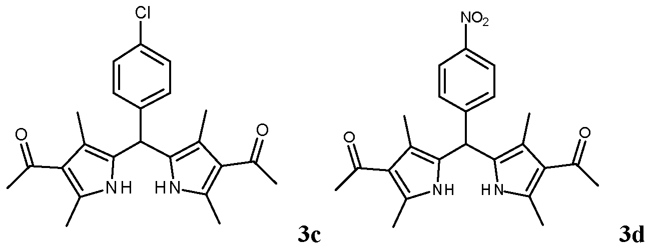

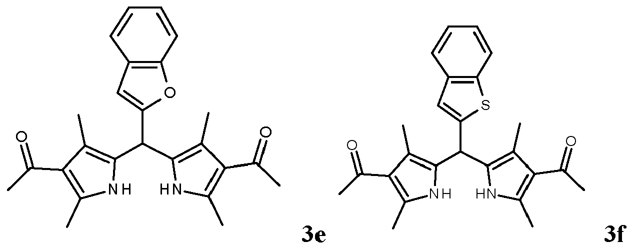

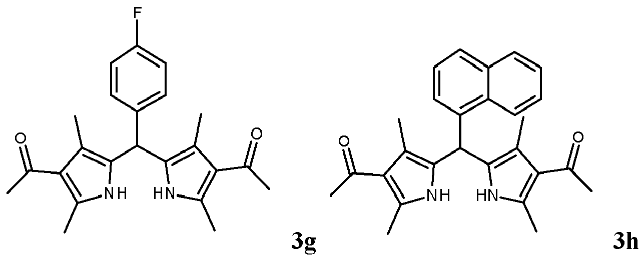

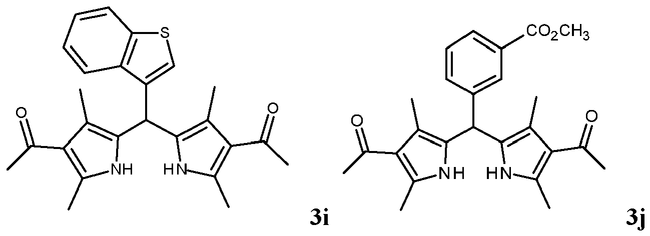

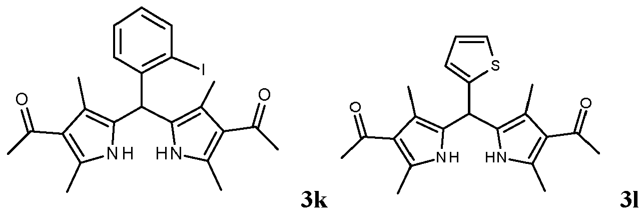

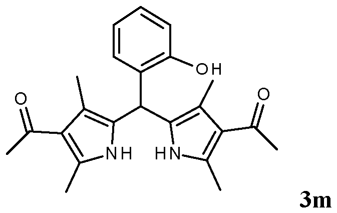

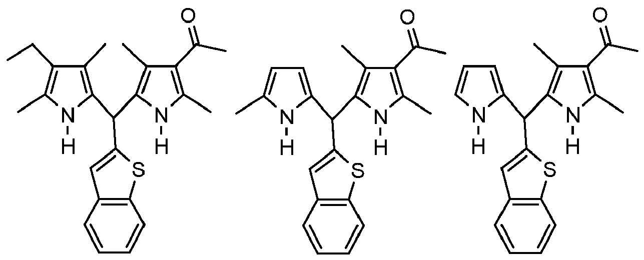

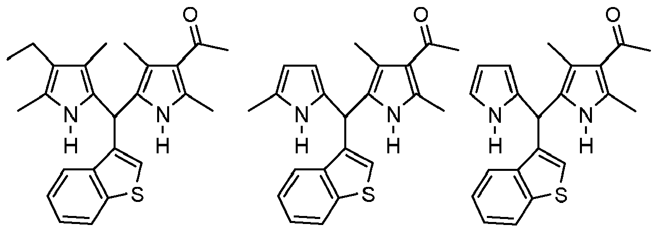

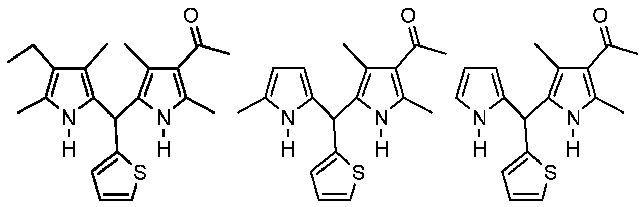

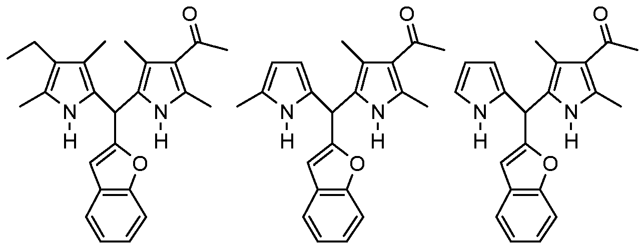

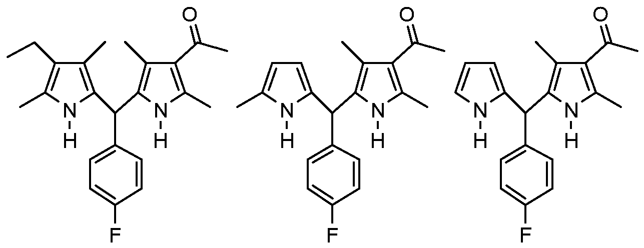

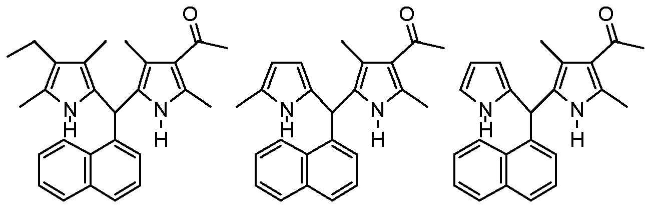

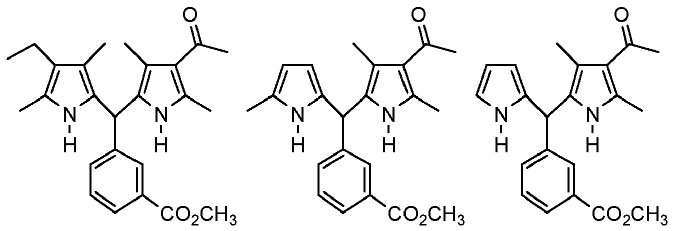

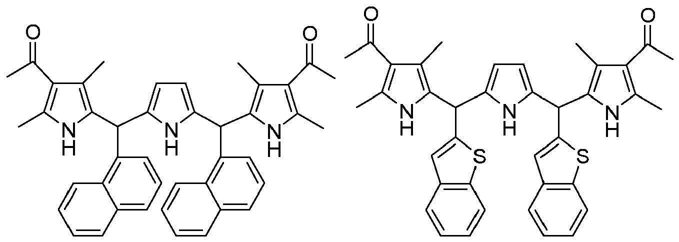

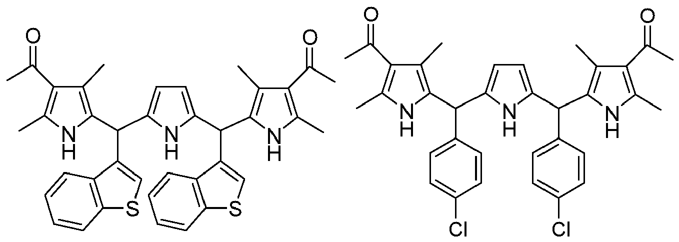

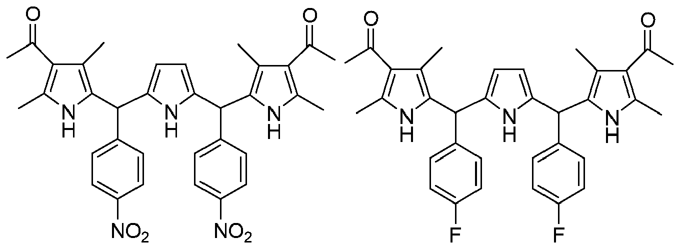

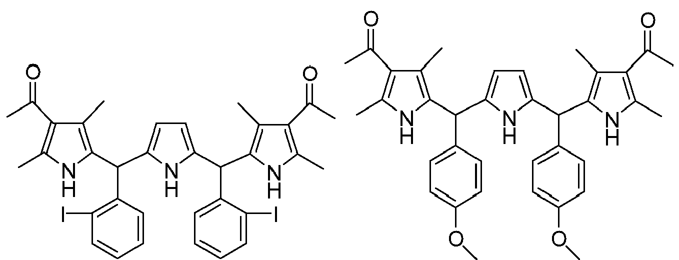

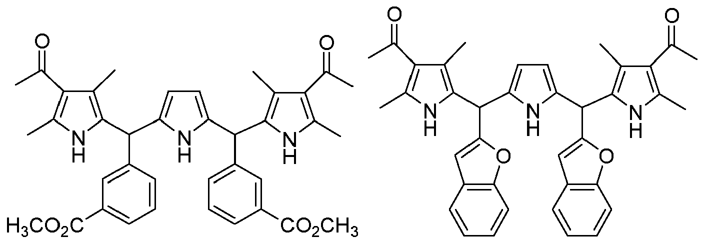

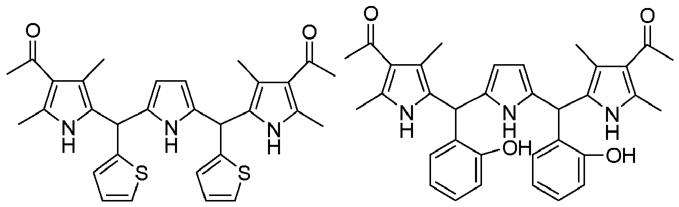

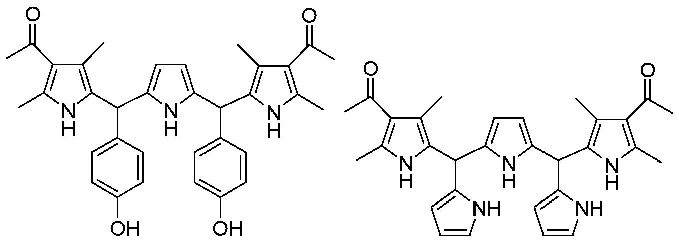

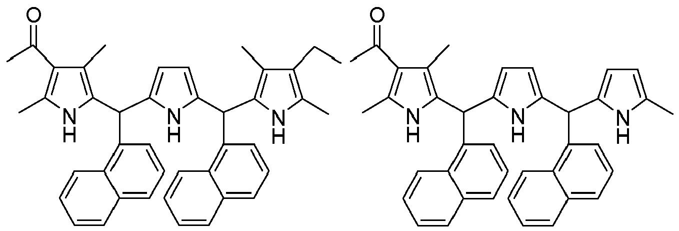

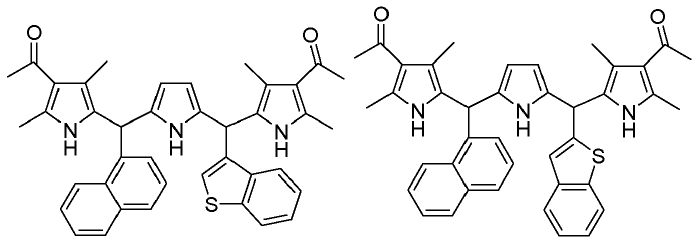

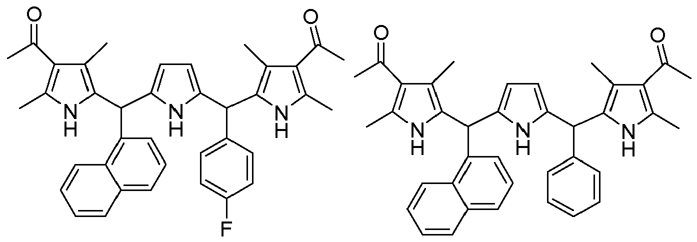

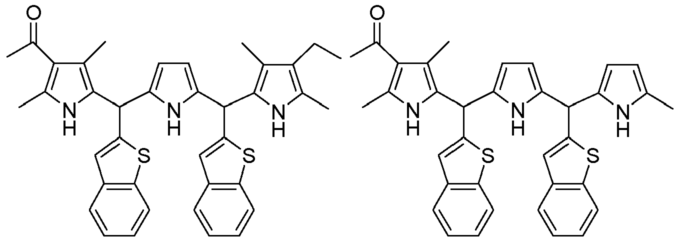

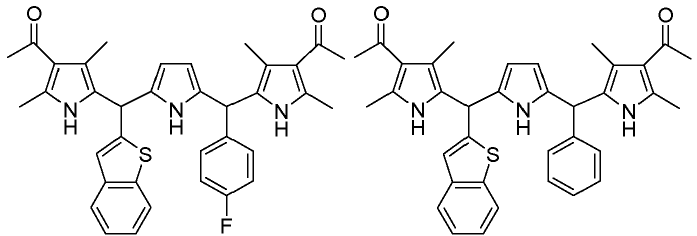

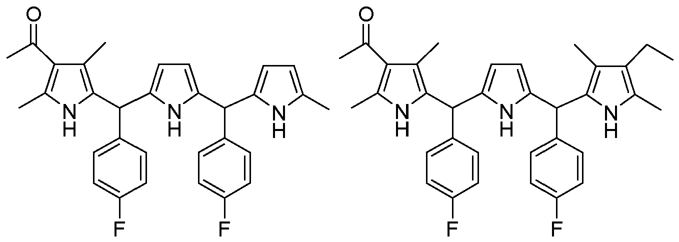

All the products described above are new.

In another aspect, the present invention relates to a process of preparation of compounds of the following formula I-21: 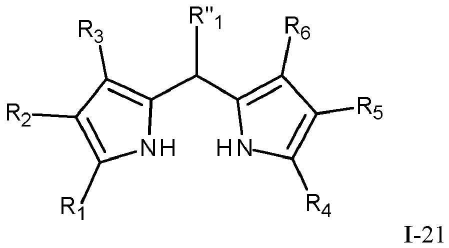 wherein R₁, R₂, R₃, R₄, R₅ and R₆ represent independently from each other:
- a H,
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
at least one of R₁, R₂ or R₃ representing a CHO, a CO-(C₁-C₇)-alkyl, or a CO-aryl, R"₁ is selected from the aryls of following formulae (a") to (p"): 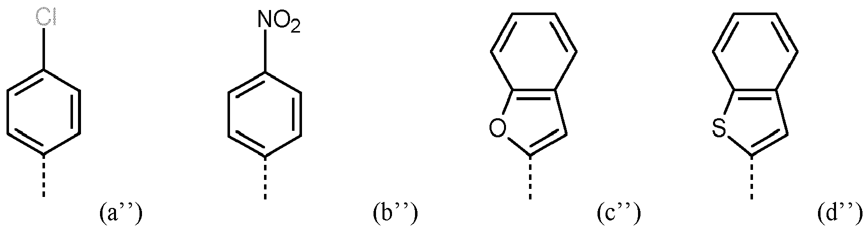 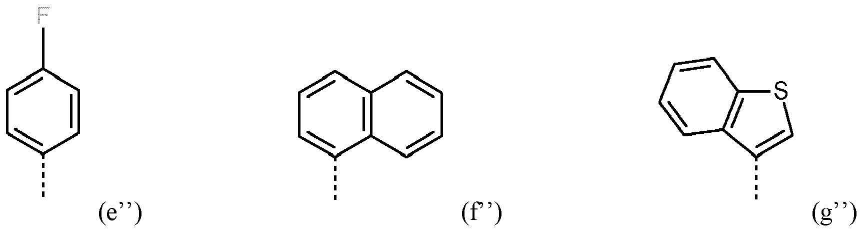 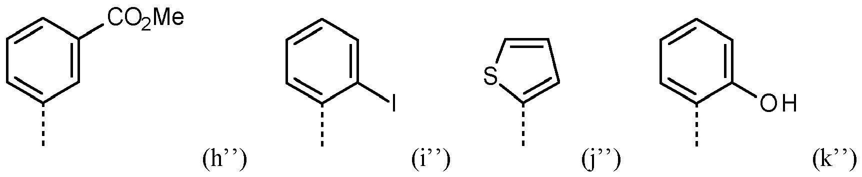 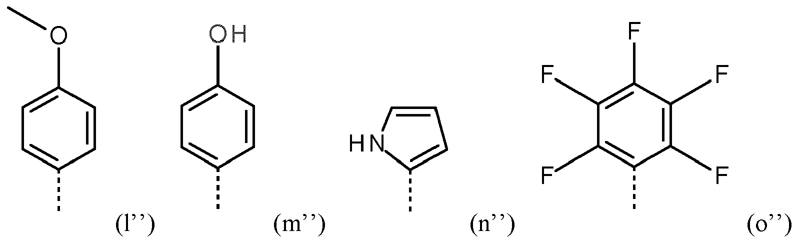 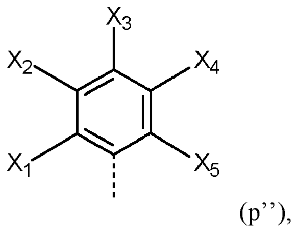 wherein X₁, X₂, X₃, X₄ and X₅ represent independently from each other:
- F, Cl, Br or I, or
- H, F, Cl, Br or I, provided at least one of X₁, X₂, X₃, X₄ and X₅, in particular at least two, in particular at least three, in particular four of X₁, X₂, X₃, X₄ and X₅ do not represent H,
said R₁, R₂, R₃, R₄, R₅, R₆ and R"₁ being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
said (C₁-C₇)-alkyl, (C₂-C₇)-alkenyl, (C₂-C₇)-alkynyl, (C₃-C₇)-cycloalkyl, (C₅-C₇)-cycloalkenyl, (C₃-C₇)-heterocycloalkyl, (C₅-C₇)-heterocycloalkenyl, CO-(C₁-C₇)-alkyl, CO₂-(C₁-C₇)-alkyl, CONH-(C₁-C₇)-alkyl, aryl, alkyl aryl and CO-aryl being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or
   heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
provided that:
- none of R₁, R₂ and R₃ forms a cycle with R₄, R₅ or R₆,
- R₁ and R₄ do not form a cycle with respectively R₂ or R₃, and R₅ or R₆,
- R₂ and R₅ do not form a cycle with respectively R₃ and R₆,
- R₁ and/or R₄ represent(s) an alkyl aryl when R"₁ is selected from groups (1"), (m") and (n"),
said process of preparation comprising:
a) a reaction between a compound of the following formula II: 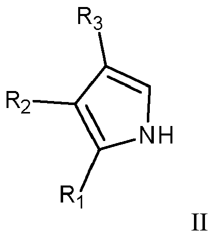 wherein R₁, R₂ and R₃ have the meaning indicated above,
   and a compound of the following formula III: 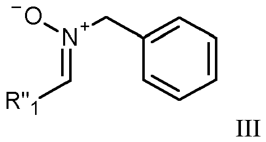 wherein R"₁, has the meaning indicated above,
   in presence of hydrogen chloride,
   to form the intermediate of the following formula IV: 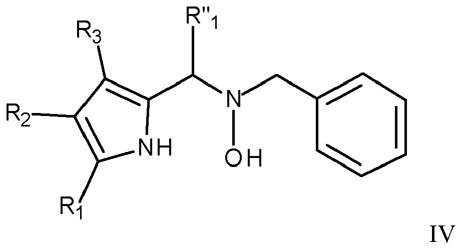 wherein R₁, R₂, R₃ and R"₁ have the meaning indicated above,
   said intermediate being then reacted with a compound of formula V: 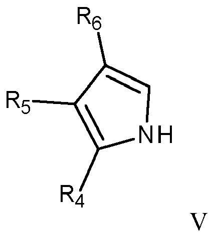 wherein R₄, R₅ and R₆ have the meaning indicated above,
   in presence of trimethylsilyl chloride or hydrogen chloride, to form a compound of formula I-21,

   or, provided that R₁, R₂ and R₃ are respectively identical to R₄, R₅, and R₆, a reaction selected from:
b) a reaction between a compound of the following formula II: 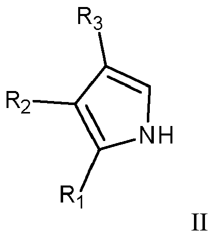 wherein R₁, R₂ and R₃ have the meaning indicated above,
   and a compound of the following formula III: 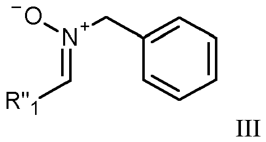 wherein R"₁, has the meaning indicated above,
   in presence of trimethylsilyl chloride or hydrogen chloride,
c) a reaction between a compound of formula II and a compound of the following formula VI: 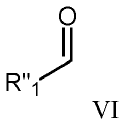 wherein R"₁, has the meaning indicated above,
   in presence of iodine under MW irradiation,
d) a reaction between a compound of formula II and a compound of formula VI in presence of trifluoroacetic acid,
   to obtain a compound of formula I-21, wherein R₁, R₂ and R₃ are respectively identical to R₄, R₅, and R₆.

In an advantageous embodiment, the present invention relates to a process of preparation of compounds of formula I-21, comprising a reaction between a compound of the following formula II: 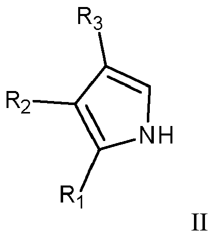 and a compound of the following formula III: 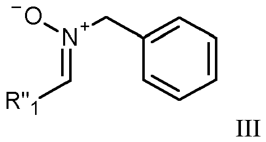 wherein R"₁, has the meaning indicated above,
in presence of hydrogen chloride,
to form the intermediate of the following formula IV: 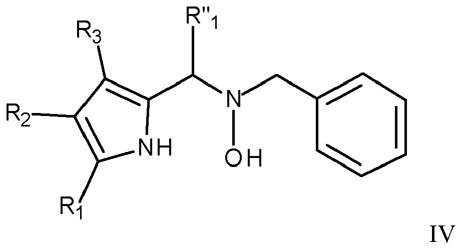 wherein R₁, R₂, R₃ and R"₁ have the meaning indicated above,
said intermediate being then reacted with a compound of formula V: 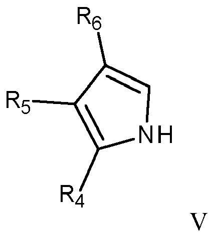 in presence of trimethylsilyl chloride or hydrogen chloride,
to form a compound of formula I-21: 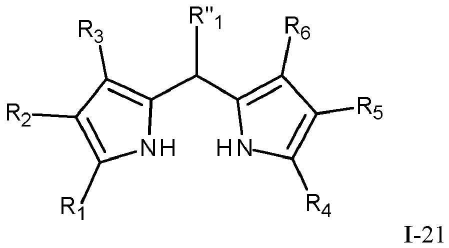 wherein R₁, R₂, R₃, R₄, R₅, R₆ and R"₁ are as defined above.

In an advantageous embodiment, the present invention relates to a process of preparation of compounds of formula I-21, comprising a reaction selected from:
a) a reaction between a compound of the following formula II: 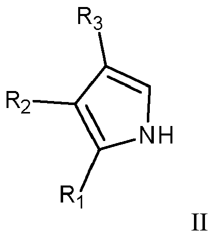 and a compound of the following formula III: 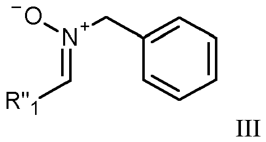 wherein R"₁, has the meaning indicated above,
   in presence of trimethylsilyl chloride or hydrogen chloride,
b) a reaction between a compound of formula II and a compound of the following formula VI: 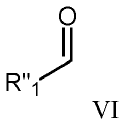 wherein R"₁, has the meaning indicated above,
   in presence of iodine under MW irradiation,
c) a reaction between a compound of formula II and a compound of formula VI in
   presence of trifluoroacetic acid,
   to obtain a compound of formula I-22: 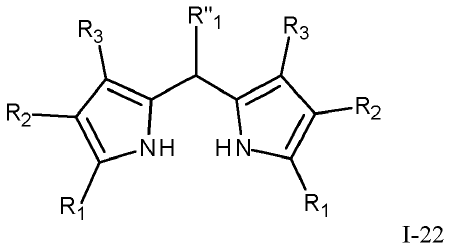 wherein R₁, R₂, R₃, R₄, R₅, R₆ and R"₁ are as defined above.

In an advantageous embodiment, the present invention relates to a process of preparation of compounds of formula I-21, of particular formula I-22bis: 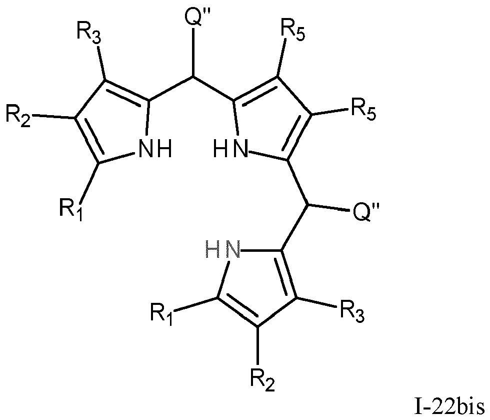 wherein Q" is an aryl selected from the aryls of following formulae (a") to (p"): 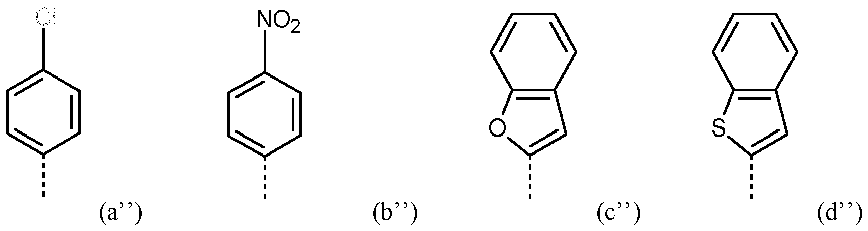 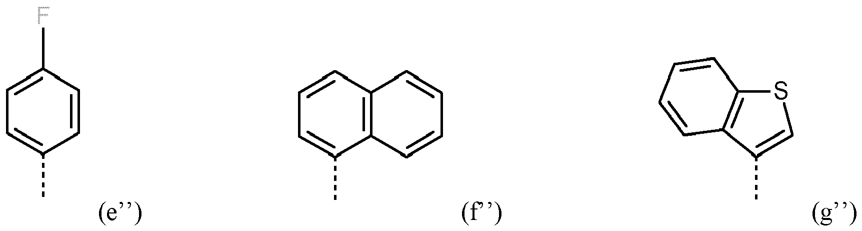 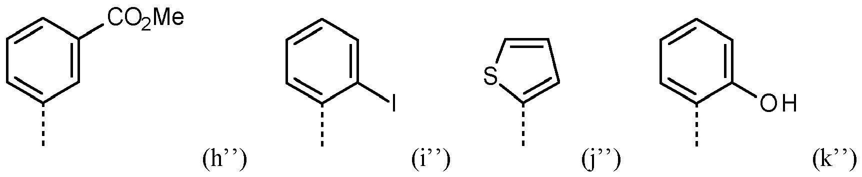 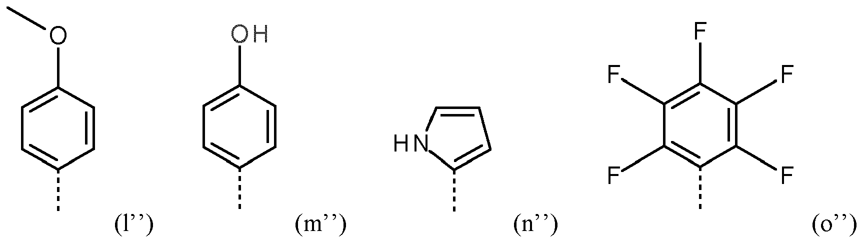 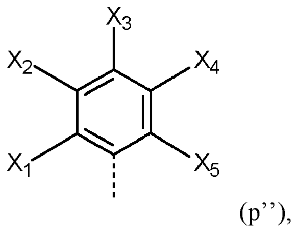 wherein X₁, X₂, X₃, X₄ and X₅ represent independently from each other:
- F, Cl, Br or I, or
- H, F, Cl, Br or I, provided at least one of X₁, X₂, X₃, X₄ and X₅, in particular at least two, in particular at least three, in particular four of X₁, X₂, X₃, X₄ and X₅ do not represent H,
said Q" being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
   H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
comprising a reaction between the intermediate of the following formula IV: 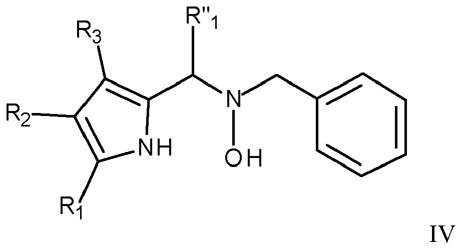 and a compound of the following formula IIbis: 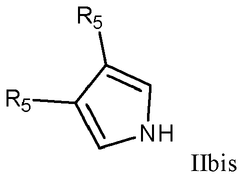 wherein R₅ is as defined above,
in presence of hydrogen chloride to obtain a compound of formula I-22bis.

### DESCRIPTION OF THE FIGURES

### EXAMPLES:

### EXPERIMENTAL PART - CHEMISTRY

The following examples describe the synthesis of 26 compounds of the invention (symmetric compounds **3a** to **3m**, unsymmetric compounds **7a** to **7h**, tripyrromethanes **6a** to **6e** and the intermediates).

### General procedures:

Dichloromethane was dried by refluxing over CaH₂ and then distilled.

Methanol was refluxed over magnesium turnings and then distilled.
**Chromatography purifications** were performed by column chromatography using Macherey-Nagel silica gel 60 (40-60 mesh).
**Melting points** were obtained on a Büchi B35 apparatus and are uncorrected.
**Infrared spectra (IR)** were obtained either as neat films on sodium chloride discs or in potassium bromide plates. All IR spectra were recorded on a Nicolet Impact-400 Fourier transform infrared spectrometer (FTIR) and the data are reported in reciprocal centimeters (cm⁻¹).
**¹H NMR Spectra** (300 or 400 MHz) and **¹³C NMR spectra** (75 or 100 MHz) were recorded on either a Bruker Advance300 or Advance400 spectrometers. Chemical shifts are given in ppm (δ) and were referenced to the internal solvent signal or to TMS used as an internal standard. When ambiguous, proton and carbon assignments were established using COSY, HMQC and/or DEPT experiments. Multiplicities are declared as follows: *s* (singlet), *br s* (broad singlet), *d* (doublet), *t* (*triplet*), *q* (quadruplet), *sept* (septet), *dd* (doublet of doublet), *ddd* (doublet of doublet of doublet), *dt* (doublet of triplet), *dq* (doublet of *quadruplet*), *m* (multiplet), *ABq* (ABq system). Coupling constants *J* are given in Hertz.
**Low Resolution Mass Spectra (LRMS)** were recorded on a Bruker Esquire 3000 plus (ESI) or a ThermoFinnigan PolarisQ ion-trap spectrometer, using DCI (ammonia/isobutane 63/37).
**High Resolution Mass Spectra (HRMS)** were recorded on Thermoquest Orbitrap spectrometer at the LCOSB, UMR 7613, Université Pierre et Marie Curie, Paris.
**Elemental analyses** were performed at the « Service d'Analyse Elémentaire du Département de Chimie Moléculaire », Grenoble.
**Microwave** reactions were carried out in a CEM Discover Microwave apparatus.

### Compounds 3a and 3b

These compounds have been prepared in one step according to M. L. Murat-Onana, C. Berini, F. Minassian, N. Pelloux-Léon, J.-N. Denis Org. Biomol. Chem. 2010, 8, 2204-2211 **(Scheme I).**

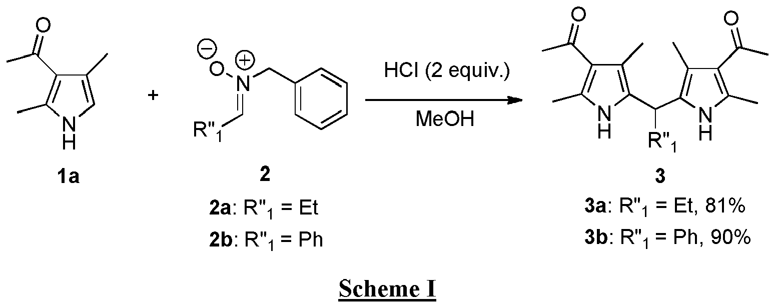

### General procedure

Freshly distilled acetyl chloride (2 equiv.) was added dropwise at 0°C to anhydrous methanol and the mixture was stirred for 15 min to form in situ hydrogen chloride (2 equiv.). The appropriate nitrone **2** (1 equiv.) was added followed by pyrrole **1** (2 equiv.). The reaction mixture was then warmed to the appropriate temperature and stirred until complete disappearance of the starting material (followed by TLC). The mixture was then treated with saturated aqueous NaHCO₃ solution up to pH 8-9. The aqueous layer was extracted three times with CH₂Cl₂. The combined organic layers were washed with brine, dried over anhydrous MgSO₄ and concentrated. The obtained 2,2'-bis(pyrrolyl)alkane (**3**) was purified by flash chromatography on silica gel (pre-treated with 3% of triethylamine, v/v).

### Example 1: 1-{5-[4-Acetyl-3,5-dimethyl-1H-pyrrol-2-yl)propyl]-2,4-dimethyl-1H-pyrrol-3-yl}ethan-1-one (3a)

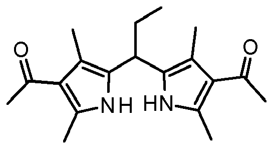

Prepared according to the above general procedure starting from 60 mg of nitrone **2a** (0.37 mmol) in 5.0 mL of MeOH, 101 mg of pyrrole **1a** (0.73 mmol) and 57 mg of acetyl chloride (0.73 mmol). The mixture was stirred at room temperature for 3 hours. Purification (eluent: pentane/EtOAc, 8/2 to 4/6) afforded 2,2'-bis(pyrrolyl)alkane **3a** (94 mg, 0.30 mmol, 81%) as a white solid.
**Mp:** 178-179°C.
**IR** (KBr disc): 3300, 3110, 3030, 2960, 2920, 2870, 2740, 1630, 1520, 1470, 1440, 1410, 1360, 940, 910, 730 cm⁻¹.
**¹H NMR** (300 MHz, CDCl₃) δ 0.89 (t, *J* = 7.2 Hz, 3H, CH₃), 1.89-1.99 (m, 2H, CH₂), 2.23 (s, 6H, 2CH₃), 2.40 (s, 6H, 2CH₃), 2.42 (s, 6H, 2CH₃), 4.00 (t, *J* = 7.8 Hz, 1H, H-1), 8.58 (s, 2H, NH).
**¹³C NMR** (75 MHz, CDCl₃) δ 11.9 (CH₃), 12.3 (C-3), 15.1 (CH₃), 27.1 (C-2), 30.9 (CH₃), 33.6 (C-1), 115.1 (C_{pyrr.}), 121.1 (C_{pyrr.}), 128.7 (C_{pyrr.}), 135.0 (C_{pyrr.}), 196.9 (C=O). **LRMS** (DCI, NH₃/isobutane): *m*/*z* 315 [(M+H)⁺], 178 (100%).
**Anal. Calcd for C₁₉H₂₆N₂O₂**: C, 72.58; H, 8.33; N, 8.91. Found: C, 72.36; H, 8.35; N, 8.52.

### Example 2: 1-{5-[4-Acetyl-3,5-dimethyl-1H-pyrrol-2-yl)(phenyl)methyl]-2,4-dimethyl-1H-pyrrol-3-yl}ethan-1-one (3b)

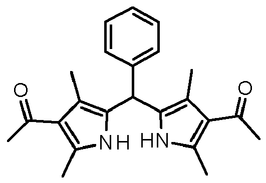

Prepared according to the above general procedure starting from 78 mg of nitrone **2b** (0.37 mmol) in 5.0 mL of MeOH, 101 mg of pyrrole **1a** (0.73 mmol) and 57 mg of acetyl chloride (0.73 mmol). The mixture was stirred at room temperature for 3.5 hours. Purification (eluent: pentane/EtOAc, 6/4 to 2/8) afforded 2,2'-bis(pyrrolyl)alkane **3b** (121 mg, 0.33 mmol, 90%) as an orange foam.
**IR** (KBr disc): 3290, 3090, 3060, 3020, 2960, 2920 2240, 1730, 1630, 1470, 1440, 1420, 1380, 1240, 1110, 1070, 1050, 950, 910, 730 cm⁻¹.
**¹H NMR** (300 MHz, CDCl₃) δ 2.13 (s, 6H, 2CH₃), 2.33 (s, 6H, 2CH₃), 2.36 (s, 6H, 2CH₃), 5.56 (s, 1H, H-1), 7.04-7.05 (m, 2H, Hₐᵣₒₘ), 7.20-7.30 (m, 3H, Hₐᵣₒₘ), 8.56 (s, 2H, NH). **¹³C NMR** (75 MHz, CDCl₃) δ 11.8 (CH₃), 15.1 (CH₃), 30.8 (CH₃), 38.3 (C-1), 116.6 (C_{pyrr}), 121.6 (C_{pyrr}), 126.2 (C_{pyrr}), 126.9 (CHₐᵣₒₘ), 128.0 (CHₐᵣₒₘ), 128.8 (CHₐᵣₒₘ), 134.4 (C_{pyrr}), 140.7 (Cₐᵣₒₘ), 196.0 (C=O).
**LRMS** (ESI⁺): *m*/*z* 385 [(M+Na)⁺], 363 [(M+H)⁺].

### Compounds 3c-l

These compounds have been prepared in one step according to P.-A. Faugeras, B. Boëns, P.-H. Elchinger, J. Vergnaud, K. Teste, R. Zerrouki Tetrahedron Lett. 2010, 51, 4630-4632 (**Scheme II**).

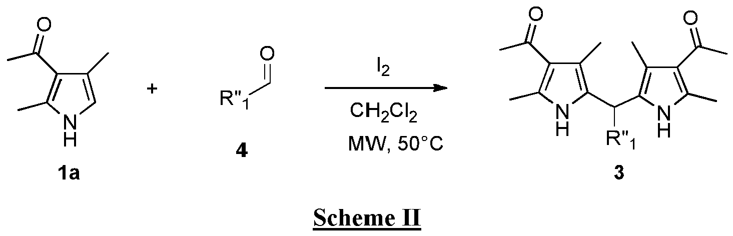

| **R"₁** | **Aldehydes** | **Products** | **yields** |
|---|---|---|---|
| 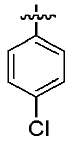 | **4a** | **3c** | 75% |
| 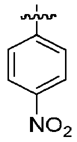 | **4b** | **3d** | Quantitative |
| 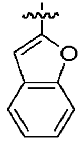 | **4c** | **3e** | Quantitative |
| 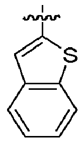 | **4d** | **3f** | 52% |
| 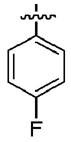 | **4e** | **3g** | 73% |
| 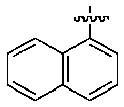 | **4f** | **3h** | 57% |
| 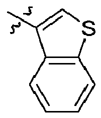 | **4g** | **3i** | 59% |
| 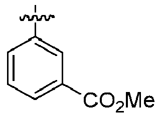 | **4h** | **3j** | 66% |
| 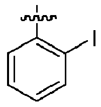 | **4i** | **3k** | 50% |
| 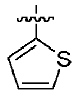 | **4j** | **31** | 74% |

### General procedure

3-Acetyl-2,4-dimethylpyrrole **1a** (80 mg, 0.583 mmol, 1.0 equiv.), aldehyde **4** (0.5 equiv.) and iodine (7 mg, 0.1 equiv.) were dissolved into dichloromethane (2 mL). The resulting mixture was stirred, then microwave irradiation (15 min., 50°C) was performed. The dichloromethane was evaporated and the obtained crude product was purified by flash chromatography on silica gel (eluents: Pentane/EtOAc 1/1 then Pentane/EtOAc 3/7) to give pure compound **3.**

### Example 3: 1-{5-[4-Acetyl-3,5-dimethyl-1H-pyrrol-2-yl)(4-chlorophenyl)methyl]-2,4-dimethyl-1H-pyrrol-3-yl}ethan-1-one (3c)

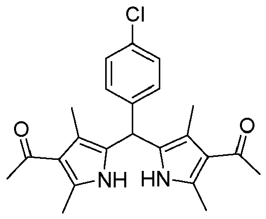

**Prepared** according to the above general procedure starting from 3-acetyl-2,4-dimethylpyrrole **1a** (80 mg, 0.583 mmol, 1.0 equiv.), aldehyde **4a** (41 mg, 0.292 mmol, 0.5 equiv.) and iodine (7 mg, 0.1 equiv.) and dichloromethane (2 mL). After purification, 2,2'-bis(pyrrolyl)alkane **3c** (87 mg, 0.219 mmol) was obtained in 75% yield as a pink solid.
**Mp:** 162-170°C.
**IR** (KBr disc): 3270, 1615, 1415 cm⁻¹.
**¹H NMR** (300 MHz, CDCl₃) δ 2.11 (s, 6H, 2CH₃), 2.36 (s, 6H, 2CH₃), 2.38 (s, 6H, 2CH₃), 5,51 (s, 1H, H-1), 6.98 (d, *J* = 7.9 Hz, 2H, H_{arom.}), 7.21 (d, *J* = 7.9 Hz, 2H, H_{arom.}), 8.37 (s, 2H, NH).
**¹³C NMR** (75 MHz, CDCl₃) δ 12.0 (CH₃), 15.3 (CH₃), 31.0 (CH₃), 37.9 (C-1), 117.0 (C_{pyrr.}), 121.9 (C_{pyrr.}), 125.8 (C_{pyrr.}), 129.0 (CH_{arom.}), 129.6 (CH_{arom.}), 133.0 (C_{arom.}), 134.9 (C_{pyrr}.), 139.5 (C_{arom.}), 196.2 (C=O).
**LRMS** (ESI⁺): *m*/*z* 419 [(M+Na)⁺], 397 [(M+H)⁺].

### Example 4: 1-{5-[4-Acetyl-3,5-dimethyl-1H-pyrrol-2-yl)(4-nitrophenyl)methyl]-2,4-dimethyl-1H-pyrrol-3-yl}ethan-1-one (3d)

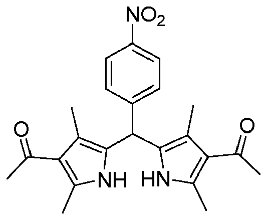

Prepared according to the above general procedure starting from 3-acetyl-2,4-dimethylpyrrole **1a** (80 mg, 0.583 mmol, 1.0 equiv.), aldehyde **4b** (44 mg, 0.292 mmol, 0.5 equiv.), iodine (7 mg, 0.1 equiv.) and dichloromethane (2 mL). After purification, 2,2'-bis(pyrrolyl)alkane **3d** (118 mg, 0.292 mmol) was obtained in a quantitative yield as an ochre solid.
**Mp**: 211°C.
**IR (KBr disc)**: 3300, 3205, 2955, 2920, 1620, 1570, 1520, 1470, 1445, 1415, 1345, 1240 cm⁻¹.
**¹H NMR** (300 MHz, CDCl₃) δ 2.11 (s, 6H, 2CH₃), 2.36 (s, 6H, 2CH₃), 2.40 (s, 6H, 2CH₃), 5.63 (s, 1H, H-1); 7.23 (d, *J* = 8.7 Hz, 2H, H_{arom.}), 8.05 (d, *J* = 8.7 Hz, 2H, H_{arom.}), 8.72 (s, 2H, NH).
**¹³C NMR** (75 MHz, CDCl₃) δ 12.1 (CH₃), 15.3 (CH₃), 31.1 (CH₃), 38.7 (C-1), 117.6 (C_{pyrr}.), 121.9 (C_{pyrr.}), 124.0 (C_{pyrr.}), 124.9 (CH_{arom.}), 129.3 (CH_{arom.}), 135.3 (C_{pyrr.}), 146.9 (C_{arom.}), 148.8 (C_{arom.}), 196.0 (C=O).
**LRMS** (ESI⁺): *m*/*z* 430 [(M+Na)⁺], 408 [(M+H)⁺].

### Example 5: 1-{5-[4-Acetyl-3,5-dimethyl-1H-pyrrol-2-yl)(1-benzofuran-2-yl)methyl]-2,4-dimethyl-1H-pyrrol-3-yl}ethan-1-one (3e)

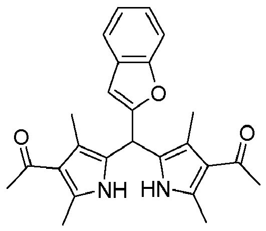

Prepared according to the above general procedure starting from 3-acetyl-2,4-dimethylpyrrole **1a** (80 mg, 0.583 mmol, 1.0 equiv.), aldehyde **4c** (42.6 mg, 0.292 mmol, 0.5 equiv.), iodine (7 mg, 0.1 equiv.) and dichloromethane (2 mL). After purification, 2,2'-bis(pyrrolyl)alkane **3e** (117 mg, 0.292 mmol) was obtained in a quantitative yield as a pink solid.
**Mp**: 176°C.
**IR (KBr disc)**: 3200, 2960, 2920, 1610, 1580, 1470, 1445, 1415, 1375, 1360, 1255 cm⁻¹.
**¹H NMR** (300 MHz, CDCl₃) δ 2.17 (s, 6H, 2CH₃), 2.40 (s, 6H, 2CH₃), 2.44 (s, 6H, 2CH₃), 5.64 (s, 1H, H-1); 6.46 (s, 1H, H_{arom.}), 7.21-7.31 (m, 2H, H_{arom.}), 7.44-7.52 (m, 2H, H_{arom.}), 7,92 (s, 2H, NH).
**¹³C NMR** (75 MHz, CDCl₃) δ 11.9 (CH₃), 15.3 (CH₃), 31.0 (CH₃), 33.7 (C-1), 105.1 (CH_{arom.}), 111.4 (CH_{arom.}), 116.9 (C_{pyrr.}), 120.9 (CH_{arom.}), 121.9 (C_{pyrr.}), 123.1 (CH_{arom.}), 123.7 (C_{pyrr.}), 124.3 (CH_{arom.}), 128.3 (C_{arom.}), 134.9 (C_{pyrr.}), 155.2 (C_{arom.}), 156.3 (C_{arom.}), 196.0 (C=O).
**MS** (ESI⁺): *m*/*z* 425 [(M+Na)⁺], 403 [(M+H)⁺].

### Example 6: 1-{5-[4-Acetyl-3,5-dimethyl-1H-pyrrol-2-yl)(1-benzothiophen-2-yl)methyl]-2,4-dimethyl-1H-pyrrol-3-yl}ethan-1-one (3f)

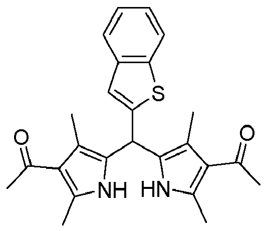

Prepared according to the above general procedure starting from 3-acetyl-2,4-dimethylpyrrole **1a** (80 mg, 0.583 mmol, 1.0 equiv.), aldehyde **4d** (47 mg, 0.292 mmol, 0.5 equiv.), iodine (7 mg, 0.1 equiv.) and dichloromethane (2 mL). After purification, 2,2'-bis(pyrrolyl)alkane **3f** (64 mg, 0.152 mmol) was obtained in a 52% yield as a pink solid.
**Mp**: 204-207°C.
**IR (KBr disc)**: 3200, 2955, 2920, 1610, 1580, 1470, 1445, 1415, 1375, 1360, 1255 cm⁻¹.
**¹H NMR** (300 MHz, CDCl₃) δ 2.16 (s, 6H, 2CH₃), 2.42 (s, 6H, 2CH₃), 2.44 (s, 6H, 2CH₃), 5.77 (s, 1H, H-1); 6.97 (s, 1H, H_{arom.}), 7.31-7.35 (m, 2H, H_{arom.}), 7.66-7.69 (m, 1H, H_{arom.}), 7.75-7.78 (m, 1H, H_{arom.}), 7.82 (s, 2H, NH).
**¹³C NMR** (75 MHz, CDCl₃) δ 12.0 (CH₃), 15.3 (CH₃), 30.9 (CH₃), 34.6 (C-1), 116.9 (C_{pyrr.}), 121.5 (C_{pyrr.}), 122.2 (CHₐᵣₒₘ), 122.5 (CHₐᵣₒₘ), 123.3 (CH_{arom.}), 124.2 (CHₐᵣₒₘ), 124.5 (CHₐᵣₒₘ), 125.8 (C_{pyrr}), 135.4 (Cₐᵣₒₘ), 139.8 (Cₐᵣₒₘ), 139.9 (Cₐᵣₒₘ), 146.2 (C_{pyrr}), 196.4 (C=O).
**LRMS** (ESI⁺): *m*/*z* 441 [(M+Na)⁺], 419 [(M+H)⁺].

### Example 7: 1-{5-[4-Acetyl-3,5-dimethyl-1H-pyrrol-2-yl)(4-fluorophenyl)methyl]-2,4-dimethyl-1H-pyrrol-3-yl}ethan-1-one (3g)

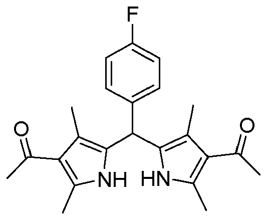

Prepared according to the above general procedure starting from 3-acetyl-2,4-dimethylpyrrole **1a** (80 mg, 0.583 mmol, 1.0 equiv.), aldehyde **4e** (36.2 mg, 0.292 mmol, 0.5 equiv.), iodine (7 mg, 0.1 equiv.) and dichloromethane (2 mL). After purification, 2,2'-bis(pyrrolyl)alkane **3g** (81 mg, 0.152 mmol) was obtained in a 73% yield as a pink solid.
**Mp**: 146-147°C
**IR** (KBr disc): 3205, 2920, 1735, 1575, 1505, 1415, 1360, 1225 cm⁻¹.
**¹H NMR** (300 MHz, CDCl₃) δ 2.09 (s, 6H, 2CH₃), 2.38 (s, 6H, 2CH₃), 2.40 (s, 6H, 2CH₃), 5.51 (s, 1H, H-1), 6.95-7.06 (m, 4H, H_{arom.}), 7.98 (s, 2H, NH).
**¹³C NMR** (75 MHz, CDCl₃) (2 rotamers) δ 12.0 (CH₃), 15.3 (CH₃), 30.9 (CH₃), 37.5 (C-1), 115.4 (CH_{arom.}), 115.7 (CH_{arom.}), 116.9 (C_{pyrr.}), 121.7 (C_{pyrr.}), 126.3 (C_{pyrr.}), 129.7 (CH_{arom.}), 129.8 (CH_{arom.}), 135.0 (C_{pyrr.}), 136.6 (Cₐᵣₒₘ), 136.7 (Cₐᵣₒₘ), 160.2 (C_{arom.}), 163.4 (C_{arom.}), 196.3 (C=O).
**LRMS** (ESI⁺): *m*/*z* 403 [(M+Na)⁺], 381 [(M+H)⁺].

### Example 8: 1-{5-[4-Acetyl-3,5-dimethyl-1H-pyrrol-2-yl)(naphtalen-1-yl)methyl]-2,4-dimethyl-1H-pyrrol-3-yl}ethan-1-one (3h)

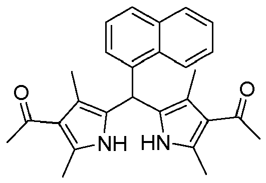

Prepared according to the above general procedure starting from 3-acetyl-2,4-dimethylpyrrole **1a** (80 mg, 0.583 mmol, 1.0 equiv.), aldehyde **4f** (46 mg, 0.292 mmol, 0.5 equiv.), iodine (7 mg, 0.1 equiv.) and dichloromethane (2 mL). After purification, 2,2'-bis(pyrrolyl)alkane **3h** (68 mg, 0.165 mmol) was obtained in a 57% yield as an orange solid.
**Mp**: 231°C.
**IR** (KBr disc): 3290, 3220, 2915, 1620, 1570, 1470, 1440, 1415, 1355, 1240 cm⁻¹.
**¹H NMR** (300 MHz, CDCl₃) δ 2.10 (s, 6H, 2CH₃), 2.35 (s, 6H, 2CH₃), 2.39 (s, 6H, 2CH₃), 6.15 (s, 1H, H-1), 7.04 (d, *J* = 7.0 Hz, 1H, H_{arom.}), 7.39 (t, *J* = 9.0 Hz, 1H, H_{arom.}), 7.46-7.50 (m, 2H, H_{arom.}), 7.66 (br s, 2H, NH), 7.79-7.90 (m, 3H, H_{arom.}).
**¹³C NMR** (75 MHz, CDCl₃) δ 12.0 (CH₃), 15.3 (CH₃), 31.1 (CH₃), 36.4 (C-1), 116.5, 122.4, 123.3, 125.7, 125.8, 126.1, 126.2 127.0, 128.5, 129.0, 131.3, 133.9, 134.2, 136.2, 195.9 (C=O).
**LRMS** (ESI⁺): *m*/*z* 413 [(M+H)⁺].

### Example 9: 1-{5-[4-Acetyl-3,5-dimethyl-1H-pyrrol-2-yl)(1-benzothiophen-3-yl)methyl]-2,4-dimethyl-1H-pyrrol-3-yl}ethan-1-one (3i)

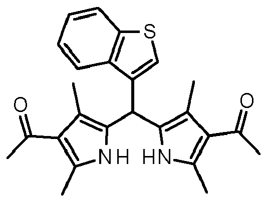

Prepared according to the above general procedure starting from 3-acetyl-2,4-dimethylpyrrole **1a** (80 mg, 0.583 mmol, 1.0 equiv.), aldehyde **4g** (47 mg, 0.292 mmol, 0.5 equiv.), iodine (7 mg, 0.1 equiv.) and dichloromethane (2 mL). After purification, 2,2'-bis(pyrrolyl)alkane **3i** (72 mg, 0.172 mmol) was obtained in a 59% yield as a pink solid.
**Mp**: 217-226°C.
**IR (KBr disc)**: 3205, 2915, 1740, 1615, 1575, 1470, 1445, 1425, 1410, 1370, 1360 cm⁻¹.
**¹H NMR** (300 MHz, CDCl₃) δ 2.17 (s, 6H, 2CH₃), 2.36 (s, 6H, 2CH₃), 2.38 (s, 6H, 2CH₃), 5.75 (s, 1H, H-1); 6.94 (s, 1H, H_{arom.}), 7.29-7.44 (m, 3H, H_{arom.}), 7.84 (d, *J* = 7.4 Hz, 1H, H_{arom.}), 8.06 (s, 2H, NH).
**¹³C NMR** (75 MHz, CDCl₃) δ 12.0 (CH₃), 15.3 (CH₃), 31.1 (CH₃), 34.0 (C-1), 116.6, 122.2, 122.3, 123.1, 124.7, 124.9, 125.0, 125.1, 134.2, 135.2, 137.7, 141.2, 196.0 (C=O).
**LRMS** (ESI⁺): *m*/*z* 419 [(M+H)⁺].

### Example 10: Methyl-3-[bis(4-acetyl-3,5-dimethyl-1H-pyrrol-2-yl)methyl]benzoate (3j)

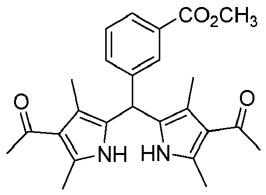

Prepared according to the above general procedure starting from 3-acetyl-2,4-dimethylpyrrole **1a** (80 mg, 0.583 mmol, 1.0 equiv.), aldehyde **4h** (48 mg, 0.292 mmol, 0.5 equiv.), iodine (7 mg, 0.1 equiv.) and dichloromethane (2 mL). After purification, 2,2'-bis(pyrrolyl)alkane **3j** (81 mg, 0.162 mmol) was obtained in a 66% yield as a pink solid.
**Mp**: 168-170°C.
**IR (KBr disc):** 3205, 3145, 2950, 2915, 1725, 1635, 1610, 1580, 1470, 1440, 1410, 1280 cm⁻¹.
**¹H NMR** (300 MHz, CDCl₃) δ 2.09 (s, 6H, 2CH₃), 2.36 (s, 6H, 2CH₃), 2.39 (s, 6H, 2CH₃), 3.85 (s, 3H, CH₃), 5.58 (s, 1H, H-1); 7.26-7,27 (m, 1H, H_{arom.}), 7.37 (t, *J* = 7.7 Hz, 1H, H_{arom.}), 7.76 (s, 1H, H_{arom.}), 7.87 (d, *J* = 7.7 Hz, 1H, H_{arom.}), 8.23 (s, 2H, NH).
**¹³C NMR** (75 MHz, CDCl₃) δ 12.0 (CH₃), 15.3 (CH₃), 31.1 (CH₃), 38.9 (C-1), 52.4 (CH₃), 117.0, 122.1, 125.5, 128.5, 129.2, 129.3, 131.0, 132.9, 134.6, 141.4, 167.0 (C=O), 196.0 (C=O).
**LRMS** (ESI⁺): *m*/*z* 421 [(M+H)⁺].

### Example 11: 1-{5-[4-Acetyl-3,5-dimethyl-1H-pyrrol-2-yl)(2-iodophenyl)methyl]-2,4-dimethyl-1H-pyrrol-3-yl}ethan-1-one (3k)

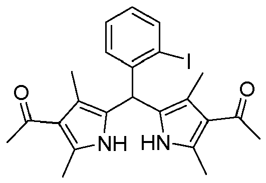

Prepared according to the above general procedure starting from 3-acetyl-2,4-dimethylpyrrole **1a** (80 mg, 0.583 mmol, 1.0 equiv.), aldehyde **4i** (68 mg, 0.292 mmol, 0.5 equiv.), iodine (7 mg, 0.1 equiv.) and dichloromethane (2 mL). After purification, 2,2'-bis(pyrrolyl)alkane **3k** (71 mg, 0.145 mmol) was obtained in a 50% yield as a pink solid.
**Mp**: 220-222°C.
**IR** (KBr disc): 3150, 2915, 1620, 1565, 1510, 1470, 1430, 1415, 1380, 1240 cm⁻¹.
**¹H NMR** (300 MHz, CDCl₃) δ 2.07 (s, 6H, 2CH₃), 2.43 (s, 12H, 4CH₃), 5.66 (s, 1H, H-1), 6.96-7.03 (m, 2H, H_{arom.}), 7.28-7.33 (m, 1H, H_{arom.}), 7.60 (br s, 2H, NH), 7.91 (d, *J* = 6.0 Hz 1H, H_{arom.}).
**¹³C NMR** (75 MHz, CDCl₃) δ 12.1 (CH₃), 15.4 (CH₃), 31.1 (CH₃), 45.0 (C-1), 100.7 (C-I), 117.3, 122.6, 124.7, 129.1, 129.2, 129.3, 133.9, 140.6, 143.1, 195.9 (C=O). **LRMS** (ESI⁺): *m*/*z* 511 [(M+Na)⁺], 489 [(M+H)⁺].

### Example 12: 1-{5-[4-Acetyl-3,5-dimethyl-1H-pyrrol-2-yl)(thiphen-2-yl)methyl]-2,4-dimethyl-1H-pyrrol-3-yl}ethan-1-one (31)

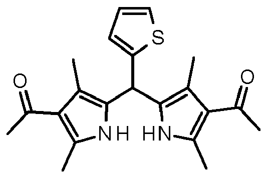

Prepared according to the above general procedure starting from 3-acetyl-2,4-dimethylpyrrole **1a** (80 mg, 0.583 mmol, 1.0 equiv.), aldehyde **4j** (33 mg, 0.292 mmol, 0.5 equiv.), iodine (7 mg, 0.1 equiv.) and dichloromethane (2 mL). After purification, 2,2'-bis(pyrrolyl)alkane **31** (79 mg, 0.214 mmol) was obtained in a 74% yield as a pink solid.
**Mp**: 248-254°C.
**IR** (KBr disc): 3205, 3090, 2915, 1610, 1410, 1360 cm⁻¹.
**¹H NMR** (300 MHz, CDCl₃) δ 2.16 (s, 6H, 2CH₃), 2.34 (s, 6H, 2CH₃), 2.40 (s, 6H, 2CH₃), 5.72 (s, 1H, H-1), 6.72-6.73 (m, 1H, H_{thioph.}), 6.92-6.95 (m, 1H, H_{thioph.}), 7.20 (dd, *J* = 5.0 and 0.9 Hz, 1H, Hₜₕᵢₒₚₕ), 8.32 (br s, 2H, NH).
**¹³C NMR** (75 MHz, CDCl₃) δ 12.0 (CH₃), 15.4 (CH₃), 31.1 (CH₃), 34.5 (C-1), 116.8, 122.1, 125.3, 125.9, 126.0, 127.3, 134.5, 144.8, 196.1 (C=O).
**LRMS** (ESI⁺): *m*/*z* 369 [(M+H)⁺].

### Example 13: 1-{5-[4-Acetyl-3,5-dimethyl-1H-pyrrol-2-yl)(2-hydroxyphenyl)methyl]-2,4-dimethyl-1H-pyrrol-3-yl}ethan-1-one (3m)

This compound has been prepared according to C.-H. Lee, J. S. Lindsey Tetrahedron 1994, 50, 11427-11440 (**Scheme III**).

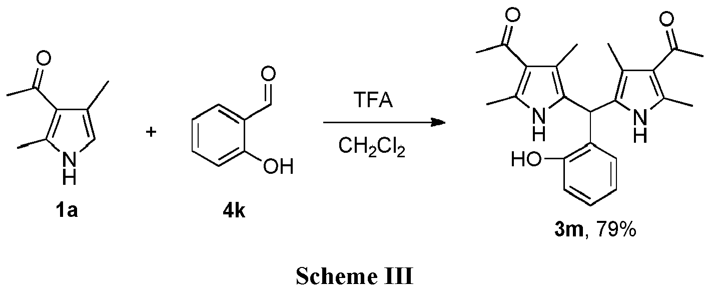

3-Acetyl-2,4-dimethylpyrrole **1** (80 mg, 0.583 mmol), salicylaldehyde **4k** (35.7 mg, 31 µL, 0.5 equiv.) and 3 drops of trifluoroacetic acid were added to dichloromethane (2 mL). The resulting mixture was stirred overnight at room temperature. The heterogeneous mixture was filtered in order to isolate compound as a pink powder **3m** (87 mg, 0.231 mmol) in 79% yield. **Mp**: 242°C.
**IR** (KBr disc): 3270, 2950, 2915, 1615, 1600, 1560, 1465, 1440, 1415, 1375, 1250, 1240 cm⁻¹.
**¹H NMR** (300 MHz, DMSO-d₆) δ 1.98 (s, 6H, 2CH₃), 2.27 (s, 6H, 2CH₃), 2.39 (s, 6H, 2CH₃), 5.71 (s, 1H, H-1), 6.73-6.78 (m, 2H, H_{arom.}), 7.01-7.03 (m, 2H, H_{arom.}), 9.44 (s, 1H, H_{arom.}), 10.25 (s, 2H, NH).
**¹³C NMR** (75 MHz, DMSO-d₆) δ 11.4 (CH₃), 14.6 (CH₃), 30.8 (CH₃), 32.1 (C-1), 114.6, 114.9 (CH_{arom.}), 118.9, 120.5 (C_{arom.}), 126.7 (CH_{arom.}), 127.3, 127.8, 129.1 (CH_{arom.}), 133.5, 154.3 (C_{arom.}), 193.8 (C=O).
**LRMS** (ESI⁺): *m*/*z* 401 [(M+Na)⁺], 379 [(M+H)⁺].

### Unsymmetrical bis(pyrrolyl)alkanes 7a-h

These compounds have been prepared in one step according to M. L. Murat-Onana, C. Berini, F. Minassian, N. Pelloux-Léon, J.-N. Denis Org. Biomol. Chem. 2010, 8, 2204-2211 **(Scheme IV).**

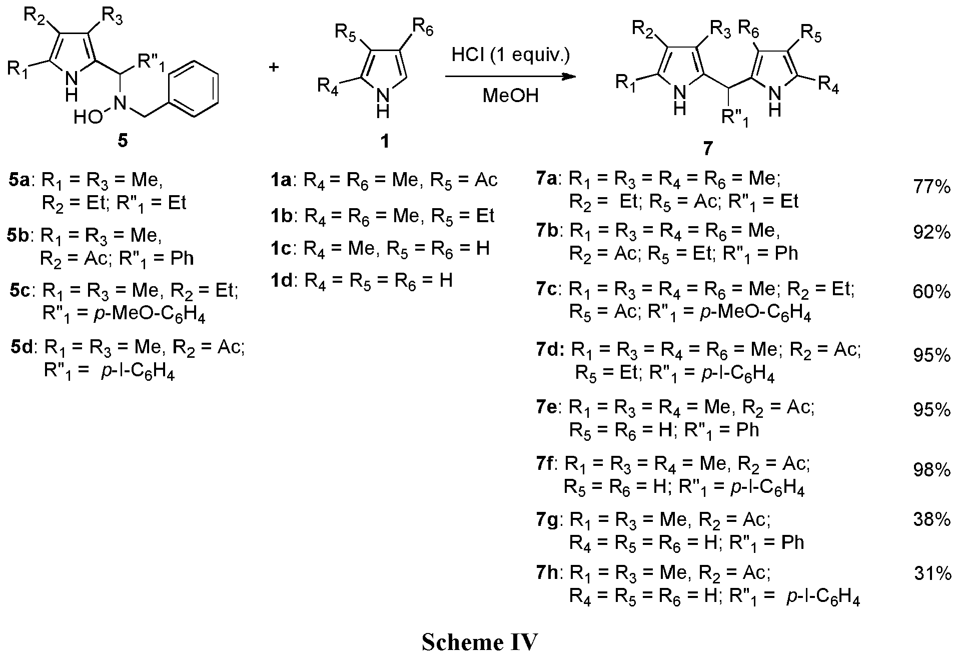

### General procedure

Freshly distilled acetyl chloride (1 equiv.) was added dropwise at 0°C to anhydrous methanol (0.2-0.5 mM) and the mixture was stirred for 15 min. The appropriate pyrrolic *N*-benzylhydroxylamine (**5**) (1 equiv.) was added and the mixture was cooled at -78 C before the addition of the appropriate pyrrole derivative (**1**). The mixture was warmed to the suitable temperature and stirred until complete disappearance of the starting material (followed by TLC). The mixture was then treated with saturated aqueous NaHCO₃ solution. The *p*H value was 8-9. The aqueous layer was extracted three times with CH₂Cl₂. The combined organic layers were washed with brine, dried over anhydrous MgSO₄ and concentrated. The obtained unsymmetrical bis(pyrrolyl)alkane 7 was purified by flash chromatography on silica gel pretreated with 3% of Et₃N (v/v).

### Example 14: 1-(5-(1-(4-Ethyl-3,5-dimethyl-1H-pyrrol-2-yl)propyl)-2,4-dimethyl-1H-pyrrol-3-yl)ethanone 7a

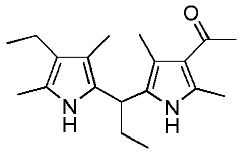

Prepared according to the above general procedure from the corresponding *N*-benzylhydroxylamine **5a** (50 mg, 0.18 mmol) in 2.0 mL of MeOH, 3-acetyl-2,4-dimethylpyrrole **1a** (23 mg, 0.17 mmol) and acetyl chloride (14 mg, 0.18 mmol). The mixture was stirred at 0 °C for 2 hours. Purification (eluent: CH₂Cl₂/EtOAc, 95/5) afforded unsymmetrical 2,2'-bis(pyrrolyl)alkane **7a** (39 mg, 0.13 mmol, 77%) as a pink foam. **Mp**: 66-67°C.
**IR (KBr disc):** 3300 (br s, NH), 2980, 2945, 2900, 1690 (CO), 1450, 1405 1385 cm⁻¹.
**¹H NMR (400 MHz, CDCl₃)** δ (ppm) 0.88 (3H, t, *J* = 7.6 Hz, CH₃), 1.04 (3H, t, *J* = 7.6 Hz, CH₃), 1.89 (3H, s, CH₃), 1.92-2.00 (2H, m, CH₂), 2.12 (3H, s, CH₃), 2.25 (3H, s, CH₃), 2.34 (2H, q, *J* = 7.6 Hz, CH₂), 2.41 (3H, s, CH₃), 2.42 (3H, s, CH₃), 3.96 (1H, t, *J* = 7.6 Hz, H₁), 7.65 (1H, br s, NH), 8.22 (1H, br s, NH).
**¹³C NMR (100 MHz, CDCl₃)** δ (ppm) 9.2 (CH₃), 11.0 (CH₃), 12.0 (CH₃), 12.3 (CH₃), 15.3 (CH₃), 15.6 (CH₃), 17.6 (CH₂), 27.3 (CH₂), 30.9 (CH₃), 35.2 (CH, C₁), 113.4 (C_{Pyr}), 115.2 (C_{Pyr}), 120.9 (C_{Pyr}), 121.3 (C_{Pyr}), 125.4 (C_{Pyr}), 128.1 (C_{Pyr}), 128.6 (C_{Pyr}), 133.8 (C_{Pyr}), 196.1 (CO).
**LRMS (ESI⁺):** *m*/*z* 323 ([M+Na]⁺, 20), 301 ([M+H]⁺, 80).
**HRMS (ESI⁺) Calcd for C₁₉H₂₈N₂ONa** 323.2093. **Found:** 323.2093 ([M+Na]⁺).

### Example 15: 1-(5-((4-Ethyl-3,5-dimethyl-1H-pyrrol-2-yl)(phenyl)methyl)-2,4-dimethyl-1H-pyrrol-3-yl)ethanone 7b

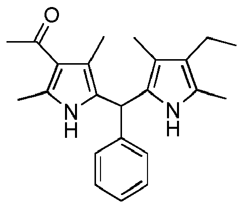

Prepared according to the above general procedure from the corresponding *N*-benzylhydroxylamine **5b** (40 mg, 0.12 mmol) in 2.0 mL of MeOH, 2,4-dimethyl-3-ethylpyrrole **1b** (15 mg, 0.12 mmol) and acetyl chloride (9 mg, 0.12 mmol). The mixture was stirred at 0°C for 1.5 hour. Purification (eluent: CH₂Cl₂/EtOAc, 9/1) afforded unsymmetrical 2,2'-bis(pyrrolyl)alkane **7b** (38 mg, 0.11 mmol, 92%) as an orange foam.
**Mp:** 84-85°C.
**IR (KBr disc):** 3300, 2960, 2920, 2860, 1630, 1440, 1410 cm⁻¹.
**¹H NMR (400 MHz, CDCl₃)** δ (ppm) 1.05 (3H, t, *J* = 7.6 Hz, CH₃), 1.78 (3H, s, CH₃), 2.07 (3H, s, CH₃), 2.08 (3H, s, CH₃), 2.33-2.42 (8H, m, CH₂, 2CH₃), 5.46 (1H, s, H₁), 6.96 (1H, br s, NH), 7.10-7.13 (2H, m, 2H_{Ar.}), 7.22-7.33 (3H, m, 3H_{Ar.}), 7.65 (1H, br s, NH).
**¹³C NMR (100 MHz, CDCl₃)** δ (ppm) 9.1 (CH₃), 11.1 (CH₃), 11.8 (CH₃), 15.2 (CH₃), 15.6 (CH₃), 17.6 (CH₂), 30.9 (CH₃), 40.0 (CH, C₁), 114.3 (C_{Pyr}), 115.8 (2C_{Pyr}), 121.5 (2C_{Pyr}), 122.0 (C_{Pyr}), 123.6 (C_{Pyr}), 126.8 (CH_{Ar}), 126.9 (C_{Pyr}), 128.2 (2CH_{Ar}), 128.8 (2CH_{Ar}), 133.9 (C_{Ar}), 141.4 (C_{Pyr}), 195.8 (CO).
**LRMS (ESI⁺):** *m*/*z* 371 ([M+Na]⁺, 23), 349 ([M+H]⁺, 77).
**HRMS (ESI⁺) Calcd for C₂₃H₂₈N₂ONa** 371.2093. **Found:** 371.2091 ([M+Na]⁺).

### Example 16: 1-(5-((4-Ethyl-3,5-dimethyl-1H-pyrrol-2-yl)(4-methoxyphenyl)methyl)-2,4-dimethyl-1H-pyrrol-3-yl)ethanone 7c

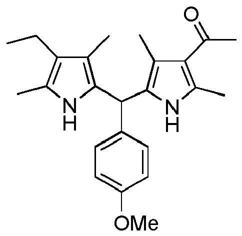

Prepared according to the above general procedure from the corresponding *N*-benzylhydroxylamine **5c** (40 mg, 0.11 mmol) in 2.0 mL of MeOH, 3-acetyl-2,4-dimethylpyrrole **1a** (15 mg, 0.11 mmol) and acetyl chloride (9 mg, 0.11 mmol). The mixture was stirred at -20°C for 5 hours. Purification (eluent: pentane/EtOAc, 3/2) afforded unsymmetrical 2,2'-bis(pyrrolyl)alkane **7c** (25 mg, 66 µmol, 60%) as a yellow solid. **Mp:** 67-68°C.
**IR (KBr disc):** 3335, 2960, 2925, 2860, 1630, 1440, 1410 cm⁻¹.
**¹H NMR (400 MHz, CDCl₃)** δ (ppm) 1.05 (3H, t, *J* = 7.5 Hz, CH₃), 1.77 (3H, s, CH₃), 2.07 (3H, s, CH₃), 2.08 (3H, s, CH₃), 2.34-2.41 (8H, m, CH₂, 2CH₃), 3.79 (3H, s, OCH₃), 5.40 (1H, s, H₁), 6.84 (2H, d, *J* = 8.8 Hz, 2H_{Ar}), 6.93 (1H, br s, NH), 7.02 (2H, d, *J* = 8.8 Hz, 2H_{Ar.}), 7.59 (1H, br s, NH).
**¹³C NMR (75 MHz, CDCl₃)** δ (ppm) 9.2 (CH₃), 11.2 (CH₃), 11.9 (CH₃), 15.3 (CH₃), 15.8 (CH₃), 17.8 (CH₂), 31.1 (CH₃), 39.4 (CH₃), 55.4 (CH, C₁), 114.3 (2CH_{Ar}), 115.8 (C_{Pyr}), 121.5 (C_{Pyr}), 121.7 (C_{Pyr}), 122.2 (C_{Pyr}), 124.2 (2C_{Pyr}), 127.3 (C_{Pyr}), 129.4 (2CH_{Ar}), 133.1 (C_{pyr}), 133.5 (C_{Ar}), 158.5 (C-OCH₃), 195.9 (CO).
**LRMS (ESI⁺):** *m*/*z* 401 ([M+Na]⁺, 50), 379 ([M+H]⁺, 50).
**HRMS (ESI⁺) Calcd for C₂₄H₃₀O₂N₂Na** 401.2199. **Found:** 401.2198 ([M+Na]⁺).

### Example 17: 1-(5-((4-Ethyl-3,5-dimethyl-1H-pyrrol-2-yl)(4-iodophenyl)methyl)-2,4-dimethyl-1H-pyrrol-3-yl)ethanone 7d

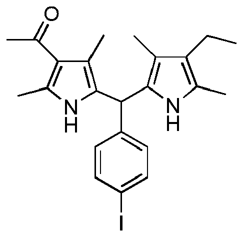

Prepared according to the above general procedure from the corresponding *N*-benzylhydroxylamine **5d** (40 mg, 84 µmol) in 2.0 mL of MeOH, 2,4-dimethyl-3-ethylpyrrole **1b** (11 mg, 89 µmol) and acetyl chloride (7 mg, 89 µmol). The mixture was stirred at 0°C for 1.5 hour. Purification (eluent: CH₂Cl₂/EtOAc, 95/5) afforded unsymmetrical 2,2'-bis(pyrrolyl)alkane **7d** (38 mg, 80 µmol, 95%) as an orange solid.
**Mp:** 84-85°C.
**IR (KBr disc):** 3420, 2960, 2920, 2850, 1615, 1475, 1440, 1010 cm⁻¹.
**¹H NMR (400 MHz, CDCl₃)** δ (ppm) 1.05 (3H, t, *J* = 7.5 Hz, CH₃), 1.77 (3H, s, CH₃), 2.05 (3H, s, CH₃), 2.09 (3H, s, CH₃), 2.35-2.42 (8H, m, CH₂, 2CH₃), 5.40 (1H, s, H₁), 6.86 (2H, d, *J*= 7.5 Hz, 2H_{Ar}), 6.93 (1H, br s, NH), 7.58 (1H, br s, NH), 7.62 (2H, d, *J* = 7.5 Hz, 2H_{Ar}).
**¹³C NMR (100 MHz, CDCl₃)** δ (ppm) 9.3 (CH₃), 11.2 (CH₃), 12.0 (CH₃), 15.3 (CH₃), 15.7 (CH₃), 17.8 (CH₂), 31.1 (CH₃), 39.7 (CH, C₁), 92.3 (C-I), 114.7 (C_{Pyr}), 116.2 (C_{Pyr}), 121.8 (C_{Pyr}), 122.0 (C_{Pyr}), 122.2 (C_{Pyr}), 123.1 (C_{Pyr}), 126.3 (C_{Pyr}), 130.5 (2CH_{Ar}), 133.5 (C_{Ar}), 138.0 (2CH_{Ar}), 141.4 (C_{Pyr}), 195.8 (CO).
**LRMS (ESI⁺)** *m*/*z* 497 ([M+Na]⁺, 7), 475 ([M+H]⁺, 93).
**HRMS (ESI⁺) Calcd for C₂₃H₂₇ON₂INa** 497.1060. **Found:** 497.1051 ([M+Na]⁺).

### Example 18: 5-((5-Methyl-1H-pyrrol-2-yl)(phenyl)methyl)-1H-pyrrol-3-yl)ethanone 7e

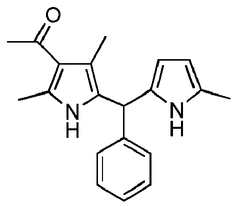

Prepared according to the above general procedure from the corresponding *N*-benzylhydroxylamine **5b** (34 mg, 0.10 mmol) in 2.0 mL of MeOH, 2-methylpyrrole **1c** (8 mg, 0.10 mmol) and acetyl chloride (8 mg, 0.10 mmol). The mixture was stirred at 0°C for 1 hour. Purification (eluent: pentane/EtOAc, 3/2) afforded unsymmetrical 2,2'-bis(pyrrolyl)alkane **7e** (29 mg, 95 µmol, 95%) as a white solid.
**Mp:** 72-73 °C.
**IR (KBr disc):** 33140, 2950, 2920, 2850, 1630, 1480, 1440, 1415 cm⁻¹.
**¹H NMR (400 MHz, CDCl₃)** δ (ppm) 2.15 (3H, s, CH₃), 2.21 (3H, s, CH₃), 2.39 (3H, s, CH₃), 2.42 (3H, s, CH₃), 5.44 (1H, s, H₁), 5.66 (1H, br s, H_{Pyr}), 5.80 (1H, br s, H_{Pyr}), 7.17 (2H, d, *J* = 7.2 Hz, 2H_{Ar}), 7.27-7.34 (3H, m, 3H_{Ar}), 7.61 (1H, br s, NH), 7.72 (1H, br s, NH).
**¹³C NMR (100 MHz, CDCl₃)** δ (ppm) 11.9 (CH₃), 13.2 (CH₃), 15.3 (CH₃), 31.1 (CH₃), 41.5 (CH, C₁), 106.2 (CH_{Pyr}), 108.0 (CH_{Pyr}), 116.3 (C_{Pyr}), 122.0 (C_{Pyr}), 126.8 (C_{Pyr}), 127.1 (CH_{Ar}), 127.7 (C_{Pyr}), 128.3 (2CH_{Ar}), 128.9 (2CH_{Ar}), 130.3 (C_{Pyr}), 133.6 (C_{Ar}), 141.6 (C_{Pyr}), 195.7 (CO).
**LRMS (ESI⁺):** *m*/*z* 329 ([M+Na]⁺, 50), 307 ([M+H]⁺, 50).
**HRMS (ESI⁺) Calcd for C₂₀H₂₂N₂ONa** 329.1624. **Found:** 309.1621 ([M+Na]⁺).

### Example 19: 1-(5-((4-Iodophenyl)(5-methyl-1H-pyrrol-2-yl)methyl)-2,4-dimethyl-1H-pyrrol-3-yl)ethanone 7f

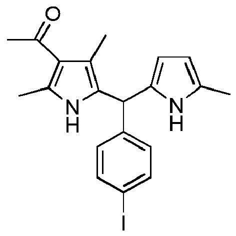

Prepared according to the above general procedure from the corresponding *N*-benzylhydroxylamine **5d** (40 mg, 84 µmol) in 2.0 mL ofMeOH, 2-methylpyrrole **1c** (7 mg, 86 µmol) and acetyl chloride (7 mg, 89 µmol). The mixture was stirred at 0°C for 1.5 hour. Purification (eluent: pentane/EtOAc, 1/1) afforded unsymmetrical 2,2'-bis(pyrrolyl)alkane **7f** (36 mg, 83 µmol, 98%) as a yellow solid.
**Mp:** 108-109°C.
**IR (KBr disc):** 3410, 2970, 2920, 2850, 1620, 1435, 1000 cm⁻¹.
**¹H NMR (400 MHz, CDCl₃)** δ (ppm) 2.12 (3H, s, CH₃), 2.21 (3H, s, CH₃), 2.40 (3H, s, CH₃), 2.42 (3H, s, CH₃), 5.37 (1H, s, H₁), 5.63-5.65 (1H, m, H_{Pyr}), 5.79-5.81 (1H, m, H_{Pyr}), 6.91 (2H, d, *J* = 8.4 Hz, 2H_{Ar}), 7.57 (1H, br s, NH), 7.62-7.64 (3H, m, 2H_{Ar} and NH).
**¹³C NMR (100 MHz, CDCl₃)** δ (ppm) 11.8 (CH₃), 13.2 (CH₃), 15.3 (CH₃), 31.1 (CH₃), 41.1 (CH, C₁), 92.4 (C-I), 106.4 (CH_{Pyr}), 108.2 (CH_{Pyr}), 116.5 (C_{Pyr}), 122.1 (C_{Pyr}), 126.1 (C_{Pyr}), 128.1 (C_{Pyr}), 129.6 (C_{Pyr}), 130.3 (2CH_{Ar}), 133.7 (C_{Ar}), 137.9 (2CH_{Ar}), 141.4 (C_{Pyr}), 195.6 (CO).
**LRMS (ESI⁺):** *m*/*z* 433 ([M+H]⁺, 100).
**HRMS (ESI⁺) Calcd for C₂₀H₂₁ON₂INa** 455.0590. **Found:** 455.0596 ([M+Na]⁺).

### Example 20: 1-(2,4-Dimethyl-5-(phenyl(1H-pyrrol-2-yl)methyl)-1H-pyrrol-3-yl)ethanone 7g

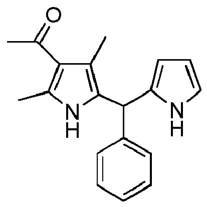

Prepared according to the above general procedure from the corresponding *N*-benzylhydroxylamine **5b** (40 mg, 0.12 mmol) in 2.0 mL of MeOH, pyrrole **1d** (16 mg, 0.24 mmol) and acetyl chloride (9 mg, 0.12 mmol). The mixture was stirred at 0°C for 1 hour. Purification (eluent: pentane/EtOAc, 1/1) afforded unsymmetrical 2,2'-bis(pyrrolyl)alkane **7g** (14 mg, 48 µmol, 41%) as a white solid and a mixture of tripyrromethane and BnNHOH. The latter mixture was washed twice with aqueous HCl 0.1 N to afford tripyrromethane **6a** (12 mg, 23 µmol, 38%).
**Mp:** 71-72°C.
**IR (KBr disc):** 3420, 2930, 1625, 1436, 1414 cm⁻¹.
**¹H NMR (400 MHz, CDCl₃)** δ (ppm) 2.15 (3H, s, CH₃), 2.40 (3H, s, CH₃), 2.41 (3H, s, CH₃), 5.50 (1H, s, H₁), 5.84 (1H, br s, H_{Pyr}), 6.17 (1H, br s, H_{Pyr}), 6.99 (1H, br s, H_{Pyr}), 7.16-7.18 (2H, m, 2H_{Ar}), 7.27-7.35 (3H, m, 3H_{Ar}), 7.58 (1H, br s, NH), 7.83 (1H, br s, NH).
**¹³C NMR (100 MHz, CDCl₃)** δ (ppm) 11.9 (CH₃), 15.3 (CH₃), 31.1 (CH₃), 41.5 (CH, C₁), 107.8 (CH_{Pyr}), 108.8 (CH_{Pyr}), 116.5 (C_{Pyr}), 117.6 (CH_{Pyr}), 122.0 (C_{Pyr}), 126.6 (C_{Pyr}), 127.2 (CH_{Ar}), 128.3 (2CH_{Ar}), 128.9 (2CH_{Ar}), 131.7 (C_{Pyr}), 133.7 (C_{Ar}), 141.4 (C_{Pyr}), 195.8 (CO). **LRMS (ESI⁺):** *m*/*z* 607 ([2M+Na]⁺, 10), 315 ([M+Na]⁺, 20), 293 ([M+H]⁺, 70).
**HRMS (ESI⁺) Calcd for C₁₉H₂₀N₂ONa** 315.1467. **Found:** 315.1465 ([M+Na]⁺).

### Example 21: 1-(5-((4-Iodophenyl)(1H-pyrrol-2-yl)methyl)-2,4-dimethyl-1H-pyrrol-3-yl)ethanone 7h

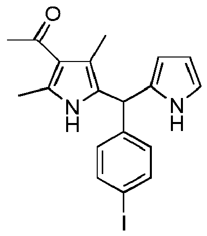

Prepared according to the above general procedure from the corresponding *N*-benzylhydroxylamine **5d** (40 mg, 84 µmol) in 2.0 mL of MeOH, pyrrole **1d** (11 mg, 0.16 mmol) and acetyl chloride (7 mg, 89 µmol). The mixture was stirred at 0°C for 1 hour. Purification (eluent: pentane/EtOAc, 1/1) afforded unsymmetrical 2,2'-bis(pyrrolyl)alkane **7h** (21 mg, 50 µmol, 59%) as a bright pink solid and a mixture of tripyrromethane and BnNHOH. The latter mixture was washed twice with aqueous HCl 0.1 N to afford tripyrromethane **6b** (10 mg, 13 µmol, 31%).
**Mp:** 82°C.
**IR (KBr disc):** 3360, 2915, 2960, 1630, 1465, 1440, 1420, 1270, 1000 cm⁻¹.
**¹H NMR (400 MHz, CDCl₃)** δ (ppm) 2.12 (3H, s, CH₃), 2.37 (3H, s, CH₃), 2.40 (3H, s, CH₃), 5.44 (1H, s, H₁), 5.81 (1H, s, H_{Pyr}), 6.16 (1H, s, H_{Pyr}), 6.73 (1H, s, H_{Pyr}), 6.90 (2H, d, *J* = 7.6 Hz, 2H_{Ar}), 7.62 (2H, d, *J* = 7.6 Hz, 2H_{Ar}), 7.78 (1H, br s, NH), 8.12 (1H, br s, NH).
**¹³C NMR (100 MHz, CDCl₃)** δ (ppm) 11.9 (CH₃), 15.3 (CH₃), 31.1 (CH₃), 41.0 (CH, C₁), 92.5 (C-1), 108.0 (CH_{Pyr}), 108.8 (CH_{Pyr}), 116.7 (C_{Pyr}), 117.9 (CH_{Pyr}), 122.0 (C_{Pyr}), 126.1 (C_{Pyr}), 130.3 (2CH_{Ar}), 131.0 (C_{Pyr}), 134.0 (C_{Ar}), 137.9 (2CH_{Ar}), 141.2 (C_{Pyr}), 195.8 (CO). **LRMS (ESI⁺):** *m*/*z* 441 ([M+Na]⁺, 6), 419 ([M+H]⁺, 94).
**HRMS (ESI⁺) Calcd for C₁₉H₁₉ON₂INa** 441.0434. **Found:** 441.0438 ([M+Na]⁺).

### General procedure for the preparation of tripyrromethanes 6a-d

These compounds have been prepared in one step according to M. L. Murat-Onana, C. Berini, F. Minassian, N. Pelloux-Léon, J.-N. Denis Org. Biomol. Chem. 2010, 8, 2204-2211 **(Scheme V).**

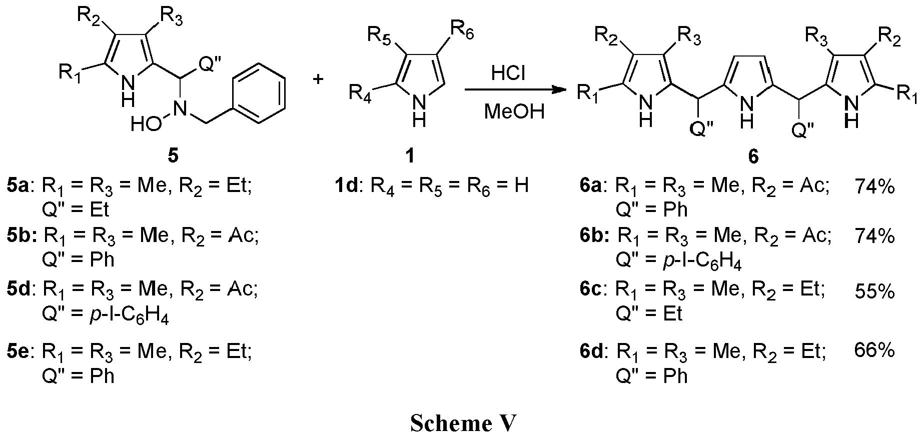

Freshly distilled acetyl chloride (1 equiv.) was added dropwise at 0°C to anhydrous methanol (0.2-0.5 mM) and the mixture was stirred for 15 min. The appropriate pyrrolic *N*-benzylhydroxylamine **5** (1 equiv.) was added and the mixture was cooled at -78°C before the addition of freshly distilled pyrrole **1d** (0.5 equiv.). The mixture was warmed to the suitable temperature and stirred until complete disappearance of the starting material (followed by TLC). The mixture was treated with saturated aqueous NaHCO₃ solution. The pH value was 8-9. The aqueous layer was then extracted three times with CH₂Cl₂. The combined organic layers were washed with brine, dried over anhydrous MgSO₄ and concentrated. The obtained tripyrromethane **6** was purified by flash chromatography on silica gel.

### Example 22: 1,1'-(5,5'-(1H-Pyrrol-2,5-diyl)bis(phenylmethylene)bis(2,4-dimethyl-1H-pyrrole-5,3-diyl))diethanone (6a).

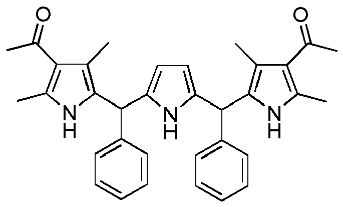

Prepared according to the above general procedure from *N*-benzylhydroxylamine **5b** (40 mg, 0.12 mmol) in 2.0 mL of MeOH, pyrrole **1d** (4 mg, 59 µmol) and acetyl chloride (9 mg, 0.11 mmol). The mixture was stirred at 0°C for 30 minutes. The medium was then treated with aqueous HCl 0.1N and flash purification (eluent: pentane/EtOAc, 1/1) afforded tripyrromethane **6a** (22 mg, 42 µmol, 74%) as a beige solid.
**Mp:** 148-149°C.
**IR (KBr disc):** 3420, 3315, 2960, 2915, 1630, 1600, 1445, 1410, 1365, 1240 cm⁻¹.
**¹H NMR (400 MHz, CDCl₃) δ** (ppm) 2.10 (6H, s, 2CH₃), 2.35 (6H, s, 2CH₃),2.38 (6H, s, 2CH₃), 5.40 (2H, s, H-1 and H-1'), 5.70 (2H, s, 2H_{Pyr}), 7.11-7.13 (4H, m, 4H_{Ar}),7.24-7.32 (6H, m, 6H_{Ar}), 7.75 (2H, br s, 2NH), 7.82 (1H, br s, NH).
**¹³C NMR (100 MHz, CDCl₃)** δ (ppm) 11.7 (2CH₃), 15.2 (2CH₃), 30.9 (2CH₃), 41.3 (2CH, C-1 and C-1'), 108.0(2CH_{Pyr}), 116.3 (2C_{pyr}), 121.8 (2C_{pyr}), 126.3 (2C_{pyr}), 127.1 (2CHₐᵣₒₘ), 128.1 (4CHₐᵣₒₘ), 128.7 (4CHₐᵣₒₘ), 131.8 (2C_{pyr}), 133.6 (2Cₐᵣₒₘ), 141.2 (2C_{pyr)}, 195.6 (2CO). **LRMS (ESI⁺):** *m*/*z* 540([M+Na]⁺, 23), 518 ([M+H]⁺, 77).
**HRMS (ESI⁺) Calcd for C₃₄H₃₅N₃O₂Na** 540.2621. **Found:** 540.2613 ([M+Na] ⁺).

### Example 23: 1,1'-(5,5'-(1H-Pyrrol-2,5-diyl)bis((4-iodophenyl)methylene)bis(2,4-dimethyl-1H-pyrrol-5,3-diyl))diethanone (6b).

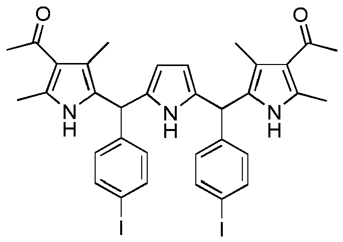

Prepared according to the above general procedure from *N*-benzylhydroxylamine **5d** (40 mg, 84 µmol) in 2.0 mL of MeOH, pyrrole **1d** (3 mg, 44 µmol) and acetyl chloride (7 mg, 89 µmol). The mixture was stirred at 0°C for 30 minutes. The medium was then washed with aqueous HCl 0.1N and flash purification (eluent: pentane/EtOAc, 1/1) afforded tripyrromethane **6b** (24 mg, 31 µmol, 74%) as a beige solid.
**Mp:** 177-178°C.
**IR (KBr disc):** 3275 (br NH), 2960, 2915, 28545, 1640, 1600, 1480, 1445, 1425, 1000 cm⁻¹. **¹H NMR (400 MHz, CDCl₃)** δ (ppm) 2.06 (3H, s, CH₃), 2.07 (3H, s, CH₃), 2.35 (3H, s, CH₃), 2.36 (3H, s, CH₃), 2.37 (3H, s, CH₃), 2.40 (3H, s, CH₃), 5.34 (2H, s, H-1 and H-1'), 5.67-5.69 (2H, m, 2H_{Pyr}), 6.85-6.88 (4H, m, 4Hₐᵣₒₘ), 7.61-7.63 (4H, d, *J* = 8.4 Hz, 4Hₐᵣₒₘ), 7.79 (1H, br s, NH), 7.84 (1H, br s, NH), 8.01 (1H, br s, NH).
**¹³C NMR (100 MHz, CDCl₃)**δ (ppm) 11.8 (2CH₃), 15.3 (2CH₃), 31.0 (2CH₃), 41.0 (CH, C-1), 41.1 (CH, C-1'), 92.7 (2C-I), 108.4 (2CH_{pyr}), 116.8 (C_{pyr}), 116.9 (C_{pyr}), 122.1 (2C_{pyr}), 125.8 (2C_{pyr}), 130.2 (4CHₐᵣₒₘ), 131.6 (C_{pyr}), 131.7 (C_{pyr}), 134.0 (2Cₐᵣₒₘ), 137.9 (4CHₐᵣₒₘ), 141.1 (2C_{pyr}), 195.7 (2CO).
**LRMS (ESI⁺):** *m*/*z* 792 ([M+Na]⁺, 33), 770 ([M+H]⁺, 67).
**HRMS (ESI⁺) Calcd for C₃₄H₃₃N₃O₂I₂Na** 792.0554. **Found:** 792.0565 ([M+Na]⁺).

### Example 24: 2,5-Bis(1-(4-ethyl-3,5-dimethyl-1H-pyrrol-2-yl)propyl)-1H-pyrrole (6c).

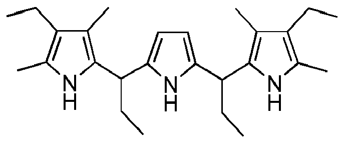

Prepared according to the above general procedure from *N*-benzylhydroxylamine **5a** (50 mg, 0.18 mmol) in 2.5 mL of MeOH, pyrrole **1d** (6 mg, 89 µmol) and acetyl chloride (14 mg, 0.18 mmol). The mixture was stirred at -40°C for 2 hours. Purification (eluent: pentane/EtOAc, 9/1) afforded tripyrromethane **6c** (19 mg, 48 µmol, 55%) as a yellow oil.
**IR (KBr disc):** 3365, 2960, 2925, 2860, 1600, 1565, 1460, 1150, 1100 cm⁻¹.
**¹H NMR (300 MHz, CDCl₃)** δ (ppm) 0.87-0.91 (6H, m, 2CH₃), 1.00-1.05 (6H, m, 2CH₃), 1.77-1.79 (2H, m, CH₃), 1.90 (3H, s, CH₃), 1.92 (3H, s, CH₃), 1.93-1.97 (2H, m, CH₂), 2.05 (3H, s, CH₃), 2.07 (3H, s, CH₃), 2.30-2.39 (4H, m, 2CH₂), 3.78 (2H, dd, *J* = 5.7 and 11.1 Hz, H-1 and H-1'), 5.97 (2H, dd, *J* = 2.1 and 5.7 Hz, 2HPyr), 7.06 (2H, br s, 2NH), 7.51 (1H, br s, NH).
**¹³CNMR (75 MHz, CDCl₃)** δ (ppm) 9.3 (2CH₃), 11.1 (2CH₃), 12.5 (2CH₃), 15.8 (2CH₃), 17.8 (2CH₂), 28.0 (2CH₂), 37.5 (CH, C-1), 37.7 (CH, C-1'), 104.1 (CH_{pyr}), 104.4 (CH_{pyr}), 113.5 (2C_{pyr}), 120.5 (2C_{pyr}), 120.8 (2C_{pyr}), 126.3 (2C_{pyr}), 133.2 (2C_{pyr}). **MS (ESI⁺):** *m*/*z* 394 ([M+H]⁺, 100).
**HRMS (ESI⁺) Calcd for C₂₆H₄₀N₃** 394.3222. **Found:** 394.3222 ([M+H]⁺).

### Example 25: 2,5-Bis((4-ethyl-3,5-dimethyl-1H-pyrrol-2-yl)(phenyl)methyl)-1H-pyrrole (6d).

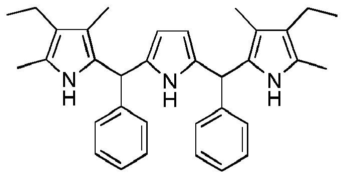

Prepared according to the above general procedure from *N*-benzylhydroxylamine **5e** (50 mg, 0.15 mmol) in 2.5 mL of MeOH, pyrrole (5 mg, 74 µmol) and acetyl chloride (12 mg, 0.15 mmol). The mixture was stirred at -40°C for 2 hours. Purification (eluent: pentane/EtOAc, 9/1) afforded tripyrromethane **6d** (24 mg, 49 µmol, 66%) as a red oil.
**IR (KBr disc):** 3420, 2950, 2920, 2850, 1590, 1460 cm⁻¹.
**¹H NMR (400 MHz, CDCl₃)** δ (ppm) 1.02 (6H, t, *J* = 7.6 Hz, 2CH₃), 1.78 (6H, s, 2CH₃), 2.06 (6H, s, 2CH₃), 2.33 (4H, q, *J* = 7.6 Hz, 2CH₂), 5.35 (2H, s, H-1 and H-1'), 5.67 (2H, br s, 2H_{pyr}), 7.08 (2H, br s, 2NH), 7.14-7.29 (10H, m, 10Hₐᵣₒₘ), 7.63 (1H, br s, NH). **¹³C NMR (75 MHz, CDCl₃)** δ (ppm) 7.5 (2CH₃), 9.6 (2CH₃), 14.3 (2CH₃), 16.3 (2CH₂), 41.6 (2CH, C-1 and C-1'), 108.0 (2CH_{pyr}), 114.6 (2C_{pyr}), 121.9 (2C_{pyr}), 122.0 (2C_{pyr}), 125.7 (2C_{pyr}), 127.6 (2CHₐᵣₒₘ), 129.4 (4CHₐᵣₒₘ), 129.6 (4CHₐᵣₒₘ), 133.5 (2Cₐᵣₒₘ), 143.9 (2C_{pyr}). **MS (ESI⁺):** *m*/*z* 490 ([M+H]⁺, 100).
**HRMS (ESI⁺) Calcd for C₃₄H₄ON₃** 490.3222. **Found:** 490.3222 ([M+H]⁺).

### Example 26: 1-(5-((5-(1-(4-Ethyl-3,5-dimethyl-1H-pyrrol-2-yl)propyl)-1H-pyrrol-2-yl)(4-iodophenyl)methyl)-2,4-dimethyl-1H-pyrrol-3-yl)ethanone (6e).

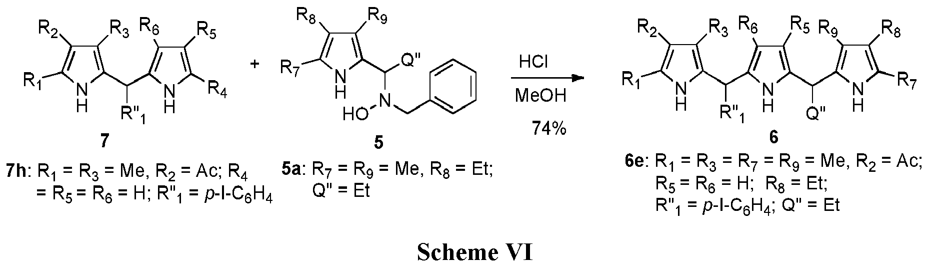

Freshly distilled acetyl chloride (6 mg, 76 µmol) was added dropwise to 2 mL of anhydrous methanol and the mixture was stirred for 15 min. The N-benzylhydroxylamine **5a** (23 mg, 80 µmol) was added and the mixture was cooled to -40°C before the addition of unsymmetrical 2,2(-bis(pyrrolyl)alkane **7h** (31 mg, 74 µmol). The medium was stirred from -40°C to -20°C during 3 hours. It was then treated with saturated aqueous NaHCO₃ solution (2 mL). The pH value was 8-9. The aqueous layer was then extracted three times with CH₂Cl₂ (3x5 mL). The combined organic layers were washed with brine (5 mL), dried over anhydrous MgSO₄ and concentrated. Purification by flash chromatography on silica gel (eluent: pentane/EtOAc, 1/1) afforded unsymmetrical tripyrromethane **6e** (32 mg, 55 µmol, 74%) as an orange oil.
**IR (KBr disc):** 3370, 2965, 2855, 1585, 1560, 1460, 1150, 1100 cm⁻¹.
**¹H NMR (300 MHz, CDCl₃)** δ (ppm) 0.88 (3H, t, *J* = 7.6 Hz, CH₃), 1.05 (3H, t, *J* = 7.6 Hz, CH₃), 1.77-1.80 (2H, m, CH₂), 1.90 (3H, s, CH₃), 1.92 (3H, s, CH₃), 1.95 (3H, s, CH₃), 2.05 (3H, s, CH₃), 2.07 (3H, s, CH₃), 2.30-2.34 (2H, m, CH₂), 3.78 (1H, t, *J* = 7.6 Hz, H-1'), 5.34 (1H, s, H-1), 5.67-5.69 (1H, m, H_{Pyr}), 5.97 (1H, br s, H_{Pyr}), 6.85-6.88 (2H, m, 2H_{Ar}), 7.61-7.63 (2H, d, *J* = 8.4 Hz, 2H_{Ar}), 7.79 (1H, br s, NH), 7.84 (1H, br s, NH), 8.01 (1H, br s, NH)
**¹³C NMR (75 MHz, CDCl₃)** δ (ppm) 9.3 (CH₃), 11.2 (CH₃), 11.8 (CH₃), 12.5 (CH₃), 15.3 (CH₃), 15.8 (CH₃), 17.8 (CH₂), 27.7 (CH₂), 31.1 (CH₃), 37.7 (CH, C-1'), 41.1 (CH, C-1), 92.4 (C-I), 104.8 (CH_{Pyr}), 108.0 (CH_{Pyr}), 113.7 (C_{Pyr}), 116.5 (C_{Pyr}), 121.1 (C_{Pyr}), 125.7 (C_{Pyr}), 126.1 (C_{Pyr}), 129.9 (C_{Pyr}), 130.1 (C_{Pyr}), 130.4 (C_{Pyr}), 133.7 (C_{Pyr}), 134.0 (2CH_{Ar}), 134.9 (C_{Ar}), 137.8 (2CH_{Ar}), 141.4 (C_{Pyr}), 195.6 (CO).
**LRMS (ESI⁺):** *m*/*z* 604 ([M+Na]⁺, 33), 582 ([M+H]⁺).

### EXPERIMENTAL PART - BIOLOGOGY

### Example 27: Antibacterial activity evaluation

### 27.1: Determinaton of the minimum inhibitory concentration (MIC)

The microdilution method recommended by the Clinical and Laboratory Standard Institute [M07-A8, Vol.29, N°2] was used. The activity of pyrrolic compounds was tested against 29 bacterial strains belonging to 17 different species and 12 different genera *(Staphylococcus, Streptococcus, Enterococcus, Listeria, Bacillus, Haemophilus, Escherichia, Klebsiella*, *Enterobacter*, *Serratia*, *Pseudomonas*, *and Acinetobacter*). Bacterial inocula were prepared in Mueller Hinton broth (MH2, bioMérieux, Marcy L'Etoile, France), supplemented with 10% sheep blood for fastidious species (*i.e.*, *Streptococcus pneumoniae* and *Haemophilus influenzae*). They were dispensed in 96 well microtiter plates (5 x 10⁵CFU/mL of final inoculum). Pyrrolic compounds were added to the wells as to obtain two-fold serial concentrations (0.25-64 mg/L). Plates were incubated at 37°C, in 5% CO₂ enriched atmosphere for fastidious species only. MICs were read after 18 hours incubation of cultures, and corresponded to the minimum pyrrolic compound concentration that allowed complete visual growth inhibition of bacteria. Drug-free cultures served as growth controls. Cultures receiving gentamicine, ciprofloxacine or cefotaxime served as positive controls. The results of MICs (mg/L) of the pyrollic compounds for the tested bacterial strains are given in the following Tables I and II:

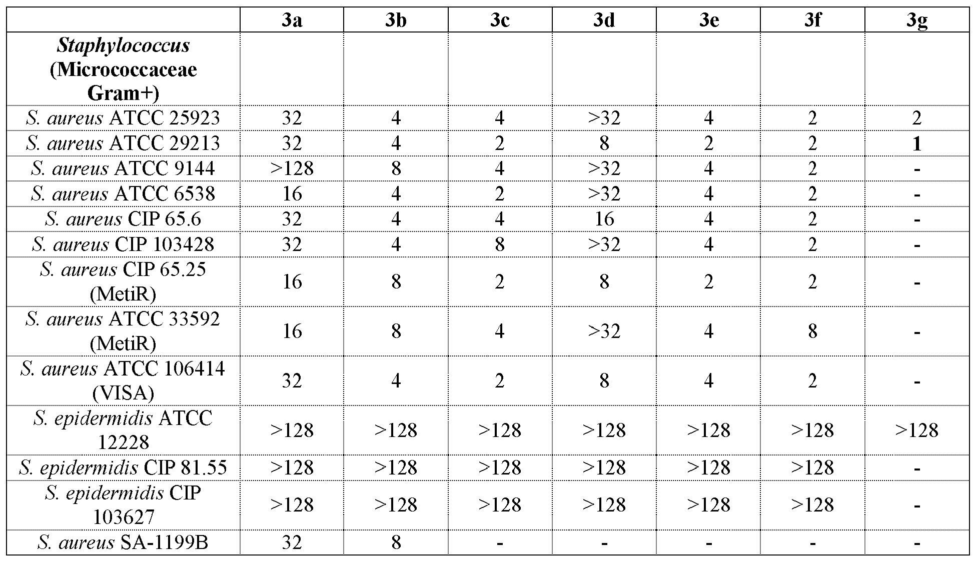

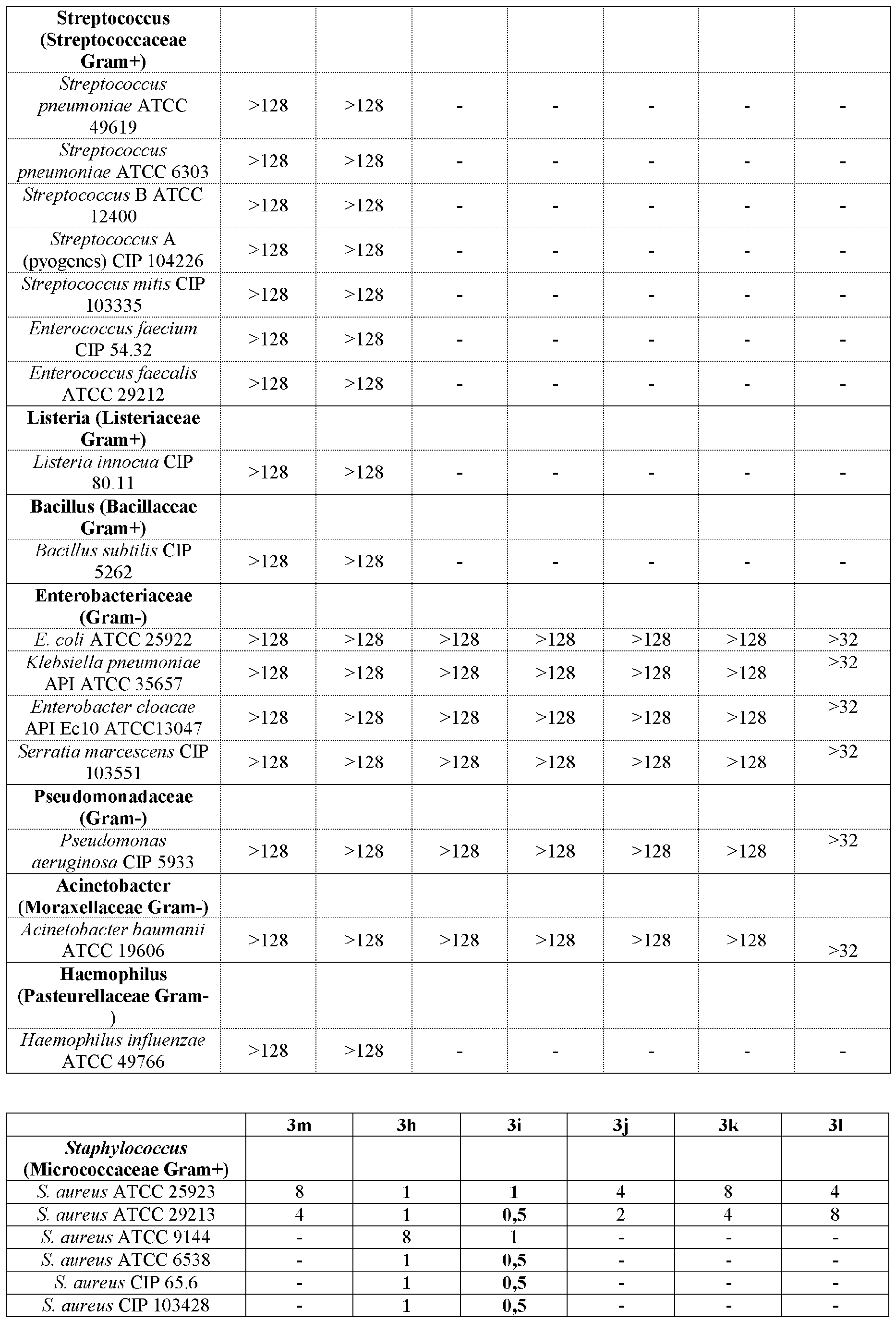

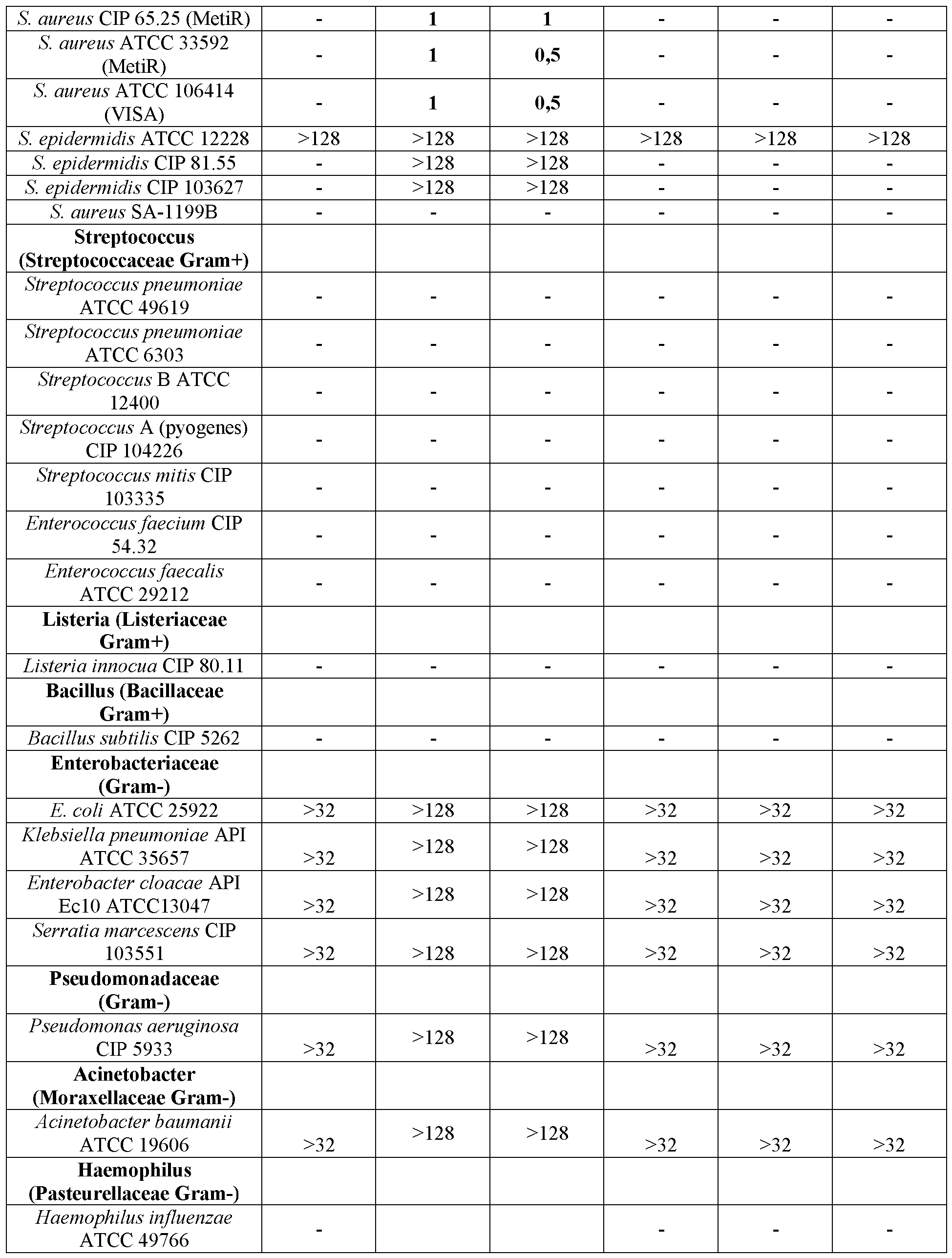

Compounds of the invention present a strong intrinsic antibacterial activity, mainly directed towards *Staphylococcus aureus.*

Compounds of the invention present an anti-*Staphylococcus* activity even against strains presenting an acquired resistance to beta-lactams, including methicillin-resistant *Staphylococcus aureus* (MRSA) strains such as CIP65.25 and ATCC 33592, and/or to glycopeptides such as the VISA/GISA (vancomycin/glycopeptide-intermediate *S. aureus*) strain CIP 106414. These multi-drug resistant bacterial strains are frequently implicated in severe hospital infections.

### 27.2 Cytotoxicity determination of the compounds of the invention

*In vitro* cytotoxicity was assayed on three cell lines - KB (human mouth carcinoma), MCR5 (human lung fibroblast) and HCT116 (human colon tumor). Results are presented in table III as % of cellular growth inhibition in presence of 10⁻⁵ M and 10⁻⁶ M of the tested bis-pyrrolic derivatives in DMSO (triplicate).

**TABLE III**

| **Compounds** | KB | HCT116 | MRC5 |
|---|---|---|---|
| | 10⁻⁵ M (10⁻⁶ M) | 10⁻⁵ M (10⁻⁶ M) | 10⁻⁵ M (10⁻⁶ M) |
| **3a** | **30** | **41** | - |
| **3b** | **32** | **43** | - |
| **3c** | **27±7** | **17±11** | **9±1** |
| **3d** | **0±4** | **0±9** | **0±6** |
| **3e** | **2±3** | **24±6** | **7±1** |
| **3f** | **23±3** | **19±4** | **0±8** |
| **3g** | **11±3** | **3±6** | **2±1** |
| **3h** | **54±4 (0±5)** | **53±2 (5±9)** | **25±4 (0±5)** |
| **3i** | **95±1 (0±7)** | **50±1 (10±6)** | **84±1 (0±7)** |
| **3j** | **11±3** | **1±3** | **5±1** |
| **3k** | **20±8** | **0±12** | **3±5** |
| **3l** | **9±5** | **11±7** | **6±3** |
| **3m** | **6±10** | **0±6** | **1±17** |

## Claims

1. Compounds of the following formula I: 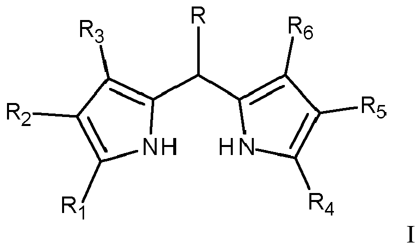 wherein:
R₁, R₂, R₃, R₄, R₅ and R₆ represent independently from each other:
- a H,
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
R represents:
- a H,
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CO₂-aryl, wherein aryl is an aromatic or heteroaromatic group,
- a CONH-(C₁-C₇)-alkyl,
said R₁, R₂, R₃, R₄, R₅, R₆ and R being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
said (C₁-C₇)-alkyl, (C₂-C₇)-alkenyl, (C₂-C₇)-alkynyl, (C₃-C₇)-cycloalkyl, (C₅-C₇)-cycloalkenyl, (C₃-C₇)-heterocycloalkyl, (C₅-C₇)-heterocycloalkenyl, CO-(C₁-C₇)-alkyl, CO₂-(C₁-C₇)-alkyl, CONH-(C₁-C₇)-alkyl, aryl, alkyl aryl and CO-aryl being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or
heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
and their pharmaceutically acceptable salts,
for use as antibacterial drugs against infection(s) caused by bacteria of the genus *Staphylococcus*, in particular *Staphylococcus aureus* species.

2. Compounds according to claims 1, wherein at least one of R₁, R₂, R₃, R₄, R₅ and R₆ represents:
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- CF₃,
- NO₂,
- CN.

3. Compounds according to anyone of claims 1 and 2, of one of the following formulae: 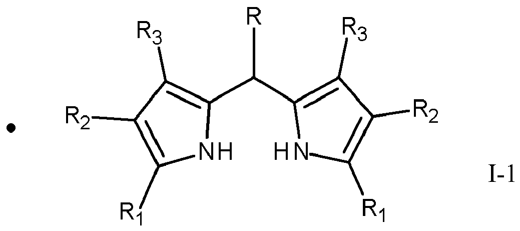 wherein R₁, R₂, R₃ and R are as defined in claim 1, R₂ representing preferably CO-CH₃, or 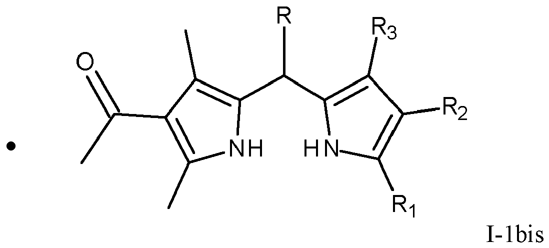 wherein R₁, R₂, R₃ and R are as defined in claim 1, or 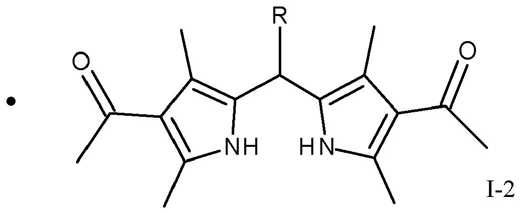 wherein R is as defined in claim 1, or 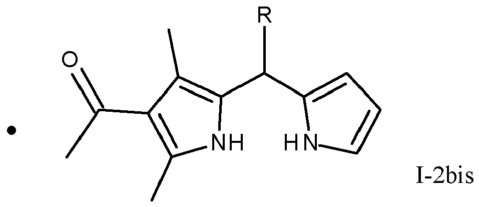 wherein R is as defined in claim 1, or 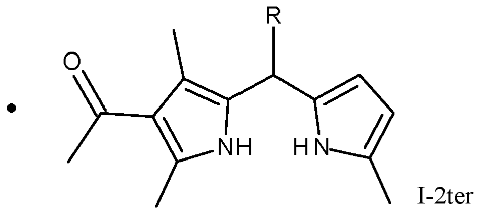 wherein R is as defined in claim 1, or 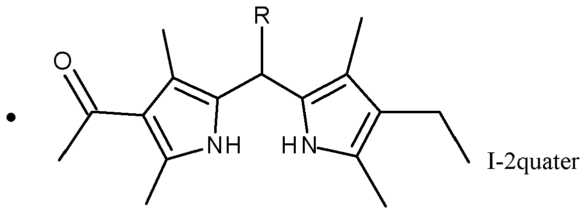 wherein R is as defined in claim 1.

4. Compounds according to anyone of claims 1 to 3, wherein R is selected from the group comprising: 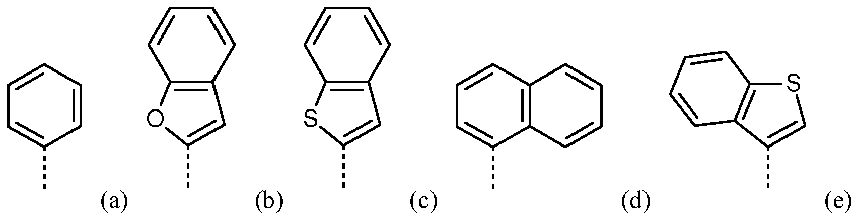 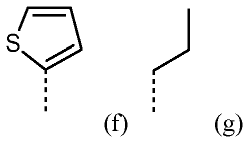 said R being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN.

5. Compounds, according to anyone of claims 1 to 4, of one of the following formulae: 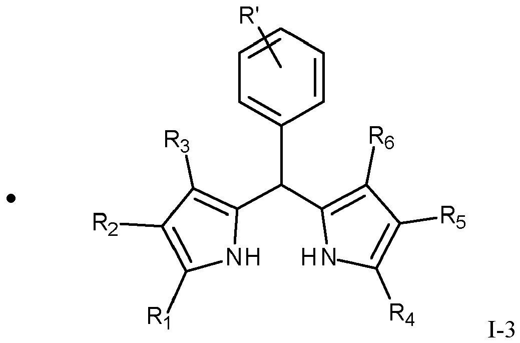 wherein R₁, R₂, R₃, R₄, R₅ and R₆ are as defined in claim 1, R₂ and R₅ representing preferably CO-CH₃, and
wherein R' represents H, or one or more substituent(s), preferably one substituent, each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN, or 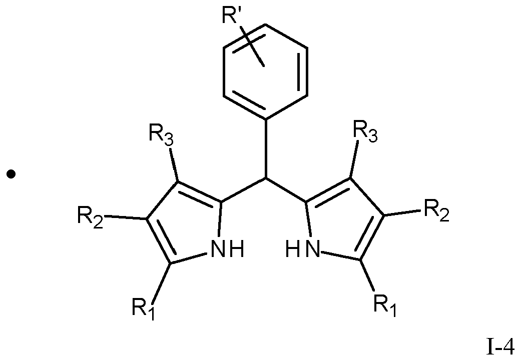 wherein R₁, R₂, R₃, and R' are as defined above, or 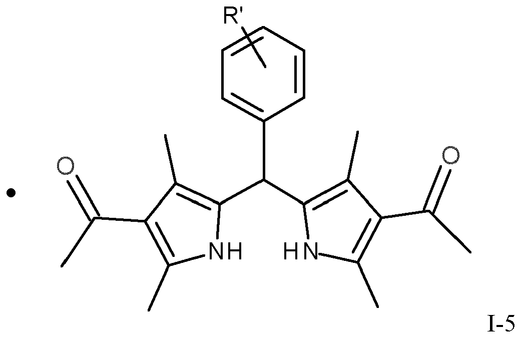 wherein R' is as defined above, or 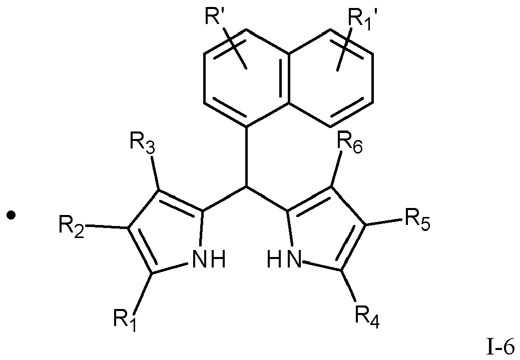 wherein R₁, R₂, R₃, R₄, R₅ and R₆ are as defined above, R₂ and R₅ representing preferably CO-CH₃, and
wherein R' and R₁' represent, independently from each other, H or one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
preferably, R' represents H and R₁' represents one substituent, or R' represents one substituent and R₁' represents H, or 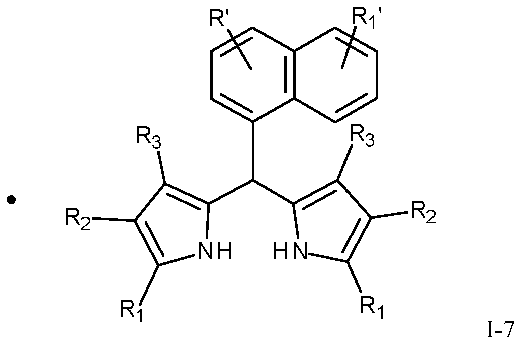 wherein R₁, R₂, R₃, R' and R₁' are as defined above, or 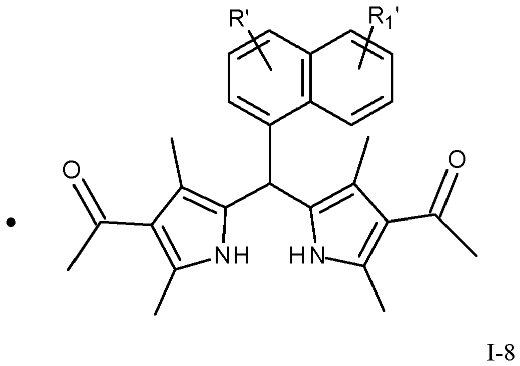 wherein R' and R₁' are as defined above, or 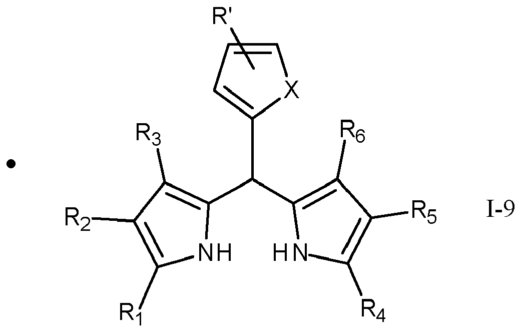 wherein R₁, R₂, R₃, R₄, R₅ and R₆ are as defined above,
wherein X is selected from the group comprising O, S and NR₂', X being preferably S, wherein R₂' represents H, a linear or branched (C₁-C₇)-alkyl, a CHO, a CO-(C₁-C₇)-alkyl, a CO-aryl, wherein aryl is an aromatic or heteroaromatic group, a CO₂-(C₁-C₇)-alkyl, a CONH-(C₁-C₇)-alkyl, a CF₃, or 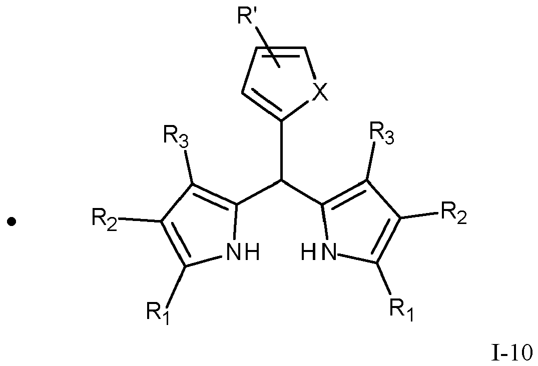 wherein R₁, R₂, R₃, R' and X are as defined above, or 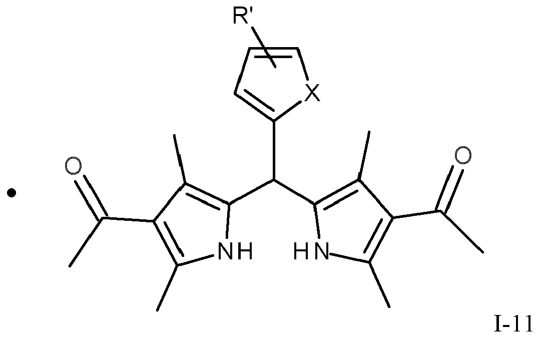 wherein R' and X are as defined above, or 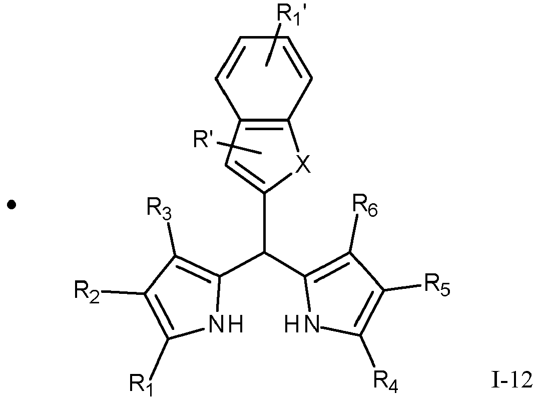 wherein R₁, R₂, R₃, R₄, R₅ and R₆ are as defined above, R₂ and R₅ representing preferably CO-CH₃, and
wherein X is selected from the group comprising O, S and NR₂', X being preferably S or O, wherein R₂' represents H, a linear or branched (C₁-C₇)-alkyl, a CHO, a CO-(C₁-C₇)-alkyl, a CO-aryl, wherein aryl is an aromatic or heteroaromatic group, a CO₂-(C₁-C₇)-alkyl, a CONH-(C₁-C₇)-alkyl, a CF₃, or 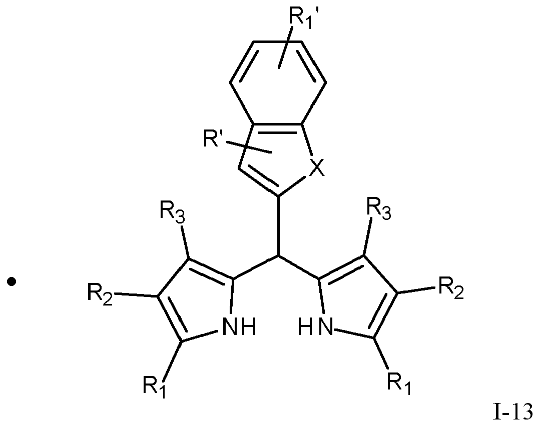 wherein R₁, R₂, R₃, R', R₁' and X are as defined above, or 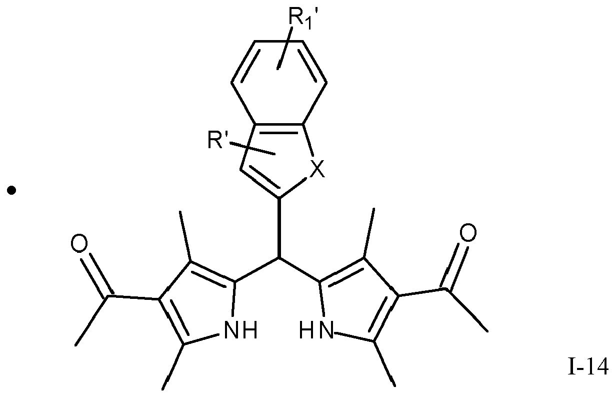 wherein R', R₁' and X are as defined above, or 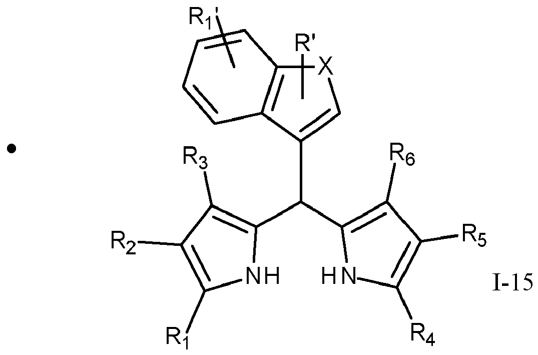 wherein R₁, R₂, R₃, R₄, R₅ and R₆ are as defined above, R₂ and R₅ representing preferably CO-CH₃, and
wherein X is selected from the group comprising O, S and NR₂', X being preferably S, wherein R₂' represents H, a linear or branched (C₁-C₇)-alkyl, a CHO, a CO-(C₁-C₇)-alkyl, a CO-aryl, wherein aryl is an aromatic or heteroaromatic group, a CO₂-(C₁-C₇)-alkyl, a CONH-(C₁-C₇)-alkyl, a CF₃, or 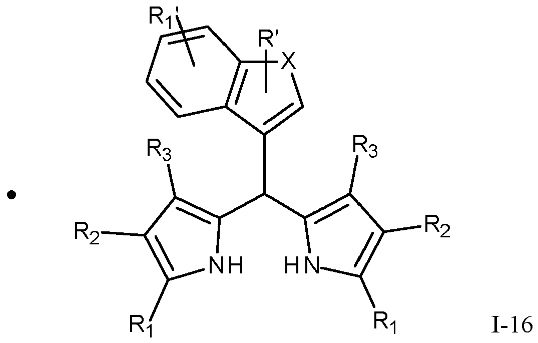 wherein R₁, R₂, R₃, R', R₁' and X are as defined above, or 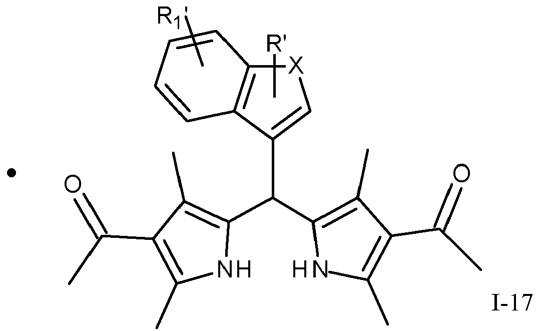 wherein R', R₁' and X are as defined above.

6. Compounds, according to claim 1, of the following formula: 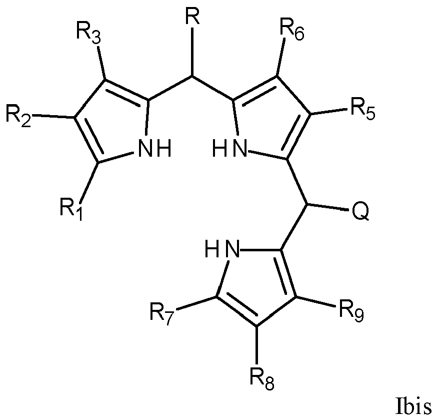 wherein R₁, R₂, R₃, R₅, R₆, and R are as defined in claim 1, and wherein:
R₇, R₈ and R₉ represent independently from each other:
- a H,
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
Q represents:
- a H,
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
said (C₁-C₇)-alkyl, (C₂-C₇)-alkenyl, (C₂-C₇)-alkynyl, (C₃-C₇)-cycloalkyl, (C₅-C₇)-cycloalkenyl, (C₃-C₇)-heterocycloalkyl, (C₅-C₇)-heterocycloalkenyl, CO-(C₁-C₇)-alkyl, CO₂-(C₁-C₇)-alkyl, CONH-(C₁-C₇)-alkyl, aryl, alkyl aryl and CO-aryl being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
and in particular, compounds of one of the following formula: 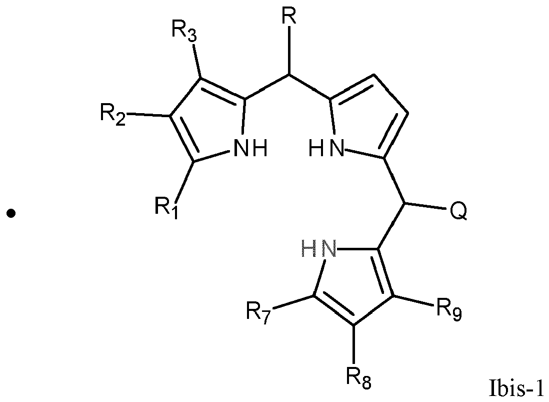 wherein R₁, R₂, R₃, R₇, R₈, R₉, Rand Q are as defined above, or 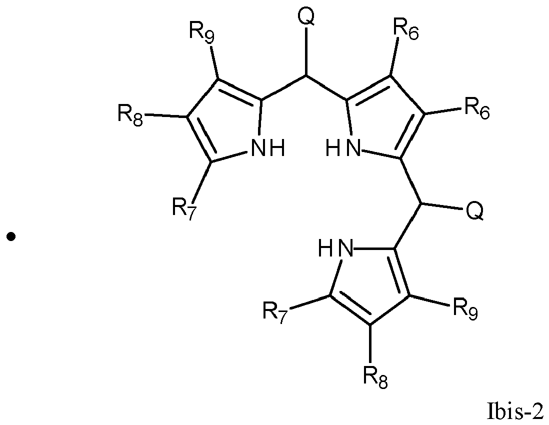 wherein R₆, R₇, R₈, R₉, and Q are as defined above, R₆ representing preferably H, or 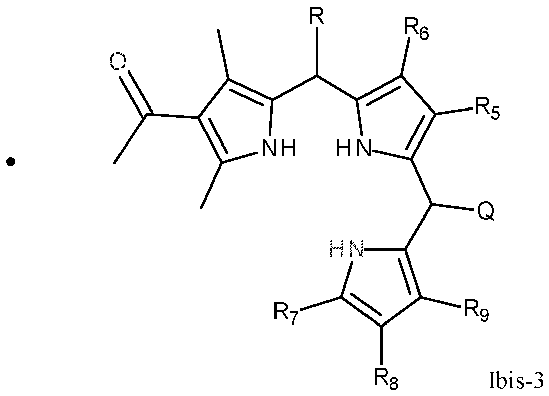 wherein R₅, R₆, R₇, R₈, R₉, R and Q are as defined above, R₆ representing preferably H, or 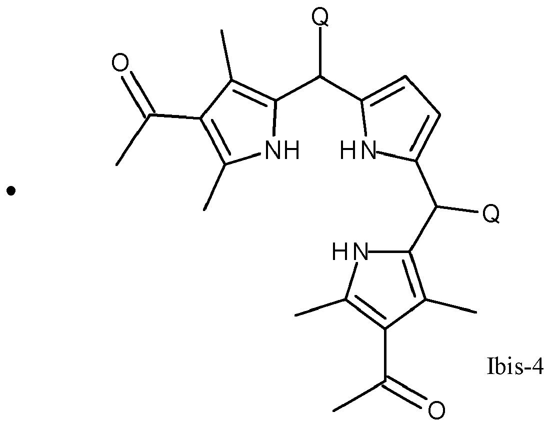 wherein Q is as defined above, or 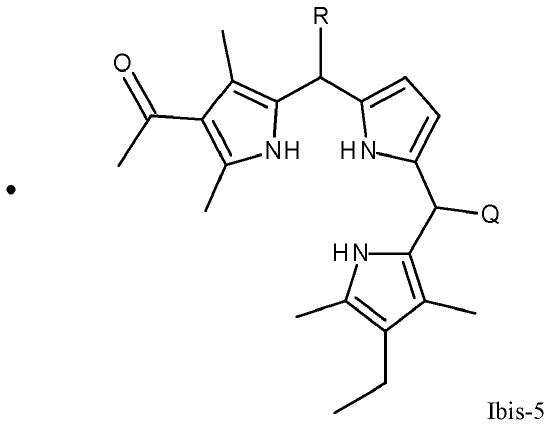 wherein R and Q are as defined above,
in particular, R and Q being, independently from each other, selected from the group comprising: 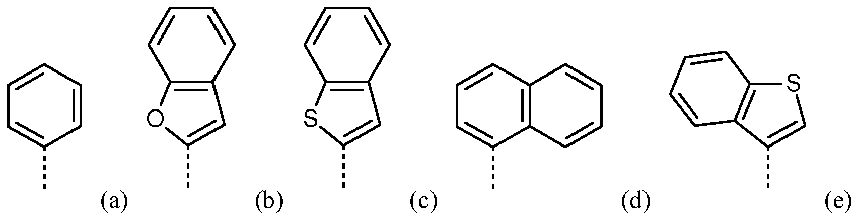 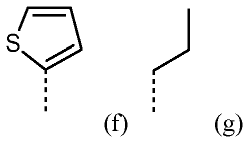 wherein the aryls (a), (b), (c), (d), (e) and (f) are substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN.

7. Compounds, according to claim 1, selected from the group comprising:

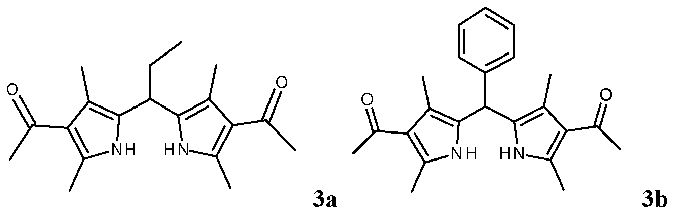

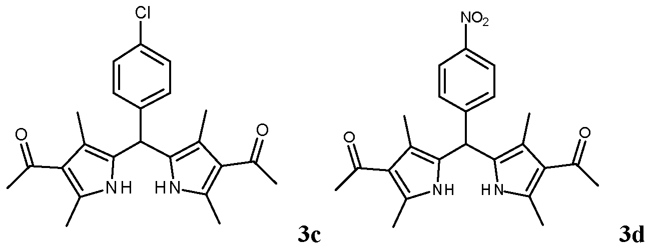

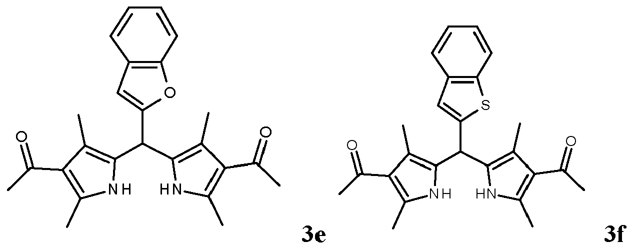

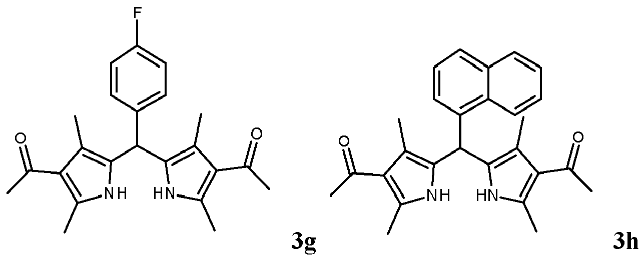

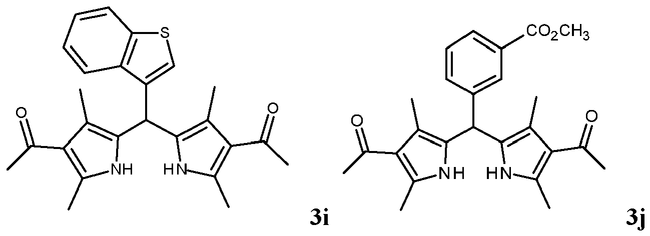

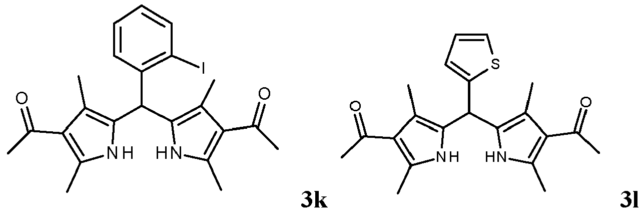

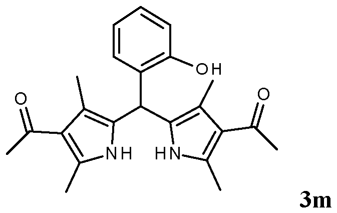

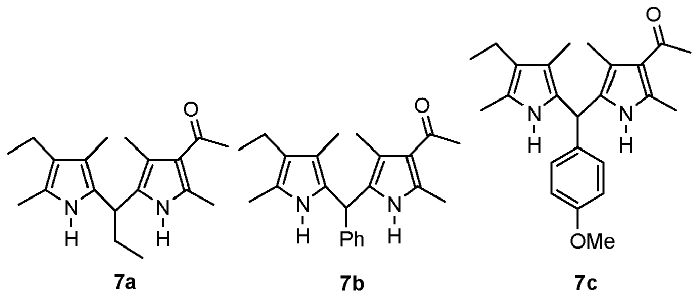

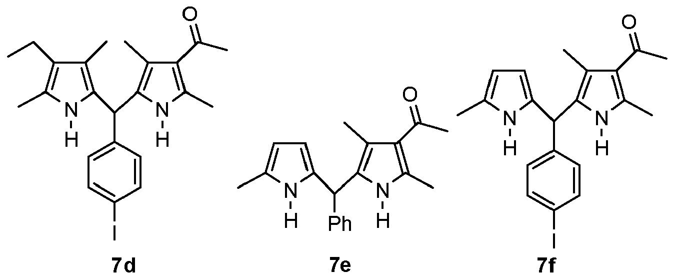

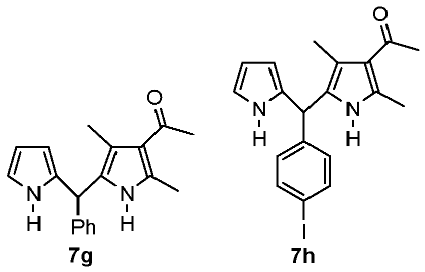

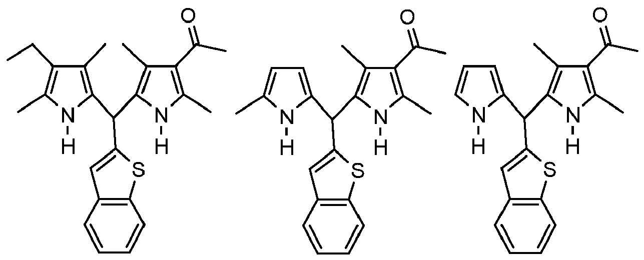

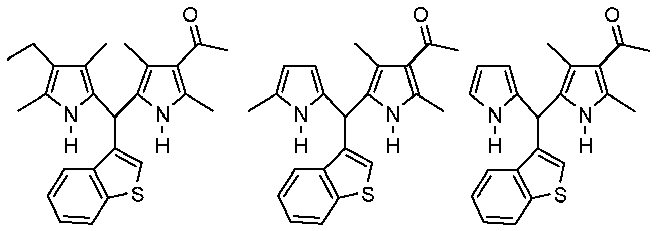

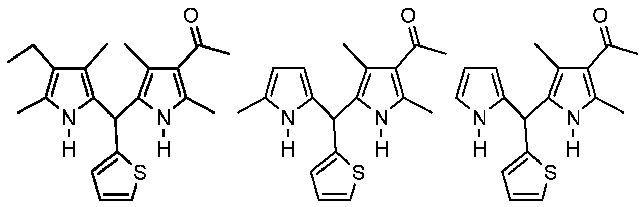

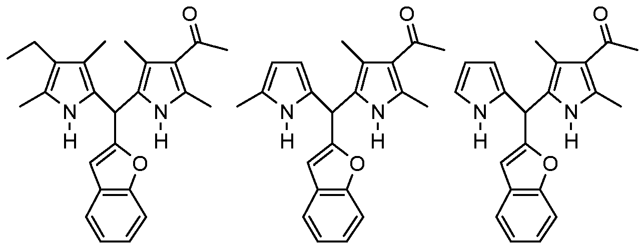

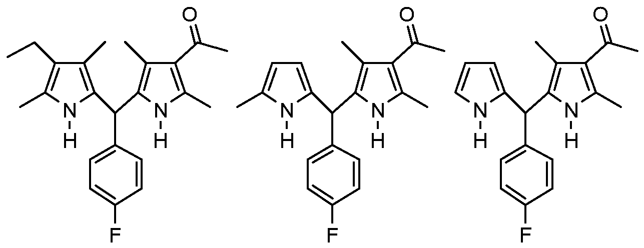

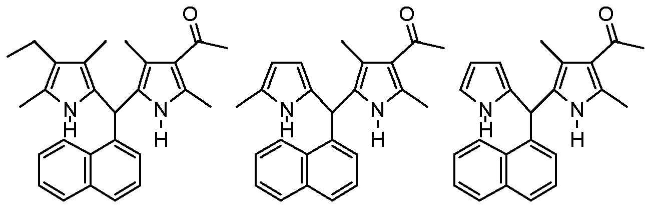

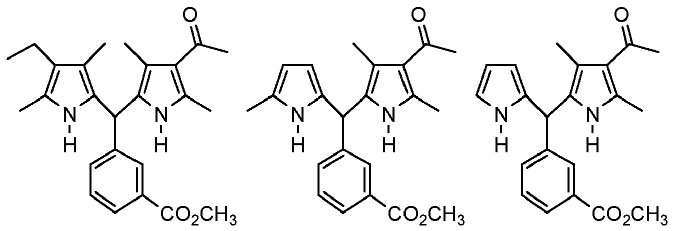

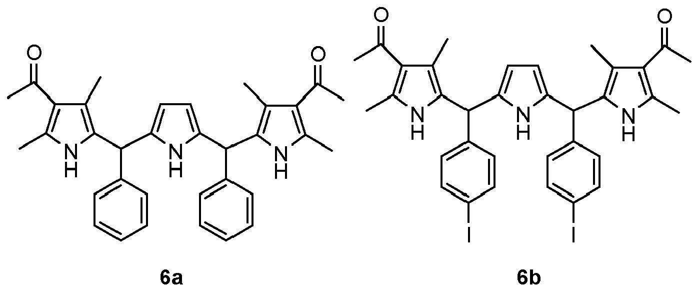

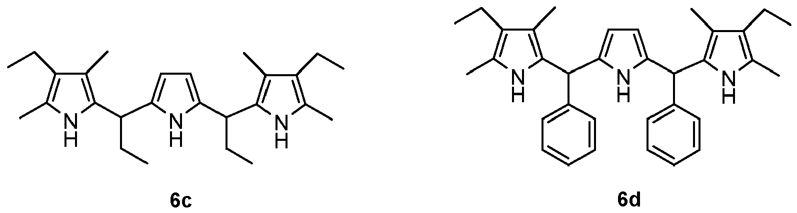

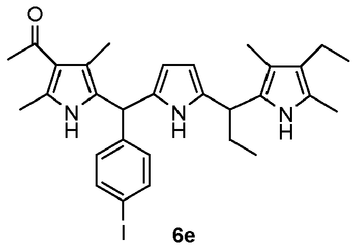

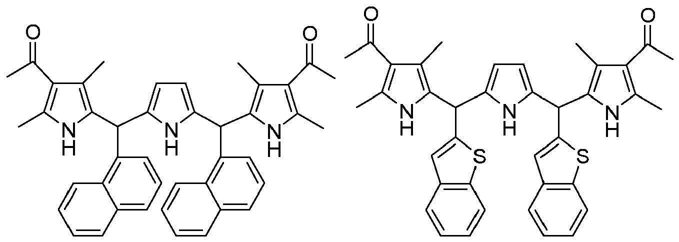

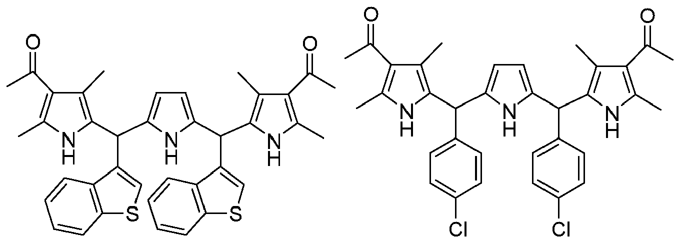

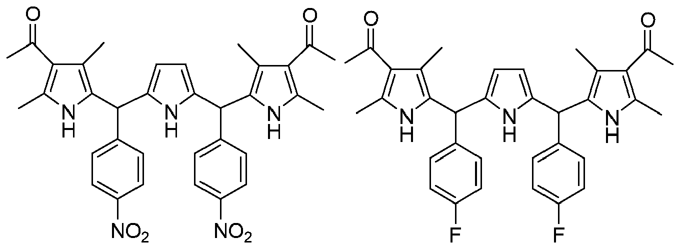

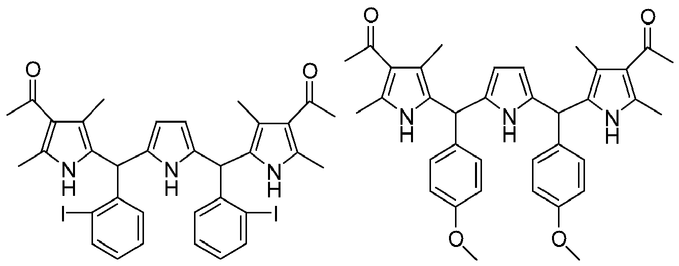

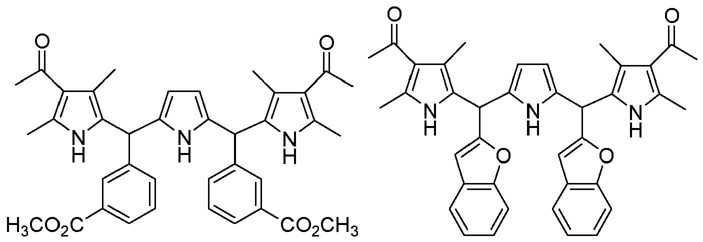

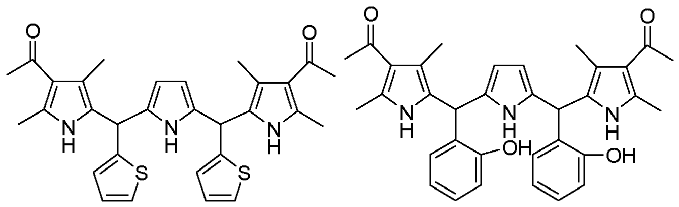

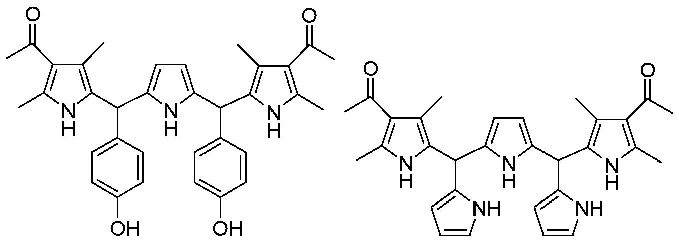

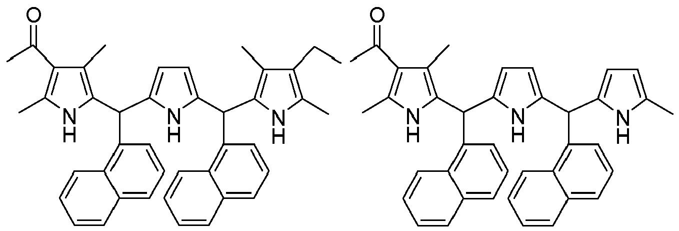

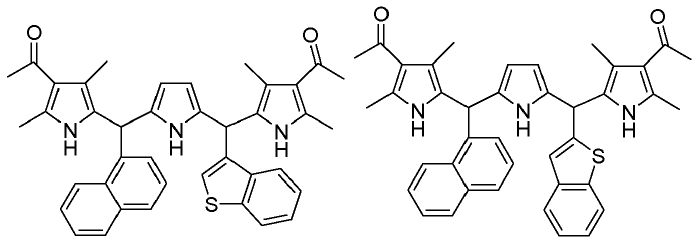

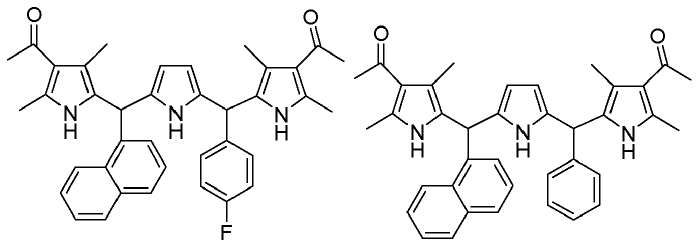

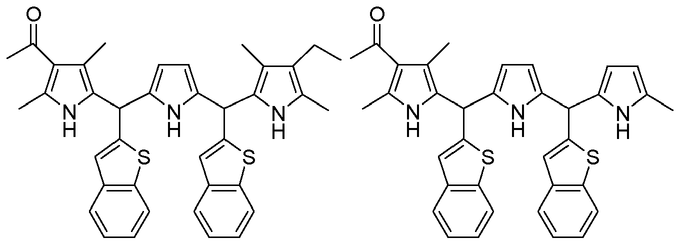

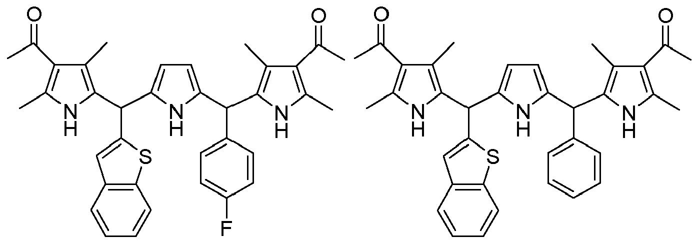

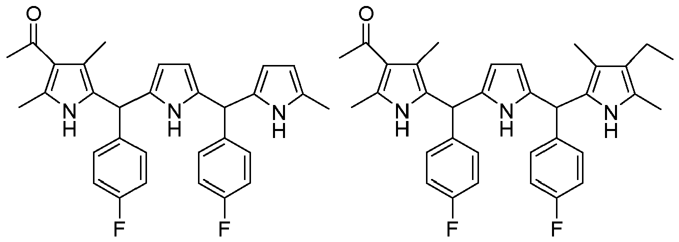

8. Compounds according to anyone of claims 1 to 7, wherein said bacteria of the genus *Staphylococcus,* in particular *Staphylococcus aureus* species, are *Staphylococcus aureus* strains susceptible or resistant to β-lactams (including methicillin-resistant strains also referred as MRSA), and/or susceptible or resistant to glycopeptides (including vancomycin-intermediate or glycopeptides- intermediate *Staphylococcus aureus* strains also referred as VISA or GISA), or multi-drug resistant *Staphylococcus aureus* strains.

9. Compounds according to anyone of claims 1 to 8, wherein said infection(s) caused by bacteria of the genus *Staphylococcus,* in particular *Staphylococcus aureus* species, belong to the group comprising:
- pimples,
- impetigo,
- furuncles,
- cellulitis folliculitis,
- carbuncles,
- scalded skin syndrome,
- abscesses,
- suppurations
- pneumonia, pleuro-pneumonia
- urinary tract infections, pyelonephritis
- peritonitis
- prostatitis
- meningitis,
- meningo-encephalitis
- endophthalmitis
- osteomyelitis,
- endocarditis, myocarditis, pericarditis
- toxic shock syndrome (TSS),
- bacteremia, and
- sepsis.

10. Pharmaceutical composition comprising a compound of the following formula I: 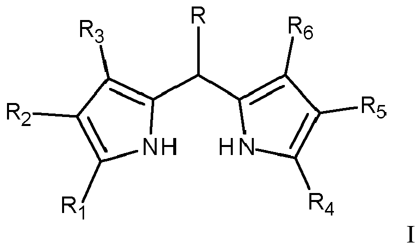 wherein:
R₁, R₂, R₃, R₄, R₅ and R₆ represent independently from each other:
- a H,
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
R represents:
- a H,
- a linear or branched (C₁-C₇)-alkyl,
- (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CO₂-aryl, wherein aryl is an aromatic or heteroaromatic group,
- a CONH-(C₁-C₇)-alkyl,
said R₁, R₂, R₃, R₄, R₅, R₆ and R being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
said (C₁-C₇)-alkyl, (C₂-C₇)-alkenyl, (C₂-C₇)-alkynyl, (C₃-C₇)-cycloalkyl, (C₅-C₇)-cycloalkenyl, (C₃-C₇)-heterocycloalkyl, (C₅-C₇)-heterocycloalkenyl, CO-(C₁-C₇)-alkyl, CO₂-(C₁-C₇)-alkyl, CONH-(C₁-C₇)-alkyl, aryl, alkyl aryl and CO-aryl being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or
heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
in association with a pharmaceutically acceptable vehicle.

11. Pharmaceutical composition according to claim 10, administrable by oral route at a dose comprised from about 10 mg/kg to about 200 mg/kg, or by intravenous route at a dose comprised from about 5 µg/kg to about 50 mg/kg.

12. Pharmaceutical composition according to anyone of claim 10 to 11, comprising a compound of formula I of one of following formulae: 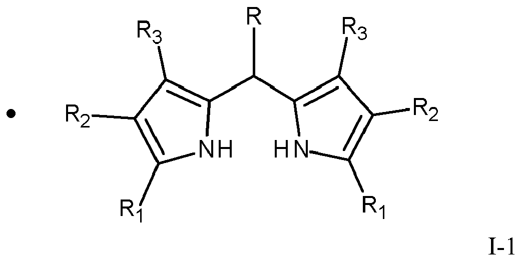 wherein R₁, R₂, R₃ and R are as defined in claim 10, R₂ representing preferably CO-CH₃, or 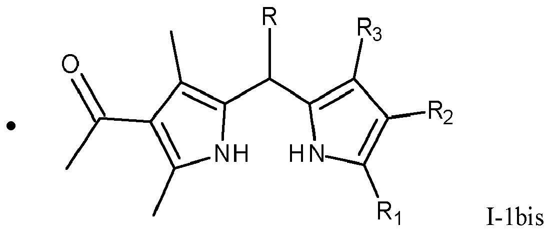 wherein R₁, R₂, R₃ and R are as defined in claim 10, or 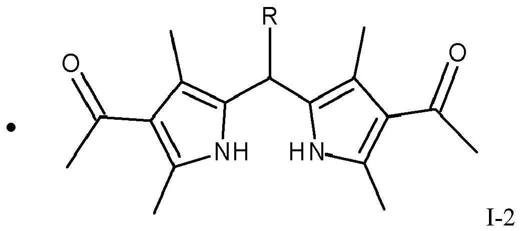 wherein R is as defined in claim 10, or 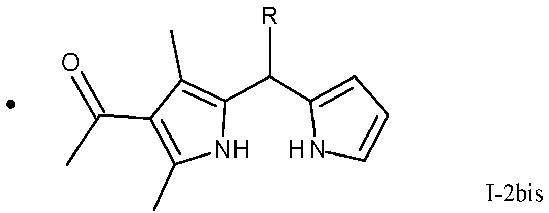 wherein R is as defined in claim 10, or 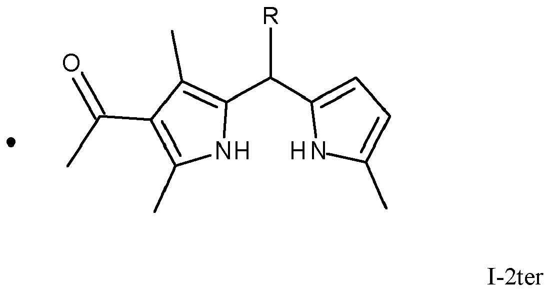 wherein R is as defined in claim 10, or 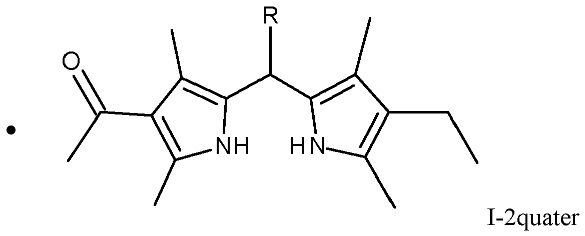 wherein R is as defined in claim 10, or 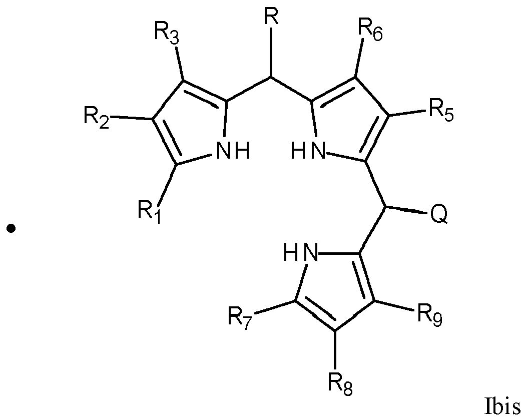 wherein R₁, R₂, R₃, R₅, R₆, and R are as defined in claim 10, and wherein:
R₇, R₈ and R₉ represent independently from each other:
- a H,
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
Q represents:
- a H,
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
said (C₁-C₇)-alkyl, (C₂-C₇)-alkenyl, (C₂-C₇)-alkynyl, (C₃-C₇)-cycloalkyl, (C₅-C₇)-cycloalkenyl, (C₃-C₇)-heterocycloalkyl, (C₅-C₇)-heterocycloalkenyl, CO-(C₁-C₇)-alkyl, CO₂-(C₁-C₇)-alkyl, CONH-(C₁-C₇)-alkyl, aryl, alkyl aryl and CO-aryl being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN, or 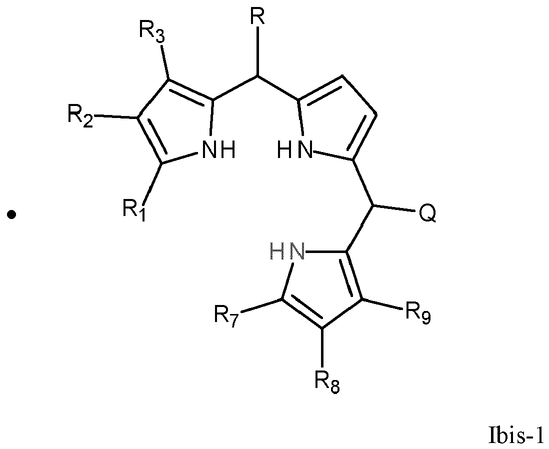 wherein R₁, R₂, R₃, R₇, R₈, R₉, Rand Q are as defined above, or 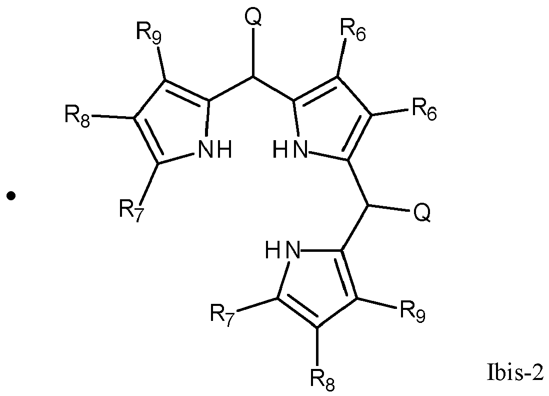 wherein R₆, R₇, R₈, R₉, and Q are as defined above, R₆ representing preferably H, or 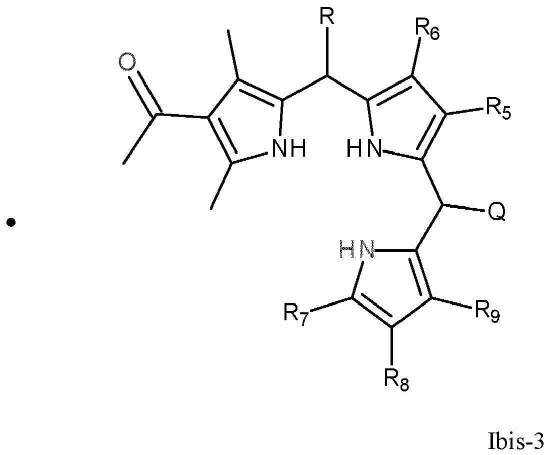 wherein R₅, R₆, R₇, R₈, R₉, R and Q are as defined above, R₆ representing preferably H, or 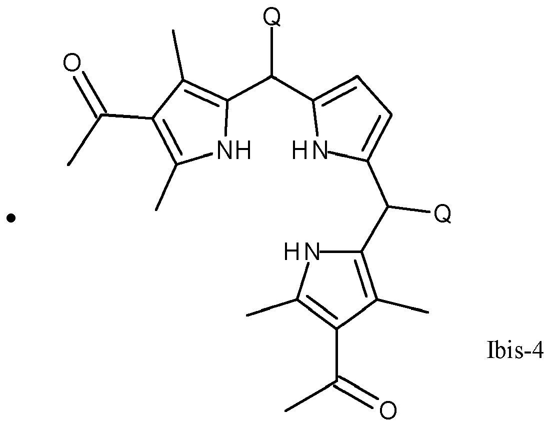 wherein Q is as defined above, or 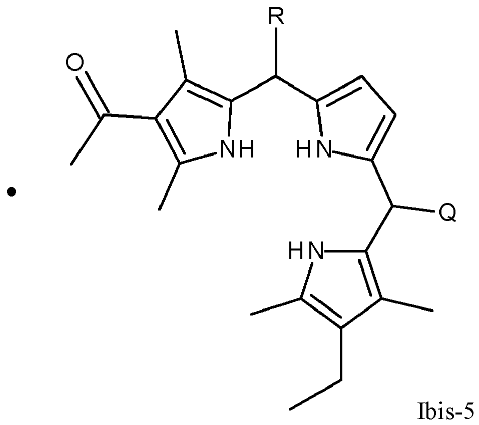 wherein R and Q are as defined above.

13. Pharmaceutical composition according to anyone of claims 10 to 12, wherein said compound is selected from the group comprising:

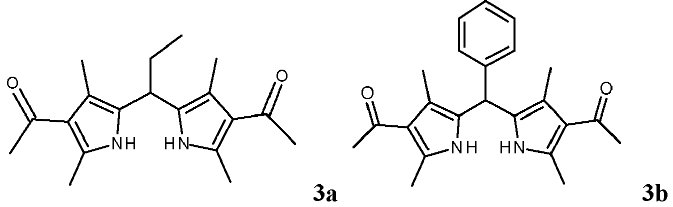

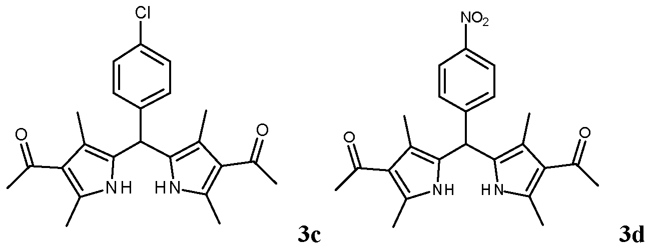

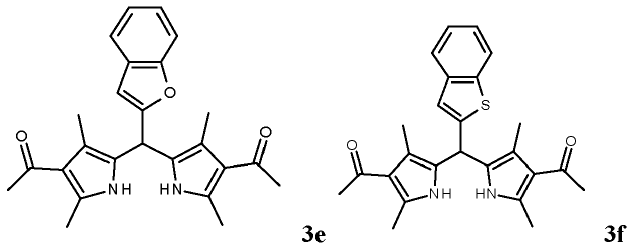

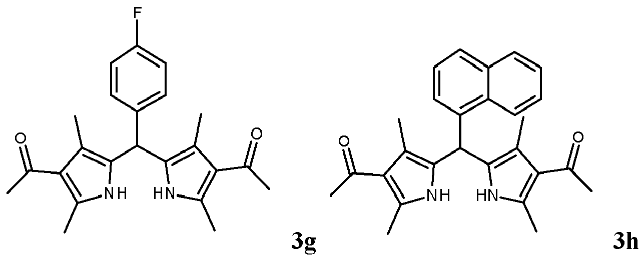

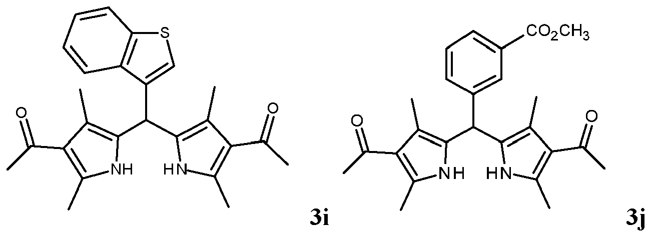

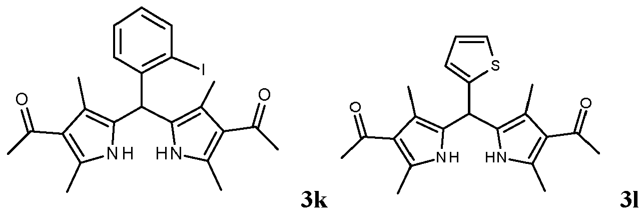

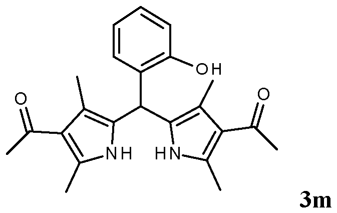

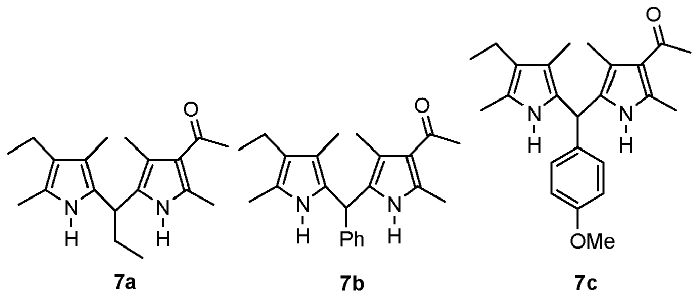

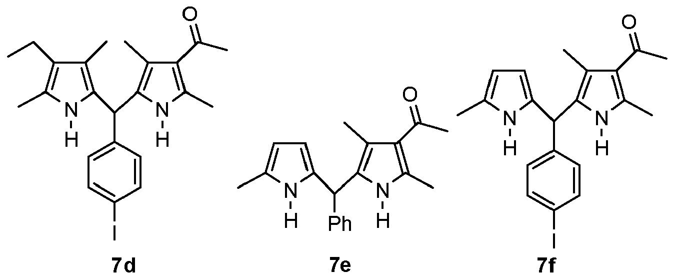

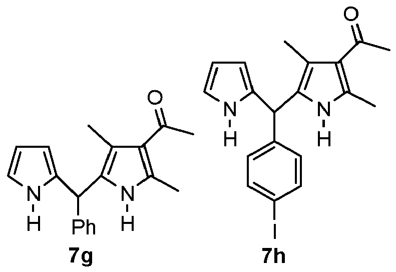

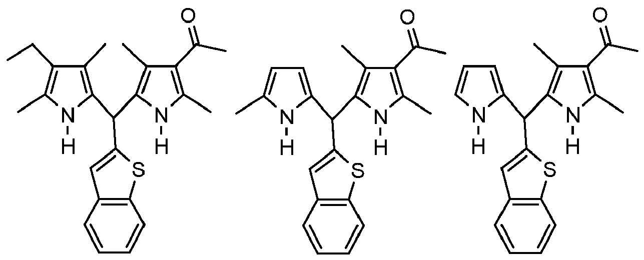

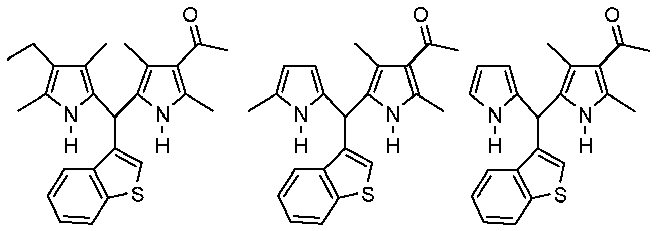

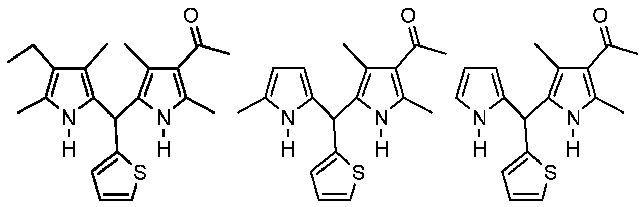

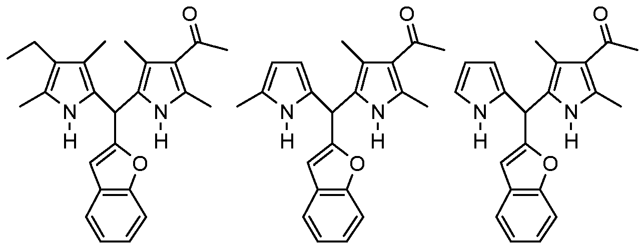

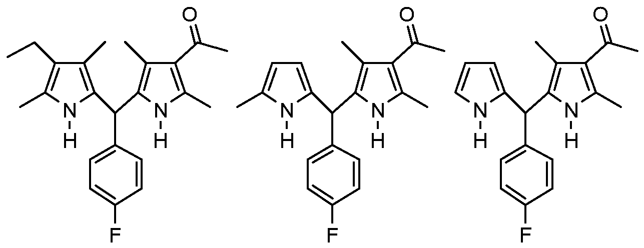

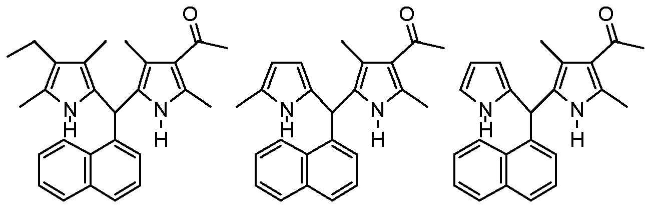

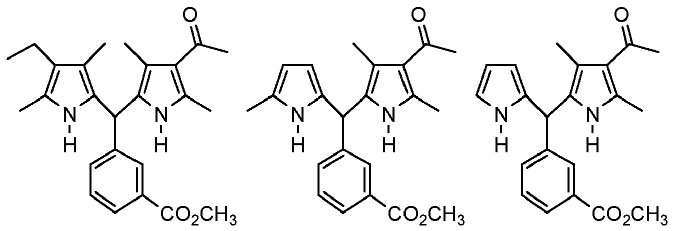

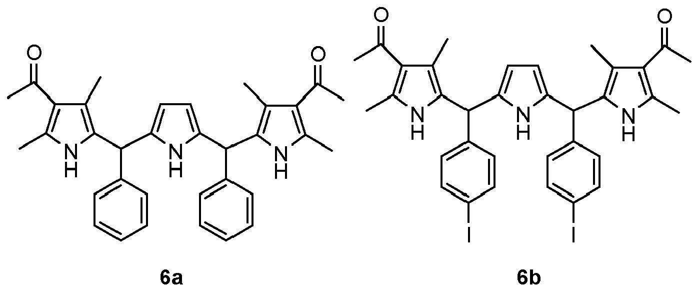

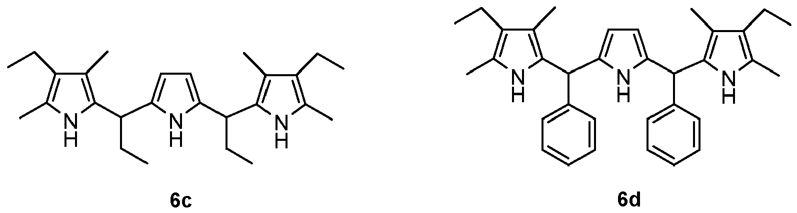

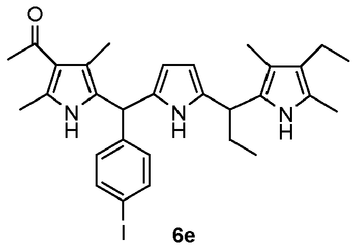

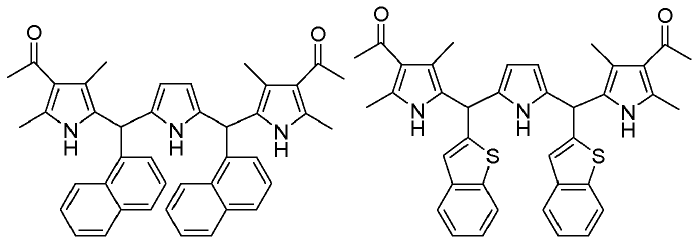

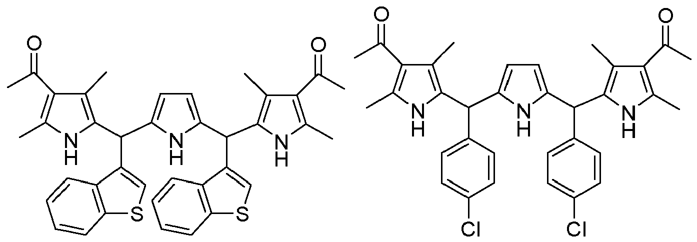

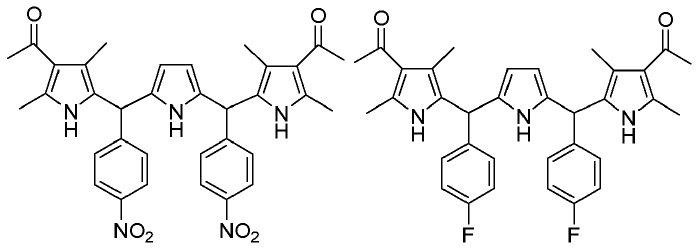

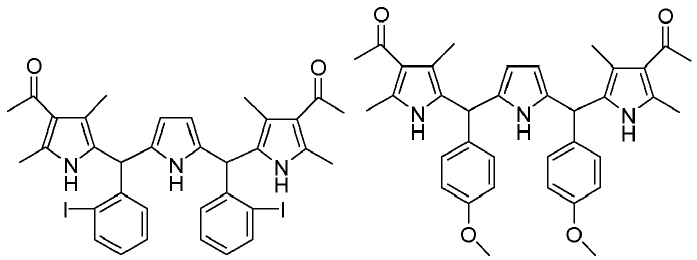

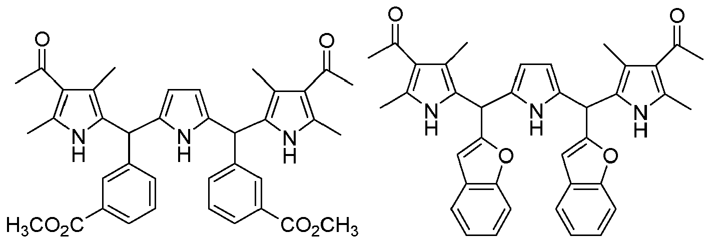

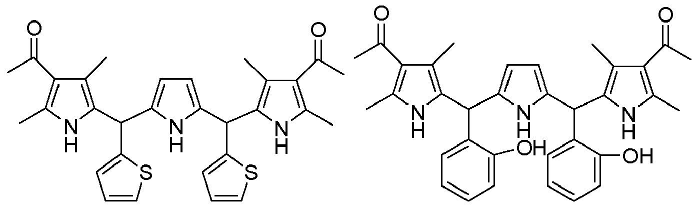

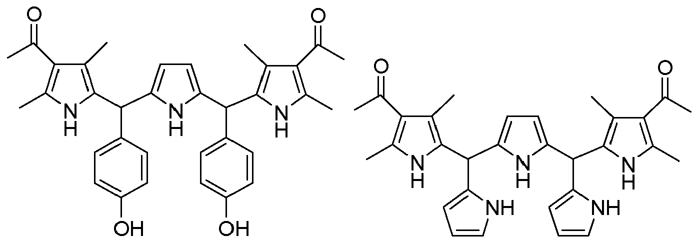

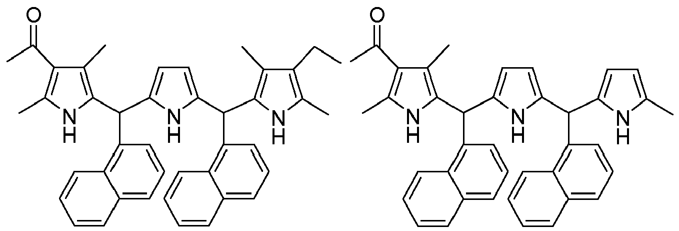

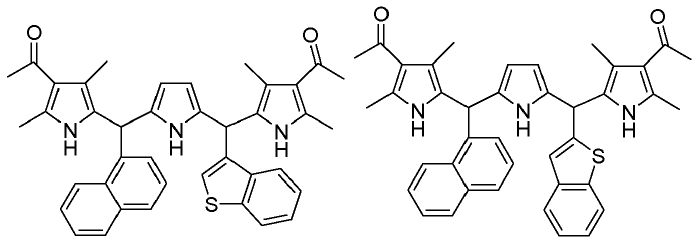

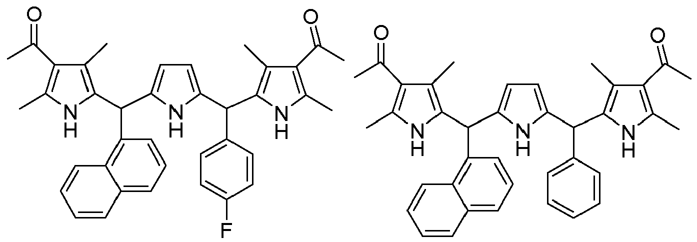

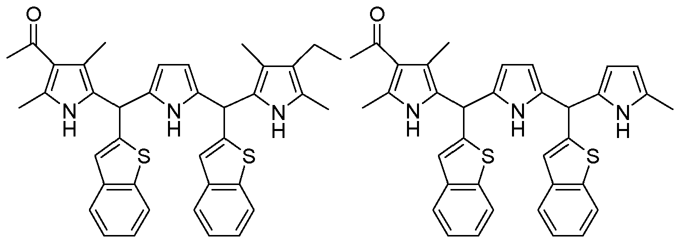

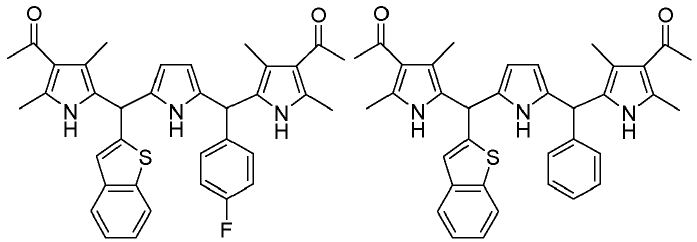

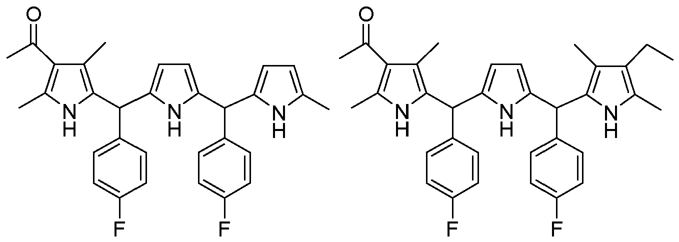

14. Products of the following formula I-21: 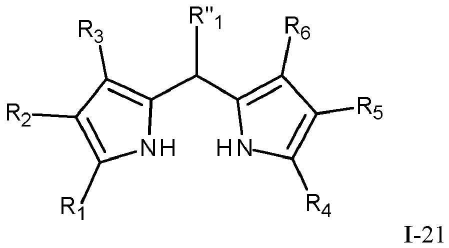 wherein:
R₁, R₂, R₃, R₄, R₅ and R₆ represent independently from each other:
- a H,
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
at least one of R₁, R₂ or R₃ representing a CHO, a CO-(C₁-C₇)-alkyl, or a CO-aryl, R"₁ is selected from the aryls of following formulae (a") to (p"): 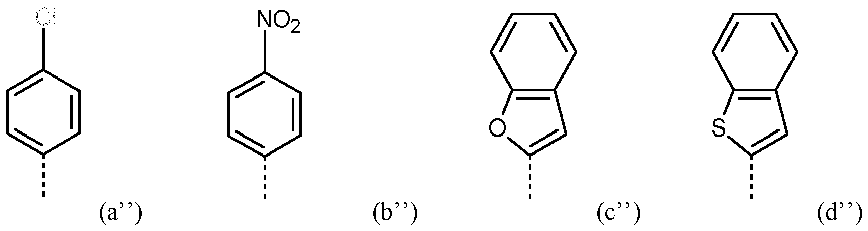 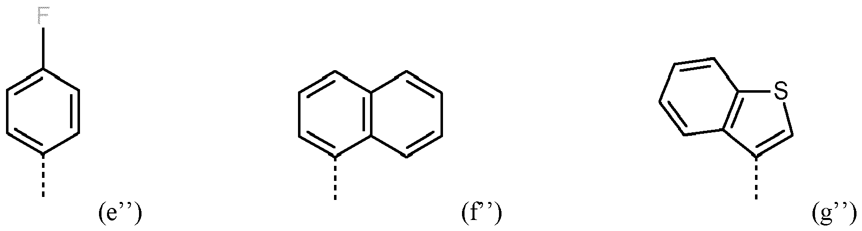 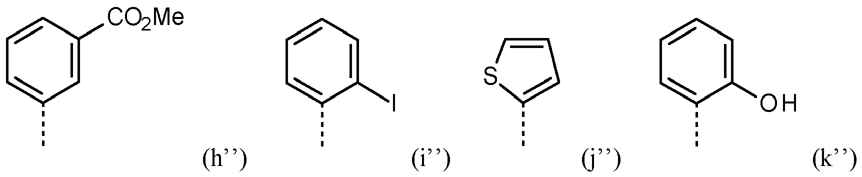 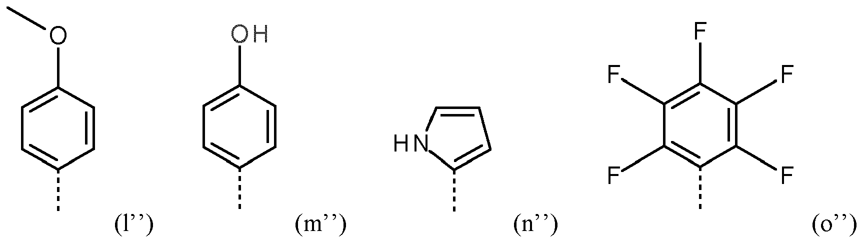 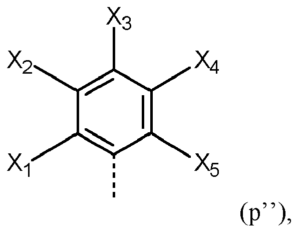 wherein X₁, X₂, X₃, X₄ and X₅ represent independently from each other:
- F, Cl, Br or I, or
- H, F, Cl, Br or I, provided at least one of X₁, X₂, X₃, X₄ and X₅, in particular at least two, in particular at least three, in particular four of X₁, X₂, X₃, X₄ and X₅ do not represent H,
said R₁, R₂, R₃, R₄, R₅, R₆ and R" ₁ being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
said (C₁-C₇)-alkyl, (C₂-C₇)-alkenyl, (C₂-C₇)-alkynyl, (C₃-C₇)-cycloalkyl, (C₅-C₇)-cycloalkenyl, (C₃-C₇)-heterocycloalkyl, (C₅-C₇)-heterocycloalkenyl, CO-(C₁-C₇)-alkyl, CO₂-(C₁-C₇)-alkyl, CONH-(C₁-C₇)-alkyl, aryl, alkyl aryl and CO-aryl being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or
heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
provided that:
- none of R₁, R₂ and R₃ forms a cycle with R₄, R₅ or R₆,
- R₁ and R₄ do not form a cycle with respectively R₂ or R₃, and R₅ or R₆,
- R₂ and R₅ do not form a cycle with respectively R₃ and R₆,
- R₁ and/or R₄ represent(s) an alkyl aryl when R" ₁ is selected from groups (1"), (m") and (n"),
in particular, products of one of the following formulae: 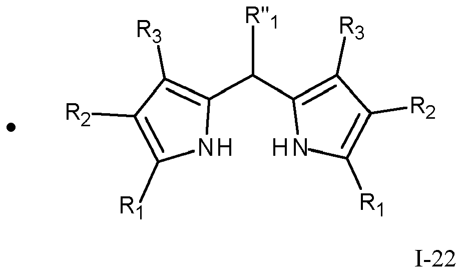 wherein R₁, R₂, R₃ and R"₁ are as defined above,
at least one of R₁, R₂ or R₃ representing a CHO, a CO-(C₁-C₇)-alkyl or a CO-aryl,
R₂ representing preferably CO-CH₃, or 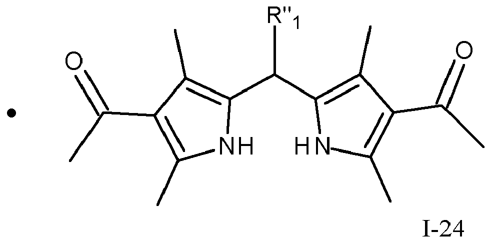 wherein R"₁ is as defined above, or 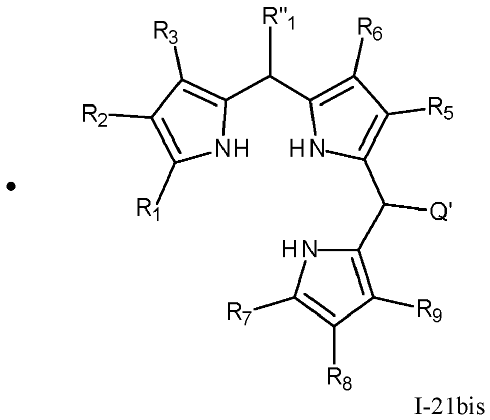 wherein R₁, R₂, R₃, R₅, R₆, and R" ₁ are as defined above, and wherein:
R₇, R₈ and R₉ represent independently from each other:
- a H,
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
Q" represents:
- a H,
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, said aryl being an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
said (C₁-C₇)-alkyl, (C₂-C₇)-alkenyl, (C₂-C₇)-alkynyl, (C₃-C₇)-cycloalkyl, (C₅-C₇)-cycloalkenyl, (C₃-C₇)-heterocycloalkyl, (C₅-C₇)-heterocycloalkenyl, CO-(C₁-C₇)-alkyl, CO₂-(C₁-C₇)-alkyl, CONH-(C₁-C₇)-alkyl, aryl, alkyl aryl and CO-aryl being substituted or not by one or more substituent(s), each independently selected from:
- a linear or branched (C₁-C₇)-alkyl,
- a linear or branched (C₂-C₇)-alkenyl,
- a linear or branched (C₂-C₇)-alkynyl,
- a (C₃-C₇)-cycloalkyl,
- a (C₅-C₇)-cycloalkenyl,
- a (C₃-C₇)-heterocycloalkyl,
- a (C₅-C₇)-heterocycloalkenyl,
- an aryl, wherein the aryl is an aromatic or heteroaromatic group,
- an alkyl aryl, wherein the aryl is an aromatic or heteroaromatic group,
- a CHO,
- a CO-(C₁-C₇)-alkyl,
- a CO-aryl, wherein aryl is an aromatic or heteroaromatic group,
- a CO₂H,
- a CO₂-(C₁-C₇)-alkyl,
- a CONH-(C₁-C₇)-alkyl,
- a halogen selected from the group comprising F, Cl, Br, and I,
- CF₃,
- ORₐ, wherein Rₐ represents:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NR_{b}R_{c}, wherein R_{b} and R_{c} represent independently from each other:
H, a linear or branched (C₁-C₇)-alkyl, a (C₃-C₇)-cycloalkyl, CO-(C₁-C₇)-alkyl, or CO-aryl, wherein aryl is an aromatic or heteroaromatic group,
- NO₂,
- CN,
Q" representing preferably a linear or branched (C₁-C₇)-alkyl or an aryl, more preferably a linear or branched (C₁-C₇)-alkyl or an aryl selected from the aryls of following formulae (a") to (r"): 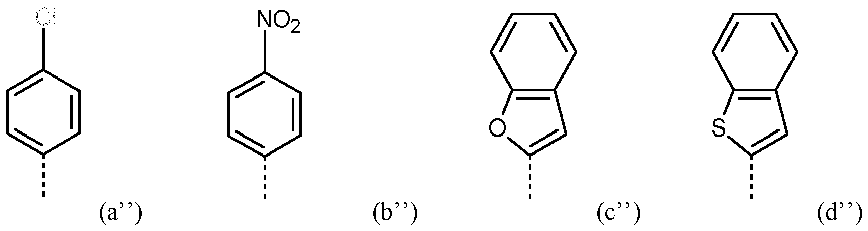 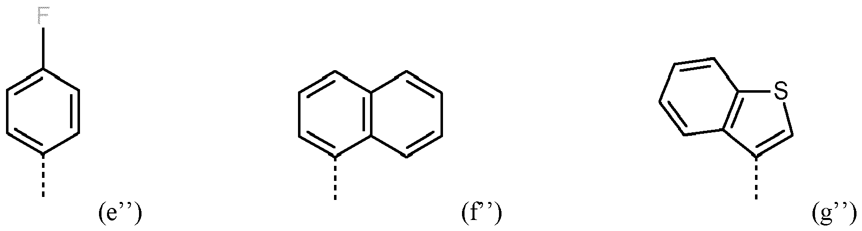 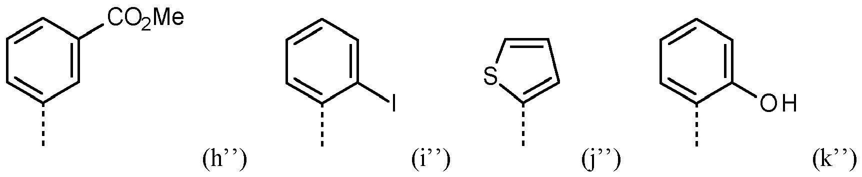 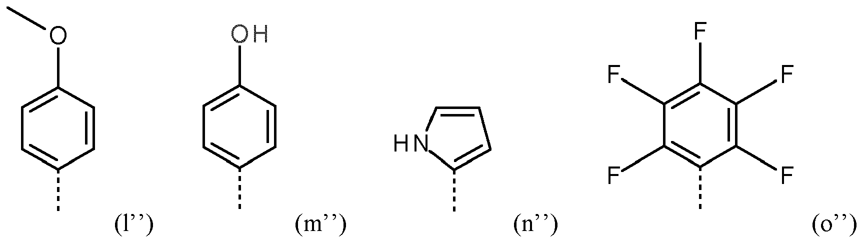 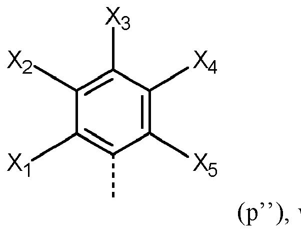 wherein X₁, X₂, X₃, X₄ and X₅ represent independently from each other:
- F, Cl, Br or I, or
- H, F, Cl, Br or I, provided at least one of X₁, X₂, X₃, X₄ and X₅, in particular at least two, in particular at least three, in particular four of X₁, X₂, X₃, X₄ and X₅ do not represent H, 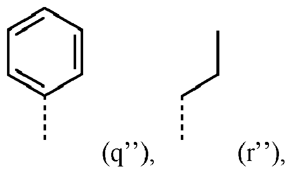 R₅ and R₆ representing preferably H.

15. Products according to claim 14 selected from the group comprising :
